# EUROPEAN PATENT APPLICATION

(11) **EP 2 246 331 A1**
(43) Date of publication of application: **03.11.2010**
(21) Application number: 09158697.4
(22) Date of filing: 24.04.2009
(51) Int. Cl.: C07D 223/16, A61K 31/55, A61P 25/00

(54) **NR2B-selective NMDA-receptor antagonists**

(71) Applicant: Westfälische Wilhelms-Universität Münster, 48149 Münster (DE)
(72) Inventor: Wünsch, Bernhard, 48161 Münster (DE); Tewes, Bastian, 48145 Münster (DE); Schepmann, Dirk, 48147 Münster (DE)
(74) Representative: Michalski Hüttermann & Partner Patentanwälte

(57) **Abstract**

The present invention relates to NMDA antagonists that target the NR2B subunit according to general formula (I) and pharmaceutical compositions comprising compounds according to general formula (I).

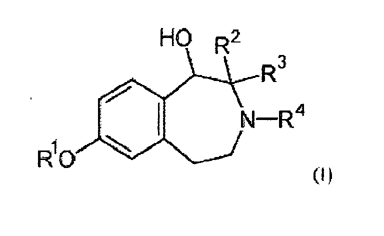

## Description

The present invention relates to derivatives of benzo-fused nitrogen heterocycles. In particular, the present invention relates to the use of derivatives of benzo-fused nitrogen heterocycles as antagonists of the NMDA NR2B receptor.

N-methyl-D-aspartate (NMDA) receptors are heteromeric assemblies of subunits, of which two major subunit families are designated NR1 and NR2. The NR2 subunit family further is divided into four subunit types denoted NR2A, NR2B, NR2C, and NR2D. The variety of NMDA receptors differs in physiological and pharmacological properties such as ion gating properties, magnesium sensitivity, pharmacological profile, and in anatomical distribution. For example, while NR1 is found throughout the brain, NR2 subunits are differentially distributed.

While NMDA receptor inhibition has therapeutic utility primarily in the treatment of pain and neurodegenerative diseases, there are significant liabilities to many available NMDA receptor antagonists that can cause potentially serious side effects. The more discrete distribution of NR2B subunit in the central nervous system may support a reduced side-effect profile of agents that act selectively at this site. However, even selective NR2B antagonists may exhibit low affinity towards the NR2B subunit of the NMDA receptor. Also, some NR2B antagonists which are claimed to be NR2B selective might not be entirely specific. Further, NR2B antagonists may exhibit unfavourable pharmacokinetics.

Thus, it would be desirable to provide novel NMDA antagonists that target the NR2B subunit of the NMDA receptor.

Therefore, the object underlying the present invention was to provide novel compounds being usable in pharmacological treatment particularly in the treatment of diseases of the central nervous system.

The problem is solved by compounds according to general formula (I) as given as follows and/or racemates, enantiomers, diastereomers, solvates, hydrates, and pharmaceutically acceptable salts and/or esters thereof: 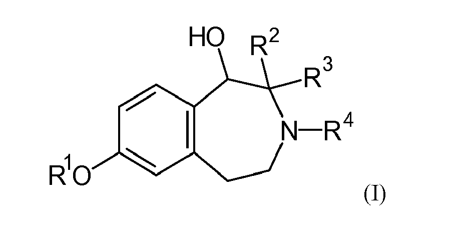 wherein:
- R¹: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; and/or C₇- C₁₄₋arylalkyl wherein the aryl group has 6 to 10 carbon atoms and the alkyl group has 1 to 4 carbon atoms;
- R²: is selected from the group comprising hydrogen; C₁-C₈-alkyl; C₂-C₈-alkenyl; C₃-C₈- cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; and/or C₇-C₁₄-arylalkyl wherein the aryl group has 6 to 10 carbon atoms and the alkyl group has 1 to 4 carbon atoms;
- R³: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms; linear or branched alkyl groups of the type -CₙH₂ₙ-U-D wherein n is 1, 2, 3 or 4, U is selected from the group comprising O, CO,
- or: COO, CONH, S, guanidine and/or NH and D is selected from the group comprising H and/or C₁-C₃-alkyl; -CH₂-C₆H₄-X wherein X is selected from the group comprising OH, SH, C₁-C₃-alkyl and/or NH₂; -CH₂-imidazole; -CH₂-indole; -CH₂-(furanyl-3-yl); -CH₂- (pyridyl-3-yl) and/or -CH₂-(imidazolyl-3-yl);
- R² and R³: together with the carbon atom to which they are attached form a 5- to 7-membered non aromatic carbocycle or heterocycle comprising from 1 to 3 hetero atoms selected from the group comprising O, N and/or S;
- R⁴: is selected from the group comprising hydrogen; C₁-C₁₀-alkyl; -W and/or -Y-Z;
- Y: is selected from the group comprising C₁-C₆-alkyl; C₂-C₆-alkenyl; C₂-C₆-alkynyl, C₃-C₆-cycloalkyl; C₄-C₁₀-cycloalkylalkyl wherein the cycloalkyl group has 3 to 6 carbon atoms and the alkyl group has 1 to 4 carbon atoms; C₆-C₁₀-aryl; C₇-C₁₄-arylalkyl wherein the aryl group has 6 to 10 carbon atoms and the alkyl group has 1 to 4 carbon atoms; C₁-C₆₋alkyl comprising at least one moiety independently selected from the group comprising oxygen, sulphur, SO, SO₂, CO, NH, N(C₁-C₃-alkyl) and/or T; a 3- to 6-membered aromatic or non aromatic carbocycle or heterocycle containing at least one of O, N or S as heteroatoms; and/or a structural element comprising a 3- to 6-membered aromatic or non aromatic carbocycle or heterocycle comprising from 1 to 3 heteroatoms selected from the group comprising O, N and/or S and a group selected from the group comprising oxygen, sulphur, SO, SO₂, CO, NH, N(C₁-C₃₋alkyl) and/or C₁-C₆-alkyl comprising at least one moiety independently selected from the group comprising oxygen, sulphur, SO, SO₂, CO, NH and/or N(C₁-C₃-alkyl);
- T: is selected from the group comprising 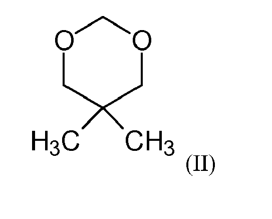
- W: is selected from the group comprising 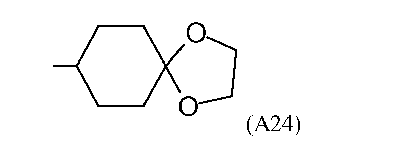
- Z: is selected from the group comprising mono-, bi- or tricyclic aromatic or non aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl;
- or R³ and R⁴: together with the ring atoms to which they are attached form a 5- to 7-membered non aromatic heterocycle comprising from 1 to 3 heteroatoms selected from the group comprising O, N and/or S.

Furthermore, the invention relates to a pharmaceutical composition comprising a compound according to the invention as an active ingredient and a method for preparing such compounds. Preferred embodiments of compounds are given in the dependant claims.

Surprisingly it was found that a compound according to the invention can exhibit a high affinity to the NR2B subunit of the NMDA receptor. Beneficially, the compounds according to the invention can exhibit pharmaceutical effects in the treatment of neuropsychiatric and/or neurodegenerative diseases.

The term "alkyl" according to the invention is to be understood as meaning straight-chain or branched alkyl groups. The term "C₁-C₈-alkyl" as used herein refers to straight-chain or branched alkyl groups having 1 to 8 carbon atoms. Preferred C₁-C₈-alkyl groups are selected from the group comprising methyl, ethyl and the isomers of propyl, butyl, pentyl, hexyl, heptyl or octyl, such as, for example, isopropyl, isobutyl, tert.-butyl, sec.-butyl and/or isopentyl.

The term "alkenyl" according to the invention is to be understood as meaning straight-chain or branched alkyl groups having at least one or several double bonds. The term "C₂-C₈-alkenyl" as used herein refers to straight-chain or branched alkenyl groups having 2 to 8 carbon atoms and at least one or several double bonds. Preferred C₂-C₈-alkenyl groups are selected from the group comprising propen-1-yl, allyl, buten-1-yl, buten-2-yl and/or buten-3-yl.

The term "alkynyl" according to the invention is to be understood as meaning straight-chain or branched alkyl groups having at least one or several carbon-carbon triple bonds. The term "C₂-C₈-alkynyl" as used herein refers to straight-chain or branched alkynyl groups having 2 to 8 carbon atoms and at least one or several triple bonds. Preferred C₂-C₈-alkenyl groups are selected from the group comprising propyn-2-yl, butyn-1-yl, butyn-2-yl, pentyn-4-yl, and/or propargyl.

The term "cycloalkyl" according to the invention is to be understood as meaning carbocycles, and includes mono-, bi- and tricyclic saturated carbocycles, as well as fused ring systems. Such fused ring systems can include one ring that is partially or fully unsaturated such as a benzene ring to form fused ring systems such as benzofused carbocycles. Cycloalkyl includes such fused ring systems as spirofused ring systems. The term "C₃-C₈-cycloalkyl" as used herein refers to carbocycles having 3 to 8 carbon atoms. Preferred cycloalkyl groups are selected from the group comprising cyclopropyl, cyclobutyl, cyclopentyl and/or cyclohexyl.

The term "C₆-C₁₀-aryl" according to the invention is to be understood as meaning aromatic groups having 6 to 10 carbon atoms. Preferably, the term "C₆-C₁₀-aryl" refers to carbocycles. Preferred C₆-C₁₀-aryl is selected from the group comprising phenyl or naphthyl.

The terms "cycloalkylalkyl", "cycloalkylalkenyl", "arylalkyl", "arylalkenyl", and "cycloalkenylalkyl" according to the invention unless specifically stated otherwise are to be understood as meaning groups which bond by the respective last-mentioned group, for example referring to "arylalkyl" by the alkyl group.

The term "C₄-C₁₀-cycloalkylalkyl wherein the cycloalkyl group has 3 to 6 carbon atoms and the alkyl group has 1 to 4 carbon atoms" preferably is a group phenylalkyl wherein the alkyl has 1 to 4 carbon atoms, more preferably selected from the group comprising benzyl, phenylethyl and/or phenylbutyl.

The term "alkyloxy" according to the invention is to be understood as meaning an alkyl group connected to the oxy connecting atom unless specifically stated otherwise. The term "C₁-C₄₋alkyloxy" as used herein refers to an alkyloxy group having 1 to 4 carbon atoms. C₁-C₄₋alkyloxy group are preferably selected from the group comprising methoxy, ethoxy, n-propoxy, isopropoxy, n-butoxy, iso-butoxy, secondary-butoxy and/or tertiary-butoxy.

As used herein, the term "heterocycle" refers to a stable 5- to 7-membered monocyclic or bicyclic heterocyclic ring or a 7- to 10-membered bicyclic heterocyclic ring which is either saturated or unsaturated, and which comprises carbon atoms and from 1 to 3 heteroatoms selected from the group comprising N, O and/or S. The term "heterocycle" includes bicyclic groups in which any of the above-defined heterocyclic rings is fused to a benzene ring. Preferred heterocycles are monocyclic heteroarylic rings.

Preferred heterocycles are selected from the group comprising furan, tetrahydrofuran, thiophene, tetrahydropyran, pyrrole, pyrrolidine, imidazole, 1,2,4-triazole, piperidine, pyridine, pyrimidine, morpholine or azacycloheptane. Further preferred heterocycles are selected from the group comprising furan-2-yl, tetrahydrofuran-2-yl, tetrahydrofuran-3-yl, tetrahydropyran-2-yl, 1,3-dioxolan-5-yl, pyrrol-1-yl, pyrrol-2-yl, pyrrolidin-1-yl, isoxazol-3-yl, isoxazol-4-yl, 1,2-di-thiazolin-5-yl, imidazol-1-yl, 1,2,4-triazol-1-yl, 1,3,4-triazol-1-yl, thiophen-2-yl, piperidin-1-yl, piperidin-4-yl, pyridin-2-yl, pyridin-3-yl, pyridin-4-yl, pyrimidin-2-yl, pyrimidin-4-yl, pyrimidin-5-yl, morpholin-1-yl, azacycloheptan-1-yl and/or benzo-1,2,3-thiadiazol-7-yl.

The term "heteroatoms" according to the invention preferably relates to N, O and S, unless specifically stated otherwise.

The term "halogen" according to the invention is to be understood as meaning fluorine, chlorine, bromine or iodine, preferably fluorine, chlorine or bromine.

The term "amino acid" according to the invention is to be understood as meaning alpha amino acids, molecules containing both amine and carboxyl functional groups attached to the same carbon, which is called the alpha-carbon. The term "amino acid" according to the invention is to be understood as being broadly defined to include any modified and unusual amino acid. Preferred amino acids are naturally occurring amino acids. Representative amino acids include, but are not limited to, the group comprising glycine, alanine, serine, threonine, arginine, lysine, aspartic acid, glutamic acid, asparagine, glutamine, phenylalanine, tyrosine, tryptophan, leucine, valine, isoleucine, cysteine, methionine, histidine and/or proline. The various alpha amino acids differ in which side chain is attached to their alpha carbon.

The term "side chains of amino acids" according to the invention is to be understood as meaning the groups attached to the alpha carbon of alpha-amino acids. For example the side chains of glycine, alanine, valine, leucine and phenylalanine are hydrogen, methyl, iso-propyl, isobutyl and benzyl, respectively.

In preferred embodiments of the compounds according to the invention the substituent R¹ is selected from the group comprising hydrogen, straight-chain or branched C₁-C₆-alkyl and/or benzyl. Preferably, the substituent R¹ is an alkyl group selected from the group comprising methyl and/or ethyl.

Advantageously, the substituent R¹ being selected from the group comprising hydrogen, straight-chain or branched C₁-C₆-alkyl and/or benzyl can result in a substantial increase in affinity and/or selectivity of the compound towards the NR2B subunit of the NMDA receptor.

Preferably, the substituent R² of the compounds according to the invention is hydrogen.

In other preferred embodiments of the compounds according to the invention the substituent R³ is a side chain of an amino acid. Preferably, the substituent R³ is a side chain of a naturally occurring amino acid selected from the group comprising glycine, alanine, serine, threonine, arginine, lysine, aspartic acid, glutamic acid, asparagine, glutamine, phenylalanine, tyrosine, tryptophan, leucine, valine, isoleucine, cysteine, methionine, histidine and/or proline.

Advantageously, the use of amino acids having S- or R-configuration provides for a synthesis of the compounds according to the invention having selected stereochemistry.

In preferred embodiments of the compounds according to the invention the substituent R² is hydrogen and the substituent R³ is a side chain of an amino acid selected from the group comprising hydrogen; linear or branched C₁-C₄-alkyl; linear or branched alkyl groups of the type -CₙH₂ₙ-U-D wherein n is 1, 2, 3 or 4, U is selected from the group comprising O, CO, COO, CONH, S, guanidine and/or NH, and D is selected from the group comprising H and/or methyl; -CH₂-C₆H₄-OH; -CH₂-imidazole and/or - CH₂-indole.

In other embodiments of the compounds according to the invention the substituents R² and R³ together with the carbon atom to which they are attached form a 5-, 6- or 7-membered non aromatic carbocycle or heterocycle, preferably a heterocycle having one N atom.

In other preferred embodiments of the compounds according to the invention the substituents R³ and R⁴ together with the ring atoms to which they are attached form a 5-membered non aromatic heterocycle having one N atom. In this embodiment the substituents R³ and R⁴ together with the ring atoms to which they are attached form the side chain of proline.

The substituent R⁴ can be C₆-C₉-alkyl, preferably C₇-C₈-alkyl, more preferably straight chain C₇-C₈-alkyl.

In preferred embodiments the substituent R⁴ is a group -Y-Z.

Preferably, Y is a non aromatic group. Preferably, Y is selected from the group comprising C₃-C₅-alkyl; C₃-C₅-alkenyl; C₅-C₆-cycloalkyl; C₃-C₅-alkyl comprising at least one moiety independently selected from the group comprising oxygen, sulphur, SO, SO₂, CO, NH, N(C₁-C₃-alkyl) and/or T; a 5-to 6-membered non aromatic carbocycle or heterocycle containing at least one of O, N or S as heteroatoms; and/or a structural element comprising a 5- to 6-membered non aromatic carbocycle or heterocycle comprising from 1 to 3 heteroatoms selected from the group comprising O, N and/or S and a group selected from the group comprising oxygen, sulphur, SO, SO₂, CO, NH, N(C₁-C₃-alkyl) and/or C₁-C₃-alkyl comprising at least one moiety independently selected from the group comprising oxygen, sulphur, SO, SO₂, CO, NH and/or N(C₁-C₃-alkyl).

The length of the substituent Y preferably is the length of a C₃-C₅-alkyl group, more preferably the length of a C₄-alkyl group. Preferably, the substituent Y is -(CH₂)ₘ- wherein m represents 3, 4 or 5, preferably 4. More preferably, the substituent Y is C₃-C₅-alkyl preferably C₄-alkyl comprising at least one moiety independently selected from the group comprising oxygen, sulphur, SO, SO₂, CO, NH, N(C₁-C₃₋alkyl) and/or T.

Advantageously, the substituent Y having a length of a C₃-C₅-alkyl group, more preferably the length of a C₄-alkyl group, can result in a substantial increase in affinity and/or selectivity of the compound towards the NR2B subunit of the NMDA receptor.

The backbone of the substituent Y preferably is stiff. Preferably, the stiffness is provided by a substituent Y comprising a double bonding, or the substituent Y being a non aromatic carbocycle or heterocycle. In preferred embodiments, the substituent Y is selected from the group comprising C₃-C₅-alkenyl; C₅-C₆-cycloalkyl; a 5- to 6-membered non aromatic carbocycle or heterocycle containing at least one of O, N or S as heteroatoms; and/or a structural element comprising a 5- to 6-membered non aromatic carbocycle or heterocycle comprising from 1 to 3 heteroatoms selected from the group comprising O, N and/or S and a group selected from the group comprising oxygen, sulphur, SO, SO₂, CO, NH, N(C₁-C₃₋alkyl) and/or C₁-C₃-alkyl comprising at least one moiety independently selected from the group comprising oxygen, sulphur, SO, SO₂, CO, NH and/or N(C₁-C₃-alkyl).

The substituent Y preferably is a 3- to 6-membered or 5- to 6-membered aromatic or non aromatic carbocycle or heterocycle containing at least one of O, N or S as heteroatom with the provision that two adjacent heteroatoms are not simultaneously oxygen.

Preferably, the substituent Z is an aromatic group. Advantageously, the substituent Z being an aromatic group can exhibit a positive effect on the affinity and/or selectivity of the compounds to the NR2B subunit of the NMDA receptor.

In preferred embodiments the substituent Z is selected from the group comprising mono-, bi- or tricyclic aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄₋alkyloxy and/or C₁-C₆-alkyl.

Preferably, the substituent Z is selected from the group comprising phenyl, biphenyl, pyridine and/or pyrimidine.

Advantageously, in embodiments where the substituent R⁴ is a group -Y-Z, wherein the substituent Y has the length of a C₄-alkyl group and the substituent Z is an aromatic group, a group -Y-Z can result in a substantial increase in affinity and/or selectivity of the compound towards the NR2B subunit of the NMDA receptor.

In preferred embodiments, the substituent -Y-Z is selected from the group of structural elements as given as follows:

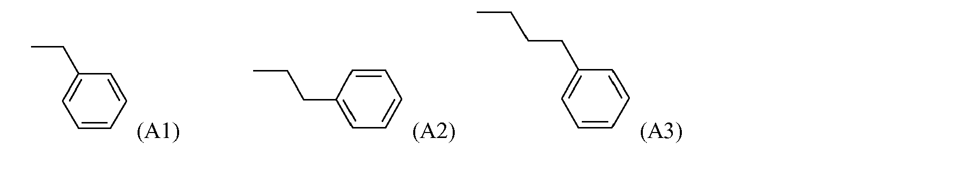

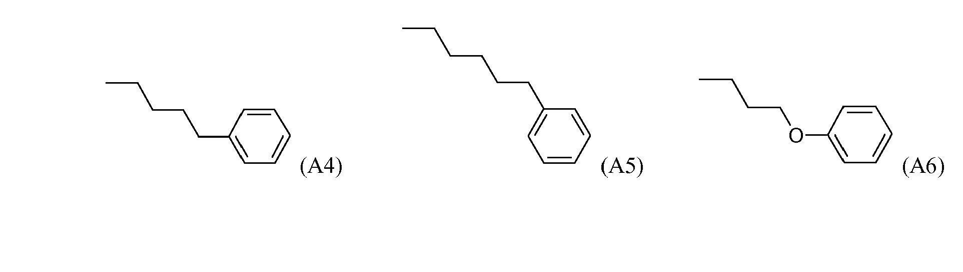

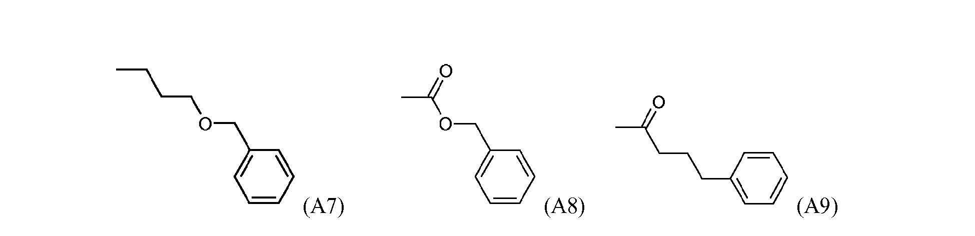

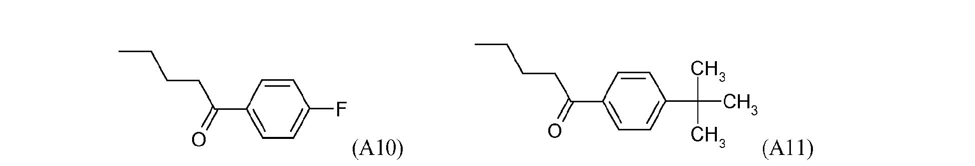

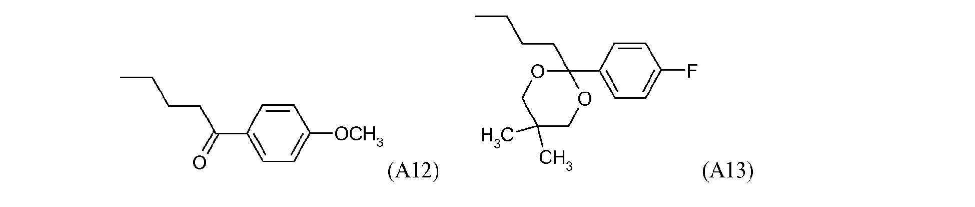

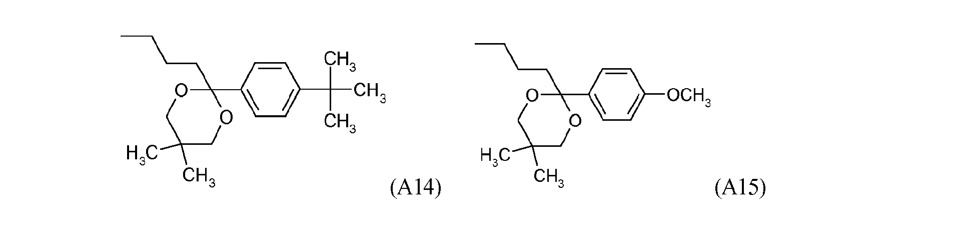

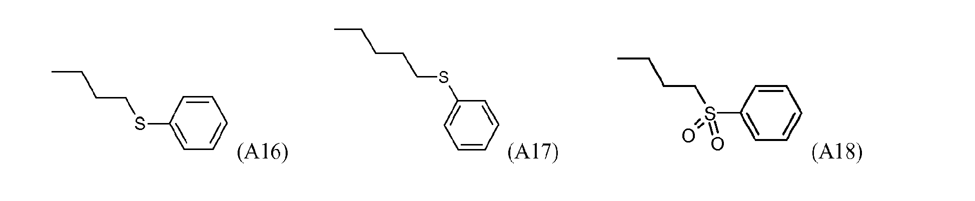

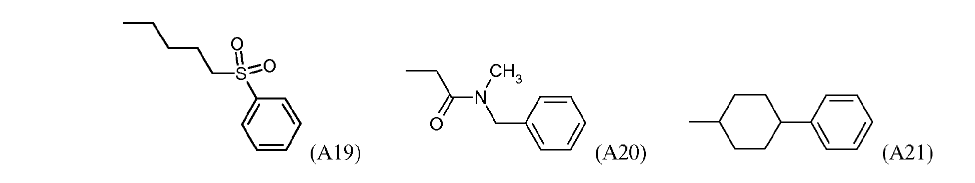

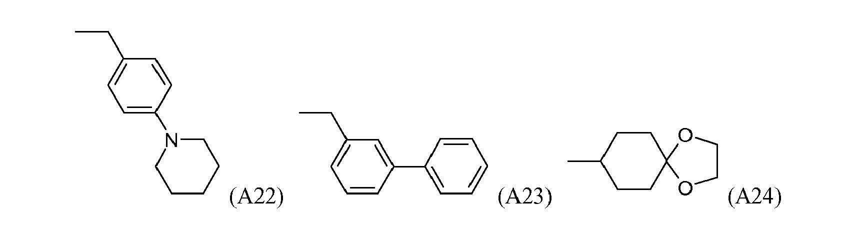

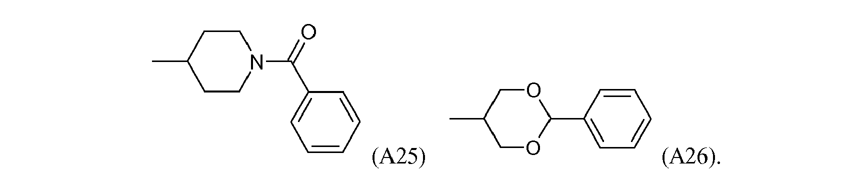

In further preferred embodiments, the substituent R⁴ is selected from the group of structural elements as given as follows:

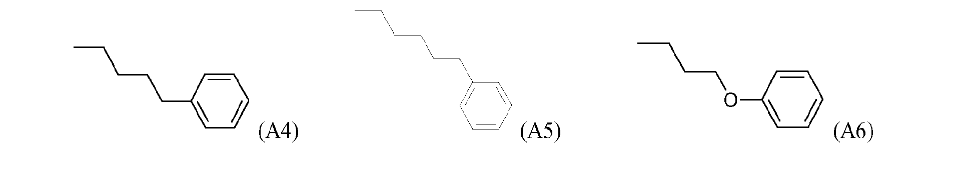

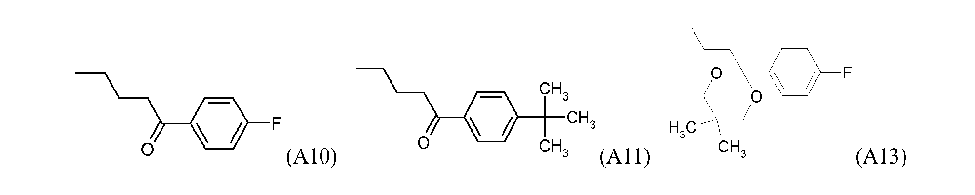

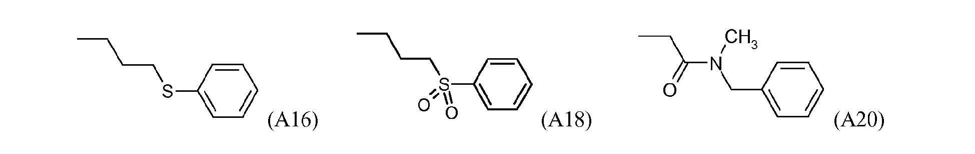

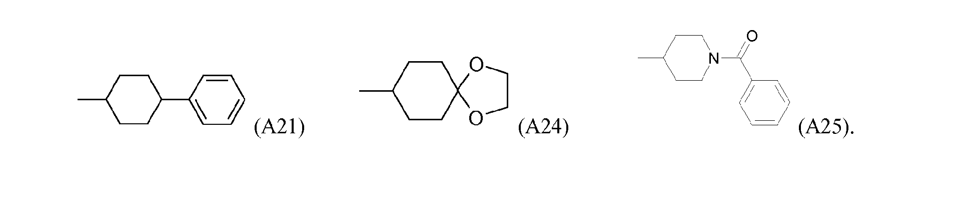

In preferred embodiments, the compound is a compound according to general formula (III) as given as follows: 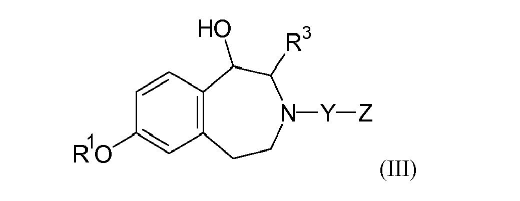 wherein:
- R¹: is selected from the group comprising hydrogen; linear or branched C₁-C₆-alkyl and/or benzyl;
- R³: is a side chain of an amino acid selected from the group comprising hydrogen; linear or branched C₁-C₄-alkyl; linear or branched alkyl groups of the type -CₙH₂ₙ-U-D wherein n is 1, 2, 3 or 4, U is selected from the group comprising O, CO, COO, CONH, S, guanidine and/or NH, and D is selected from the group comprising H and/or methyl; -CH₂-C₆H₄-OH; -CH₂-imidazole and/or -CH₂-indole;
- Y: is selected from the group comprising -(CH₂)ₘ- wherein m represents 3, 4 or 5; C₃-C₅-alkenyl; C₅-C₆-cycloalkyl; C₃-C₅-alkyl comprising at least one moiety independently selected from the group comprising oxygen, sulphur, SO, SO₂, CO, NH and/or N(C₁-C₃-alkyl); a 5- to 6-membered non aromatic carbocycle or heterocycle comprising from 1 to 3 heteroatoms selected from the group comprising O, N and/or S; and/or a structural element comprising a 5- to 6-membered non aromatic carbocycle and a group selected from the group comprising oxygen, sulphur, SO, SO₂, CO, NH, N(C₁-C₃- alkyl) and/or C₁-C₃-alkyl comprising at least one moiety independently selected from the group comprising oxygen, sulphur, SO, SO₂, CO, NH and/or N(C₁-C₃-alkyl);
- Z: is selected from the group comprising mono-, bi- or tricyclic aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl.

In especially preferred embodiments, the compound is selected from the group comprising compounds according to the formulas as given as follows:

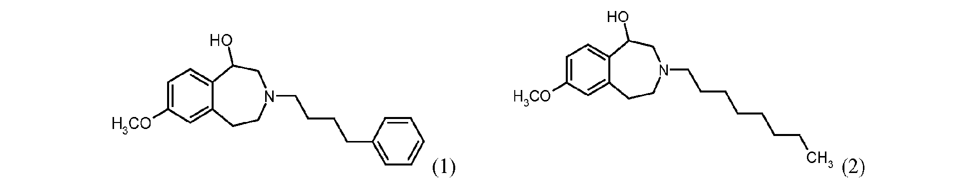

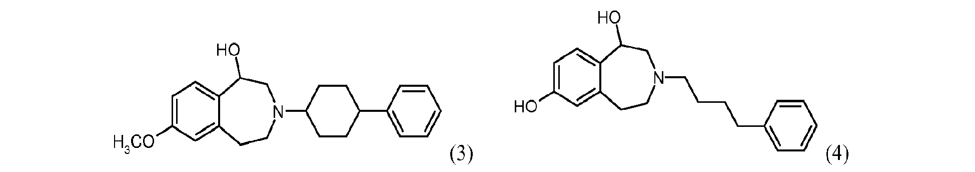

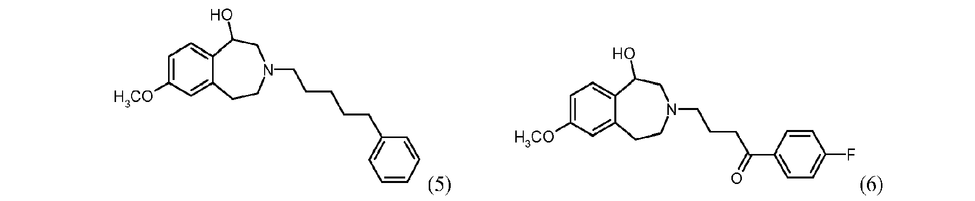

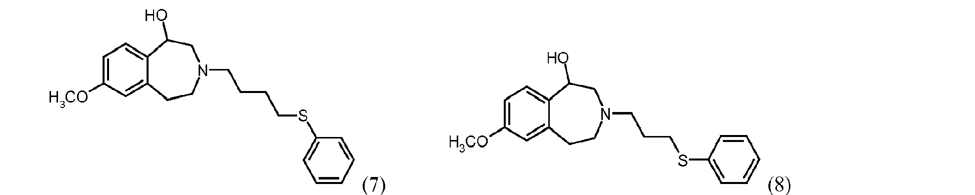

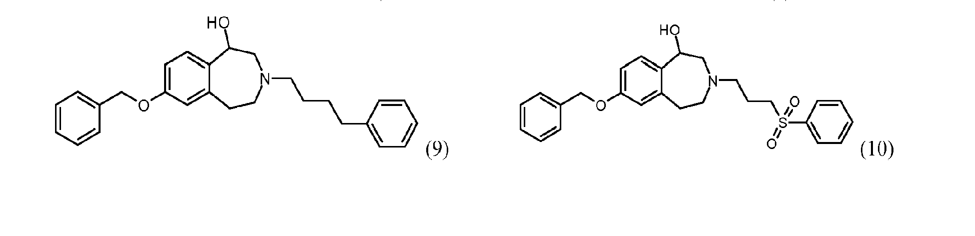

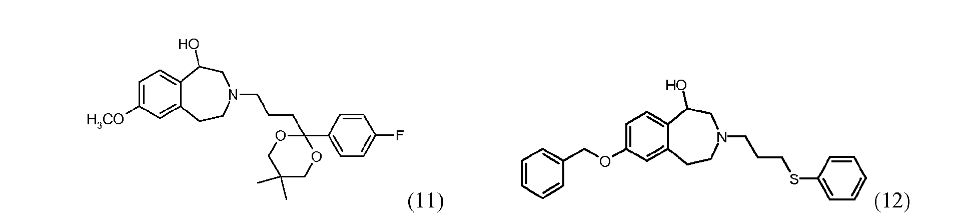

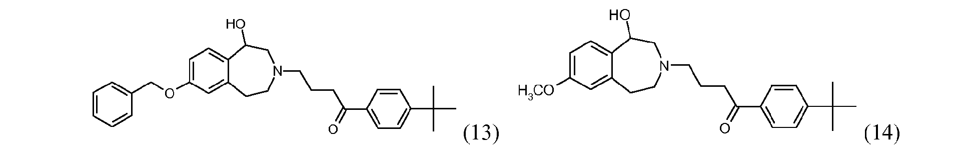

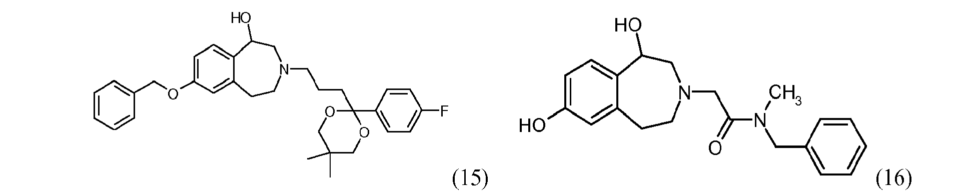

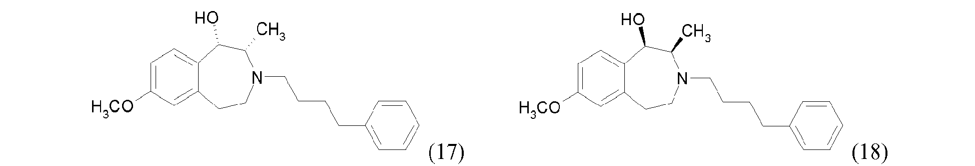

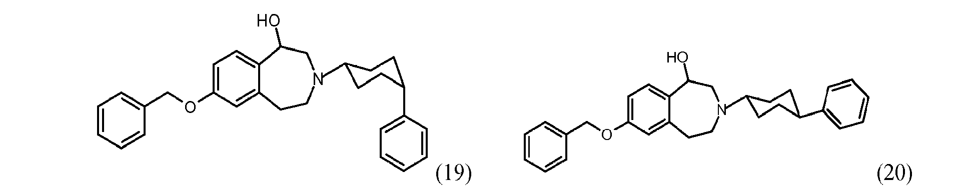

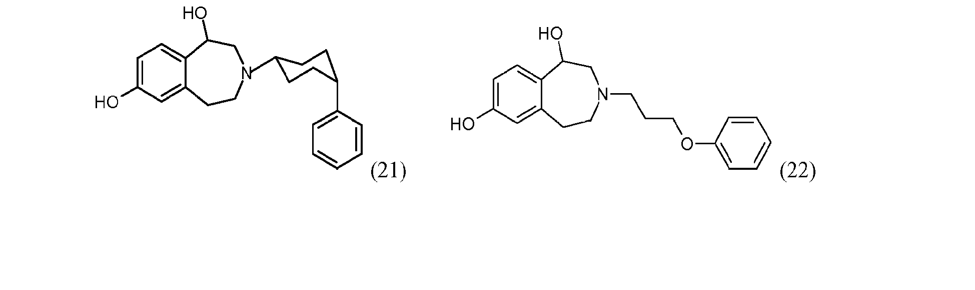

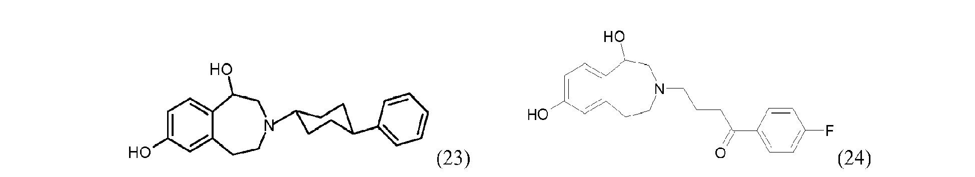

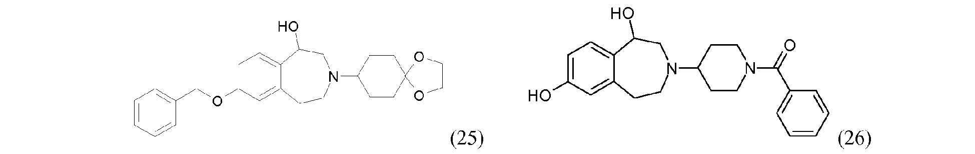

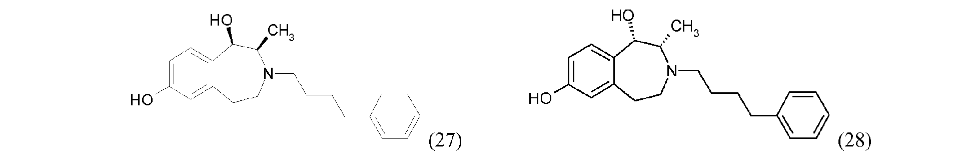

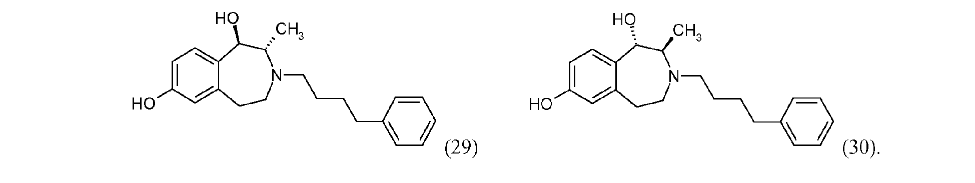

Advantageously, the compounds selected from the group comprising compounds according to the formulas (1) to (30) can exhibit a good affinity and/or selectivity to the NR2B subunit of the NMDA receptor.

The compounds described herein can contain one or more double bonds and may thus give rise to cis/trans isomers as well as other conformational isomers. The present invention includes all such possible isomers as well as mixtures of such isomers.

Further, the compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The formulas are either shown with or without a definitive stereochemistry at certain positions. If shown without a definitive stereochemistry the present invention includes all stereoisomers of the respective formula and solvates, hydrates, and pharmaceutically acceptable salts and esters thereof. Further, mixtures of stereoisomers as well as isolated specific stereoisomers are also included.

Unless specifically stated otherwise, differ compounds, groups or substituents denoted with Arabic numerals and such compounds, groups or substituents denoted with Roman numerals from each other, that is, compounds, groups or substituents are different compounds, groups or substituents.

A further aspect of the present invention relates to compounds according to general formula (I) as given as follows and/or racemates, enantiomers, diastereomers, solvates, hydrates, and pharmaceutically acceptable salts and/or esters thereof: 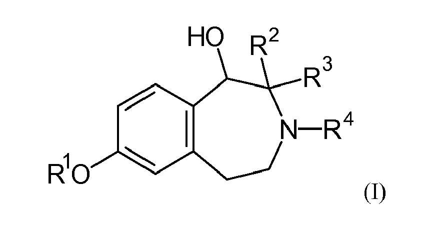 wherein:
- R¹: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; and/or C₇- C₁₄₋arylalkyl wherein the aryl group has 6 to 10 carbon atoms and the alkyl group has 1 to 4 carbon atoms;
- R²: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms;

- R³: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms; linear or branched alkyl groups of the type -CₙH₂ₙ-U-D wherein n is 1, 2, 3 or 4, U is selected from the group comprising O, CO, COO, CONH, S, guanidine and/or NH and D is selected from the group comprising H and/or C1-C₃-alkyl; -CH₂-C₆H₄-X wherein X is selected from the group comprising OH, SH, C₁-C₃-alkyl and/or NH₂; -CH₂-imidazole; -CH₂-indole; -CH₂-(furanyl-3-yl); -CH₂- (pyridyl-3-yl) and/or -CH₂-(imidazolyl-3-yl);
- or R² and R³: together with the carbon atom to which they are attached form a 5- to 7-membered non aromatic carbocycle or heterocycle comprising from 1 to 3 hetero atoms selected from the group comprising O, N and/or S;
- R⁴: is selected from the group comprising hydrogen; C₁-C₁₀-alkyl; -W and/or -Y-Z;
- Y: is selected from the group comprising C₁-C₆-alkyl; C₂-C₆-alkenyl; C₂-C₆-alkynyl, C₃-C₆-cycloalkyl; C₄-C₁₀-cycloalkylalkyl wherein the cycloalkyl group has 3 to 6 carbon atoms and the alkyl group has 1 to 4 carbon atoms; C₆-C₁₀-aryl; C₇-C₁₄-arylalkyl wherein the aryl group has 6 to 10 carbon atoms and the alkyl group has 1 to 4 carbon atoms; C₁-C₆-alkyl comprising at least one moiety independently selected from the group comprising oxygen, sulphur, SO, SO₂, CO, NH, N(C₁-C₃-alkyl) and/or T; a 3- to 6-membered aromatic or non aromatic carbocycle or heterocycle containing at least one of O, N or S as heteroatoms; and/or a structural element comprising a 3- to 6-membered aromatic or non aromatic carbocycle or heterocycle comprising from 1 to 3 heteroatoms selected from the group comprising O, N and/or S and a group selected from the group comprising oxygen, sulphur, SO, SO₂, CO, NH, N(C₁-C₃-alkyl) and/or C₁-C₆-alkyl comprising at least one moiety independently selected from the group comprising oxygen, sulphur, SO, SO₂, CO, NH and/or N(C₁-C₃-alkyl);
- T: is selected from the group comprising 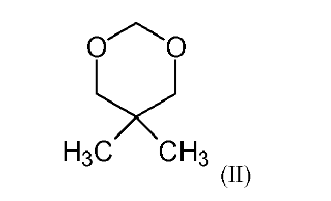 W is selected from the group comprising 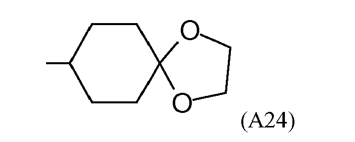
- Z: is selected from the group comprising mono-, bi- or tricyclic aromatic or non aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl;
- or R³ and R⁴: together with the ring atoms to which they are attached form a 5- to 7-membered non aromatic heterocycle comprising from 1 to 3 heteroatoms selected from the group comprising O, N and/or S for use as a medicament.

Another aspect of the present invention relates to the compounds according to the invention and/or racemates, enantiomers, diastereomers, solvates, hydrates, and pharmaceutically acceptable salts and/or esters thereof for use in the treatment of therapeutic and/or prophylactic treatment of a disease selected from the group comprising neuropathological, neuropsychiatric and/or neurodegenerative diseases selected from the group comprising chronic or acute pain, stroke preferably ischemic or hemorrhagic stroke, ischemic brain injury, traumatic brain injury, Parkinson's disease, Alzheimer's disease, Huntington's disease, depression, amyotrophic lateral sclerosis, schizophrenia, anxiety, migraine, epilepsy, addictive disorders, alcohol abuse, intoxication disorders, nicotine abuse, psychoactive substances abuse, withdrawal syndromes, nicotine addiction, cocaine addiction, alcohol and amphetamine addiction, attention deficit hyperactivity disorder, cognition impairment, anxiety disorders, generalized anxiety disorder, panic attacks, panic disorder, bipolar disorder, manic depression, manic-depressive disorder, behavioral disturbances, obsessive compulsive disorders, posttraumatic stress disorder, acute stress disorder, social phobia, simple phobias, pre-menstrual dysphoric disorder, cognitive memory disorders, learning disorders, social anxiety disorder, major depressive disorder, postnatal depression, dysthymia, depression associated with Alzheimer's disease, Parkinson's disease, or psychosis, eating disorders, obesity, anorexia nervosa, bulimia nervosa, binge eating disorder, analgesia, Lesch-Nyhan syndrome, neurodegenerative diseases, late luteal phase syndrome or narcolepsy, psychiatric symptoms, anger, rejection sensitivity, movement disorders, extrapyramidal syndrome, Tic disorders, restless leg syndrome, tardive dyskinesia, supranuclear palsy, sleep disorders, sleep related eating disorder, night eating syndrome, stress urinary incontinence, migraine, neuropathic pain, diabetic neuropathy, fibromyalgia syndrome, chronic fatigue syndrome, sexual dysfunction, premature ejaculation, male impotence, and/or thermoregulatory disorders, and/or for use in a neuroprotective treatment.

Preferred diseases are selected from the group comprising chronic or acute pain, stroke preferably ischemic or hemorrhagic stroke, ischemic brain injury, traumatic brain injury, Parkinson's disease, Alzheimer's disease, Huntington's disease, depression, amyotrophic lateral sclerosis, schizophrenia, anxiety, migraine, addictive disorders, alcohol abuse, intoxication disorders, nicotine abuse, psychoactive substances abuse, withdrawal syndromes, nicotine addiction, cocaine addiction, alcohol and/or amphetamine addiction.

The present invention also relates to the use of compounds according to general formula (I) as given as follows and/or racemates, enantiomers, diastereomers, solvates, hydrates, and pharmaceutically acceptable salts and/or esters thereof: 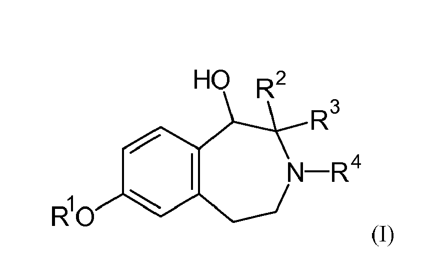 wherein:
- R¹: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; and/or C₇- C₁₄-arylalkyl wherein the aryl group has 6 to 10 carbon atoms and the alkyl group has 1 to 4 carbon atoms;
- R²: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms;
- R³: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms; linear or branched alkyl groups of the type -CₙH₂ₙ-U-D wherein n is 1, 2, 3 or 4, U is selected from the group comprising O, CO, COO, CONH, S, guanidine and/or NH and D is selected from the group comprising H and/or C₁-C₃-alkyl; -CH₂-C₆H₄-X wherein X is selected from the group comprising OH, SH, C₁-C₃-alkyl and/or NH₂; -CH₂-imidazole; -CH₂-indole; -CH₂-(furanyl-3-yl); -CH₂- (pyridyl-3-yl) and/or -CH₂-(imidazolyl-3-yl);
- or R² and R³: together with the carbon atom to which they are attached form a 5- to 7-membered non aromatic carbocycle or heterocycle comprising from 1 to 3 hetero atoms selected from the group comprising O, N and/or S;
- R⁴: is selected from the group comprising hydrogen; C₁-C₁₀-alkyl; -W and/or -Y-Z;
- Y: is selected from the group comprising C₁-C₆-alkyl; C₂-C₆-alkenyl; C₂-C₆-alkynyl, C₃-C₆-cycloalkyl; C₄-C₁₀-cycloalkylalkyl wherein the cycloalkyl group has 3 to 6 carbon atoms and the alkyl group has 1 to 4 carbon atoms; C₆-C₁₀-aryl; C₇-C₁₄-arylalkyl wherein the aryl group has 6 to 10 carbon atoms and the alkyl group has 1 to 4 carbon atoms; C₁-C₆-alkyl comprising at least one moiety independently selected from the group comprising oxygen, sulphur, SO, SO₂, CO, NH, N(C₁-C₃-alkyl) and/or T; a 3- to 6-membered aromatic or non aromatic carbocycle or heterocycle containing at least one of O, N or S as heteroatoms; and/or a structural element comprising a 3- to 6-membered aromatic or non aromatic carbocycle or heterocycle comprising from 1 to 3 heteroatoms selected from the group comprising O, N and/or S and a group selected from the group comprising oxygen, sulphur, SO, SO₂, CO, NH, N(C₁-C₃-alkyl) and/or C₁-C₆-alkyl comprising at least one moiety independently selected from the group comprising oxygen, sulphur, SO, SO₂, CO, NH and/or N(C₁-C₃-alkyl);
- T: is selected from the group comprising 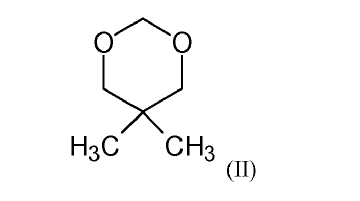 W is selected from the group comprising 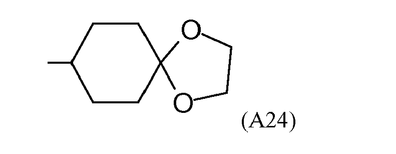
- Z: is selected from the group comprising mono-, bi- or tricyclic aromatic or non aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl;
- or R³ and R⁴: together with the ring atoms to which they are attached form a 5- to 7-membered non aromatic heterocycle comprising from 1 to 3 heteroatoms selected from the group comprising O, N and/or S, for the manufacture of a medicament.

In preferred embodiments, the present invention relates to the use of compounds according to general formula (III) as given as follows: 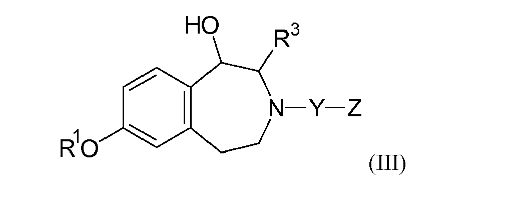 wherein:
- R¹: is selected from the group comprising hydrogen; linear or branched C₁-C₆-alkyl and/or benzyl;
- R³: is a side chain of an amino acid selected from the group comprising hydrogen; linear or branched C₁-C₄-alkyl; linear or branched alkyl groups of the type -CₙH₂ₙ-U-D wherein n is 1, 2, 3 or 4, U is selected from the group comprising O, CO, COO, CONH, S, guanidine and/or NH, and D is selected from the group comprising H and/or methyl; -CH₂-C₆H₄-OH; -CH₂-imidazole and/or -CH₂-indole;
- Y: is selected from the group comprising
- Z: -(CH₂)ₘ- wherein m represents 3, 4 or 5; C₃-C₅-alkenyl; C₅-C₆-cycloalkyl; C₃-C₅-alkyl comprising at least one moiety independently selected from the group comprising oxygen, sulphur, SO, SO₂, CO, NH and/or N(C₁-C₃-alkyl); a 5- to 6-membered non aromatic carbocycle or heterocycle comprising from 1 to 3 heteroatoms selected from the group comprising O, N and/or S; and/or a structural element comprising a 5- to 6-membered non aromatic carbocycle and a group selected from the group comprising oxygen, sulphur, SO, SO₂, CO, NH, N(C₁-C₃- alkyl) and/or C₁-C₃-alkyl comprising at least one moiety independently selected from the group comprising oxygen, sulphur, SO, SO₂, CO, NH and/or N(C₁-C₃-alkyl); is selected from the group comprising mono-, bi- or tricyclic aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl, and/or racemates, enantiomers, diastereomers, solvates, hydrates, and pharmaceutically acceptable salts and/or esters thereof for the manufacture of a medicament.

Advantageously, especially a neuroprotective effect can be achieved by administering a compound according to the invention.

Preferably, compounds according to the formulas (1) to (30) and/or racemates, enantiomers, diastereomers, solvates, hydrates, and pharmaceutically acceptable salts and/or esters thereof are usable for the manufacture of a medicament.

A further aspect of the present invention relates to the use of compounds according to the present invention and/or racemates, enantiomers, diastereomers, solvates, hydrates, and pharmaceutically acceptable salts and/or esters thereof as a medicament.

The compounds according to the present invention are usable in form of mixtures of stereoisomers as well as isolated specific stereoisomers. Further, the compounds according to the present invention are usable in form of solvates, hydrates, and pharmaceutically acceptable salts and/or esters thereof.

Preferably, pharmaceutically acceptable salts of the compounds according to the present invention are usable. The term "pharmaceutically acceptable salts" refers to salts prepared from pharmaceutically acceptable non-toxic bases or acids.

When the compound of the present invention is acidic, its corresponding salt can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particularly preferred are citric, hydrobromic, hydrochloric, maleic, phosphoric, sulfuric, and tartaric acids.

Without being bound to a specific theory, it is supposed that the compounds according to the present invention are specifically usable to treat a disease for which an NMDA NR2B receptor antagonist is indicated.

In preferred embodiments the compounds according to the present invention are specifically usable to treat neurological disorders.

Advantageously, the compounds according to the present invention can exhibit neuroprotective activity.

A further aspect of the present invention relates to the use of compounds according to the invention and/or racemates, enantiomers, diastereomers, solvates, hydrates, and pharmaceutically acceptable salts and/or esters thereof for the manufacture of a medicament for therapeutic and/or prophylactic treatment of a disease selected from the group comprising neuropathological, neuropsychiatric and/or neurodegenerative diseases selected from the group comprising chronic or acute pain, stroke preferably ischemic or hemorrhagic stroke, ischemic brain injury, traumatic brain injury, Parkinson's disease, Alzheimer's disease, Huntington's disease, depression, amyotrophic lateral sclerosis, schizophrenia, anxiety, migraine, epilepsy, addictive disorders, alcohol abuse, intoxication disorders, nicotine abuse, psychoactive substances abuse, withdrawal syndromes, nicotine addiction, cocaine addiction, alcohol and amphetamine addiction, attention deficit hyperactivity disorder, cognition impairment, anxiety disorders, generalized anxiety disorder, panic attacks, panic disorder, bipolar disorder, manic depression, manic-depressive disorder, behavioral disturbances, obsessive compulsive disorders, posttraumatic stress disorder, acute stress disorder, social phobia, simple phobias, pre-menstrual dysphoric disorder, cognitive memory disorders, learning disorders, social anxiety disorder, major depressive disorder, postnatal depression, dysthymia, depression associated with Alzheimer's disease, Parkinson's disease, or psychosis, eating disorders, obesity, anorexia nervosa, bulimia nervosa, binge eating disorder, analgesia, Lesch-Nyhan syndrome, neurodegenerative diseases, late luteal phase syndrome or narcolepsy, psychiatric symptoms, anger, rejection sensitivity, movement disorders, extrapyramidal syndrome, Tic disorders, restless leg syndrome, tardive dyskinesia, supranuclear palsy, sleep disorders, sleep related eating disorder, night eating syndrome, stress urinary incontinence, migraine, neuropathic pain, diabetic neuropathy, fibromyalgia syndrome, chronic fatigue syndrome, sexual dysfunction, premature ejaculation, male impotence, and/or thermoregulatory disorders, and/or for neuroprotective treatment.

The term "prophylactic treatment" of a disease according to the invention is to be understood as meaning that the compositions according to the invention can be applied before symptoms of the disease are manifest. Especially, the term "prophylactic treatment" of a disease is to be understood as meaning a medical treatment.

Advantageously, the compounds according to the invention can be especially useful for the treatment of diseases for which an NMDA NR2B receptor antagonist is indicated selected from the group comprising chronic or acute pain, stroke preferably ischemic or hemorrhagic stroke, ischemic brain injury, traumatic brain injury, Parkinson's disease, Alzheimer's disease, Huntington's disease, depression, schizophrenia, anxiety, migraine, addictive disorders, alcohol abuse, intoxication disorders, nicotine abuse, psychoactive substances abuse, withdrawal syndromes, nicotine addiction, cocaine addiction, alcohol and/or amphetamine addiction.

Thus, in preferred embodiments the disease is selected from the group comprising chronic or acute pain, stroke preferably ischemic or hemorrhagic stroke, ischemic brain injury, traumatic brain injury, Parkinson's disease, Alzheimer's disease, Huntington's disease, depression, schizophrenia, anxiety, migraine, addictive disorders, alcohol abuse, intoxication disorders, nicotine abuse, psychoactive substances abuse, withdrawal syndromes, nicotine addiction, cocaine addiction, alcohol and/or amphetamine addiction.

For example, the compounds according to the invention and/or racemates, enantiomers, diastereomers, solvates, hydrates, and pharmaceutically acceptable salts and/or esters thereof are usable as antipsycotics, anti depressants, anxiolytics, antiepiletic drugs and/or neuroprotective drugs.

A further aspect of the present invention relates to a pharmaceutical composition comprising as an active ingredient a compound according to general formula (I) as given as follows and/or racemates, enantiomers, diastereomers, solvates, hydrates, and pharmaceutically acceptable salts and/or esters thereof: 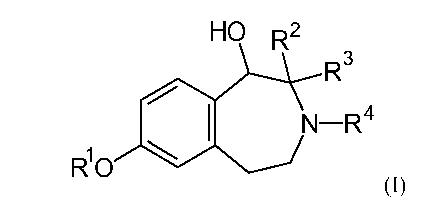 wherein:
- R¹: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; and/or C₇- C₁₄₋arylalkyl wherein the aryl group has 6 to 10 carbon atoms and the alkyl group has 1 to 4 carbon atoms;
- R²: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms;
- R³: is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms; linear or branched alkyl groups of the type -CₙH₂ₙ-U-D wherein n is 1, 2, 3 or 4, U is selected from the group comprising O, CO, COO, CONH, S, guanidine and/or NH and D is selected from the group comprising H and/or C₁-C₃-alkyl; -CH₂-C₆H₄-X wherein X is selected from the group comprising OH, SH, C₁-C₃-alkyl and/or NH₂; -CH₂-imidazole; -CH₂-indole; -CH₂-(furanyl-3-yl); -CH₂- (pyridyl-3-yl) and/or -CH₂-(imidazolyl-3-yl);
- or R² and R³: together with the carbon atom to which they are attached form a 5- to 7-membered non aromatic carbocycle or heterocycle comprising from 1 to 3 hetero atoms selected from the group comprising O, N and/or S;
- R⁴: is selected from the group comprising hydrogen; C₁-C₁₀-alkyl; -W and/or -Y-Z;
- Y: is selected from the group comprising C₁-C₆-alkyl; C₂-C₆-alkenyl; C₂-C₆-alkynyl, C₃-C₆-cycloalkyl; C₄-C₁₀-cycloalkylalkyl wherein the cycloalkyl group has 3 to 6 carbon atoms and the alkyl group has 1 to 4 carbon atoms; C₆-C₁₀-aryl; C₇-C₁₄-arylalkyl wherein the aryl group has 6 to 10 carbon atoms and the alkyl group has 1 to 4 carbon atoms;

- T: C₁-C₆-alkyl comprising at least one moiety independently selected from the group comprising oxygen, sulphur, SO, SO₂, CO, NH, N(C₁-C₃-alkyl) and/or T; a 3- to 6-membered aromatic or non aromatic carbocycle or heterocycle containing at least one of O, N or S as heteroatoms; and/or a structural element comprising a 3- to 6-membered aromatic or non aromatic carbocycle or heterocycle comprising from 1 to 3 heteroatoms selected from the group comprising O, N and/or S and a group selected from the group comprising oxygen, sulphur, SO, SO₂, CO, NH, N(C₁-C₃-alkyl) and/or C₁-C₆-alkyl comprising at least one moiety independently selected from the group comprising oxygen, sulphur, SO, SO₂, CO, NH and/or N(C₁-C₃-alkyl); is selected from the group comprising 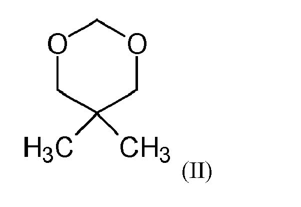 W is selected from the group comprising 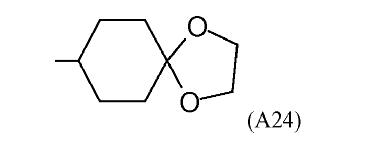
- Z: is selected from the group comprising mono-, bi- or tricyclic aromatic or non aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl;
- or R³ and R⁴: together with the ring atoms to which they are attached form a 5- to 7-membered non aromatic heterocycle comprising from 1 to 3 heteroatoms selected from the group comprising O, N and/or S.

In preferred embodiments, the present invention relates to a pharmaceutical composition comprising as an active ingredient compounds according to general formula (III) as given as follows: 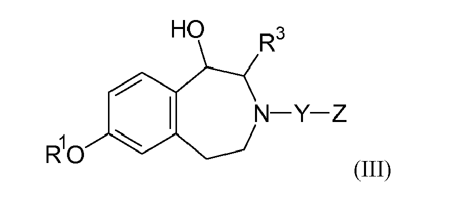 wherein:
- R¹: is selected from the group comprising hydrogen; linear or branched C₁-C₆-alkyl and/or benzyl;
- R³: is a side chain of an amino acid selected from the group comprising hydrogen; linear or branched C₁-C₄-alkyl; linear or branched alkyl groups of the type -CₙH₂ₙ-U-D wherein n is 1, 2, 3 or 4, U is selected from the group comprising O, CO, COO, CONH, S, guanidine and/or NH, and D is selected from the group comprising H and/or methyl; -CH₂-C₆H₄-OH; -CH₂-imidazole and/or -CH₂-indole;
- Y: is selected from the group comprising -(CH₂)ₘ- wherein m represents 3, 4 or 5; C₃-C₅-alkenyl; C₅-C₆-cycloalkyl; C₃-C₅-alkyl comprising at least one moiety independently selected from the group comprising oxygen, sulphur, SO, SO₂, CO, NH and/or N(C₁-C₃-alkyl); a 5- to 6-membered non aromatic carbocycle or heterocycle comprising from 1 to 3 heteroatoms selected from the group comprising O, N and/or S; and/or a structural element comprising a 5- to 6-membered non aromatic carbocycle and a group selected from the group comprising oxygen, sulphur, SO, SO₂, CO, NH, N(C₁-C₃- alkyl) and/or C₁-C₃-alkyl comprising at least one moiety independently selected from the group comprising oxygen, sulphur, SO, SO₂, CO, NH and/or N(C₁-C₃-alkyl);
- Z: is selected from the group comprising mono-, bi- or tricyclic aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl racemates, enantiomers, diastereomers, solvates, hydrates, and pharmaceutically acceptable salts and/or esters thereof.

Preferably, the present invention relates to a pharmaceutical composition comprising as an active ingredient compounds according to the formulas (1) to (30) and/or racemates, enantiomers, diastereomers, solvates, hydrates, and pharmaceutically acceptable salts and/or esters thereof usable for the manufacture of a medicament.

The compounds and/or racemates, enantiomers, diastereomers, solvates, hydrates, and pharmaceutically acceptable salts and/or esters thereof can be included in pharmaceutical compositions in combination with one or more other therapeutically active compounds.

Preferred pharmaceutical compositions are usable for the therapeutic and/or prophylactic treatment of a disease selected from the group comprising neuropathological, neuropsychiatric and/or neurodegenerative diseases, preferably selected from the group comprising chronic or acute pain, stroke preferably ischemic or hemorrhagic stroke, ischemic brain injury, traumatic brain injury, Parkinson's disease, Alzheimer's disease, Huntington's disease, depression, amyotrophic lateral sclerosis, schizophrenia, anxiety, migraine, addictive disorders, alcohol abuse, intoxication disorders, nicotine abuse, psychoactive substances abuse, withdrawal syndromes, nicotine addiction, cocaine addiction, alcohol and/or amphetamine addiction.

Preferably, the pharmaceutical compositions of the present invention comprise a compound according to the invention and/or racemates, enantiomers, diastereomers, solvates, hydrates, and pharmaceutically acceptable salts and/or esters thereof as an active ingredient, a pharmaceutically acceptable carrier and optionally other therapeutic ingredients or adjuvants.

The pharmaceutical carrier can be, for example, a solid, liquid, or gas. Suitable carriers and adjuvants can be solid or liquid and correspond to the substances ordinarily employed in formulation technology for pharmaceutical formulations. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

The compositions can be suitable for oral, dermal, rectal, topical, and parenteral administration. Parenteral administration includes subcutaneous, intramuscular, and intravenous administration. The pharmaceutical compositions may be conveniently presented in unit dosage form and prepared by any of the methods well known in the art of pharmacy.

The pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion or as a water-in-oil liquid emulsion. Further, the pharmaceutical composition may be administered by controlled release means and/or delivery devices.

For compositions for oral dosage form, convenient pharmaceutical media may be employed. For example, water, glycols, oils, alcohols, flavoring agents, preservatives, coloring agents and the like may be used to form oral liquid preparations such as solutions. Carriers such as starches, sugars, microcrystalline cellulose, diluents, granulating agents, lubricants, binders, disintegrating agents, and the like may be used to form oral solid preparations such as powders, capsules and tablets. Optionally, tablets may be coated by standard aqueous or non aqueous techniques.

Pharmaceutical compositions of the present invention suitable for parenteral administration may be prepared as solutions or suspensions of the active compounds in water. A suitable excipient can be included such as, for example, hydroxypropylcellulose. Dispersions can also be prepared in glycerol, liquid polyethylene glycols, and mixtures thereof in oils. Pharmaceutical compositions of the present invention suitable for injectable use include sterile aqueous solutions or dispersions. Further, a preservative can be included to prevent the growth of microorganisms.

The pharmaceutical compositions of the present invention can be in a form suitable for topical use such as, for example, an aerosol, cream, ointment, lotion, dusting powder, or the like. Further, the compositions can be in a form suitable for use in transdermal devices. The pharmaceutical composition may also be prepared in powder or liquid concentrate form. The pharmaceutical composition of the present invention can include one or more additional carrier ingredients such as diluents, buffers, flavoring agents, binders, surface-active agents, thickeners, lubricants, preservatives and the like.

The pharmaceutical composition may be produced under sterile conditions using standard pharmaceutical techniques well known to those skilled in the art.

The compounds of the present invention may be prepared using a variety of processes well known by a person skilled in the art.

In a preferred embodiment, the compounds of the present invention wherein R³ is not hydrogen may be prepared by a method comprising the steps of:
a) Preparation of a 2-(3-alkoxyphenyl)ethanol;
b) Esterification of the COOH group of an amino acid other than glycine;
c) Introduction of a protecting group to the nitrogen of the esterified amino acid of step b);
d) Nucleophilic substitution of the protected amino acid of step c) with 2-(3-alkoxyphenyl)ethanol of step a);
e) Alkaline hydrolysis of the alkyl ester of the amino acetate of step d) to the respective carboxylic acid;
f) Cyclisation of the carboxylic acid of step e) by a Friedel-Crafts acylation;
g) Reduction of the ketone product of step f) to the respective alcohol;
h) Elimination of the protecting group from the nitrogen and thereby obtaining the secondary amine of the compound of step g);
i) Introduction of a group R⁴ to the nitrogen of the compound of step h) by alkylation;
j) optionally hydrogenolytic elimination of the alkoxy group of step a) to receive a group R¹ that is hydrogen.

### Unless otherwise indicated, R¹, R², R³, and R⁴ are as defined above.

Referring to this method, R³ preferably is selected from the group comprising hydrogen; linear or branched C₁-C₈-alkyl; C₂-C₈-alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄-arylalkyl wherein the aryl group has 6 to 10 carbon atoms and the alkyl group has 1 to 4 carbon atoms; linear or branched alkyl groups of the type -CₙH₂ₙ-U-D wherein n is 1, 2, 3 or 4, U is selected from the group comprising O, CO, COO, CONH, S, guanidine and/or NH and D is selected from the group comprising H and/or C₁-C₃-alkyl; -CH₂-C₆H₄-X wherein X is selected from the group comprising OH, SH, C₁-C₃-alkyl and/or NH₂; -CH₂-imidazole; -CH₂-indole; -CH₂-(furanyl-3-yl); -CH₂-(pyridyl-3-yl) and/or -CH₂-(imidazolyl-3-yl).

The Preparation of a 2-(3-alkoxyphenyl)ethanol of step a) preferably is a reaction of 1-bromo-4-methoxybenzene with ethylene sulfate to 2-(3-alkoxyphenyl)ethanol or a selective benzylation of 3-(2-hydroxyethyl)phenol to 2-(3-benzyloxyphenyl)ethanol.

The alkoxy group of a 2-(3-alkoxyphenyl)ethanol of step a) preferably is selected from the group comprising C₁-C₆-alkyloxy and/or benzyloxy, preferably is C₁-C₆-alkyloxy. A benzylation is preferred in embodiments when R¹ is benzyl or hydrogen and the benzyl group will be replaced by hydrogen in a later step.

The esterification of step b) preferably takes place using methanol and SOCl₂. Preferably, the esterification of step b) is carried out in the presence of an acid, preferably sulphuric acid.

The amino acid of step b) is an amino acid other than glycine since the method provides for compounds wherein R³ is not hydrogen. The amino acid preferably is a naturally occurring amino acid selected from the group comprising alanine, serine, threonine, arginine, lysine, aspartic acid, glutamic acid, asparagine, glutamine, phenylalanine, tyrosine, tryptophan, leucine, valine, isoleucine, cysteine, methionine, histidine and/or proline.

Preferably, if the amino acids have a functional group such as an hydroxyl or sulfhydryl group the functional groups are protected by a protecting group.

The amino acid of step b) can have a chiral center usually assigned by a prefix R or S, according to whether its configuration is right- or left-handed. Accordingly, the amino acid of step b) for example can be the R- or S-enantiomer of alanine. Advantageously, using an amino acid having a selected chiral center results in compounds having a selected chirality.

In step c) a group as a protecting group is introduced to the nitrogen of the esterified amino acid of step b) thereby replacing one of the hydrogen atoms of the primary amine by a protecting group. The protecting group preferably is selected from the group comprising sulfonic esters such as tosylates, mesylates, and trifluoromethanesulfonates (triflates). Preferably, step c) is a tosylation of the nitrogen of the esterified amino acid of step b). Preferably, the tosylation is carried out in the presence of a base, preferably selected from the group comprising pyridine, triethylamine and/or diisopropylamine.

In step d) a nucleophilic substitution of the protected amino acid, for example with a tosylate group, with 2-(3-alkoxyphenyl)ethanol of step a) is carried out. Preferably, the nucleophilic substitution is a Mitsunobu reaction. The Mitsunobu Reaction allows the conversion of the primary alcohol group of the 2-(3-alkoxyphenyl)ethanol into an amine by nucleophilic substitution of the amino acid. The Mitsunobu reaction preferably is carried out using triphenylphosphine and diethyl azodicarboxylate (DEAD). A preferred solvent is tetrahydrofuran.

The alkaline hydrolysis of the alkyl ester of the amino acetate of step d) to the respective carboxylic acid in step e) preferably is carried out in the presence of LiOH. A preferred solvent is a mixture of tetrahydrofuran and water.

The cyclisation of the carboxylic acid by a Friedel-Crafts acylation in step f) preferably is carried out in the presence of 2,2,2-Trifluoroacetamide and stannous chloride (SnCl₂) or in the presence of phosphorpentoxide. A preferred solvent is dichloromethane. The Friedel-Crafts acylation allows the cyclisation from a reaction between the arene moiety and an acyl chloride of the carboxylic acid moiety of the compound.

The reduction of the cyclised ketone product of step f) to the respective alcohol in step g) preferably is carried out with a mild reducing agent such as LiAIH₄ or NaBH₄, preferably with NaBH₄. A preferred solvent for the reduction is methanol.

In step h) the protecting group such as a tosylate, mesylate, and trifluoromethanesulfonate from the nitrogen can be eliminated thereby obtaining the secondary amine of the compound wherein R⁴ is hydrogen. The elimination can be carried out using magnesium in methanol.

For embodiments of the compound wherein R⁴ is not hydrogen but selected from the group comprising C₁-C₁₀-alkyl, -W and/or -Y-Z the group R⁴ is introduced to the nitrogen by an alkylation reaction. The alkylation can be carried out in the presence of help-bases. Preferred help-bases are selected from the group comprising sodium carbonate, sodium hydrogen carbonate, potassium carbonate, and/or potassium hydrogen carbonate.

Optionally, especially when the respective group R¹ should be hydrogene, the alkyoxy group of step a) can undergo hydrogenolytic elimination to receive a group R¹ that is hydrogen.

In a preferred embodiment, the compounds of the present invention wherein R² and R³ is hydrogen may be prepared by a method comprising the steps of:
1) Introduction of a protecting group to the nitrogen of 2-(3-alkoxyphenyl)ethane amine;
2) Nucleophilic substitution of the protected amine of step 1) with ethyl bromoacetate;
3) Alkaline hydrolysis of the ethyl ester of the amino acetate of step 2) to the respective carboxylic acid;
4) Cyclisation of the carboxylic acid of step 3) by a Friedel-Crafts acylation;
5) optionally Splitting the alkyl ether of the compound of step 4) and receiving the phenol;
6) optionally Benzylation of the phenol of step 5);
7) Reduction of the ketone product of step 4) or step 6) to the respective alcohol;
8) Elimination of the protecting group from the nitrogen and thereby obtaining the secondary amine of the compound of step 7);
9) Introduction of a group R⁴ to the nitrogen of the compound of step 8) by alkylation or acylation;
10) optionally hydrogenolytic elimination of the benzyl group to receive a group R¹ that is hydrogen.

The alkoxy group of the 2-(3-alkoxyphenyl)ethane amine of step 1) preferably is selected from the group comprising C₁-C₆-alkyloxy and/or benzyloxy, preferably C₁-C₆-alkyloxy.

In step 1) a group as a protecting group is introduced to the nitrogen of the 2-(3-alkoxyphenyl)ethane amine, thereby replacing one of the hydrogen atoms of the primary amine group by a protecting group. The protecting group preferably is selected from the group comprising sulfonic esters such as tosylates, mesylates, and trifluoromethanesulfonates (triflates). Preferably, step 1) is a tosylation of the nitrogen. Preferably, the tosylation is carried out in the presence of a base, preferably selected from the group comprising pyridine, triethylamine and/or diisopropylamine.

In step 2) a nucleophilic substitution of the protected amine, for example with a tosylate group, of step 1) with ethyl bromoacetate is carried out. Preferably, the nucleophilic substitution is carried out using K₂CO₃.

In step 3), the alkaline hydrolysis of the ethyl ester of the amino acetate to the respective carboxylic acid preferably is carried out in the presence of sodium hydroxide in ethanol.

The cyclisation of the carboxylic acid by a Friedel-Crafts acylation in step 4) preferably is carried out in the presence of of phosphorpentoxide. A preferred solvent is dichloromethane. The Friedel-Crafts acylation allows the cyclisation from a reaction between the arene moiety and an acyl chloride of the carboxylic acid moiety of the compound.

Optionally, especially when the respective group R¹ should be hydrogene, the alkyl ether of the compound of step 4) can become splitted to receive the phenol thereby having a group R¹ that is hydrogen. The splitting of the alkyl ether preferably is carried out in the presence of aluminium chloride. A preferred solvent is dichloromethane.

However, to protect the hydroxyl group, in a next optional step the phenol of step 5) is benzylated. The benzylation of the phenol preferably is carried out using benzyl bromide and potassium carbonate.

The reduction of the cyclised ketone product of step 4) or step 6) to the respective alcohol in step 7) preferably is carried out with a mild reducing agent such as LiAIH₄ or NaBH₄, preferably with NaBH₄. A preferred solvent for the reduction is methanol.

In step 8) the protecting group such as a tosylate, mesylate, and trifluoromethanesulfonate from the nitrogen can be eliminated thereby obtaining the secondary amine of the compound wherein R⁴ is hydrogen. The elimination can be carried out using magnesium in methanol.

For embodiments of the compound wherein R⁴ is not hydrogen but selected from the group comprising C₁-C₁₀-alkyl, -W and/or -Y-Z the group R⁴ is introduced to the nitrogen by an alkylation or an acylation reaction in step 9). The alkylation can be carried out in the presence of help-bases. Preferred help-bases are selected from the group comprising sodium carbonate, sodium hydrogen carbonate, potassium carbonate, and/or potassium hydrogen carbonate.

Optionally, especially when the respective group R¹ should be hydrogen, the benzyl group introduced optionally in step 6) can undergo hydrogenolytic elimination to receive a group R¹ that is hydrogen. The Examples which follow serve to illustrate the invention in more detail but do not constitute a limitation thereof.

Unless stated otherwise, chemical reactions with substances sensitive towards hydrolysis or oxidation were carried out in water free glass equipment under inert gas. Nitrogen (Air Liquide, Düsseldorf, Germany) dried via a 5 Å molecular sieve was used as inert gas. For reactions at 4 °C cooling was achieved using a ice/water bath. For reactions at -78 °C cooling was achieved using a mixture of dry ice and acetone. If experiments were carried out at room or ambient temperature, that is, experiments were carried out at a temperature in the range of 18-25°C.

The solutions were used in per analysis quality. Tetrahydrofuran (THF) was distilled over sodium and benzophenone under nitrogen. Methanol (CH₃OH) was destilled over magnesium methanolate and stored on molecular sieve (0.3 nm). Dichlormethane (CH₂Cl₂) and dichlorethane were destilled over calcium hydride and stored on molecular sieve (0.4 nm).

A purification of compounds was carried out using flash column chromatography, a variant of column chromatography. Flash column chromatography was carried out using Merck silica gel 60 (40 - 63 µm). Pressure was achieved by nitrogen. The mobile phase, column diameter (∅), filling level of silica gel, and volume of fractions was adjusted to experimental conditions and is given in detail in the respective examples. Further indicated is the retention factor (R_{f} value).

### Example 1

### Preparation of 7-Methoxy-2,3,4,5-tetrahydro-1H-3-benzazepin-1-ol

step 1.1 Preparation of N-[2-(3-Methoxyphenyl)ethyl]-4-toluenesulfonamide 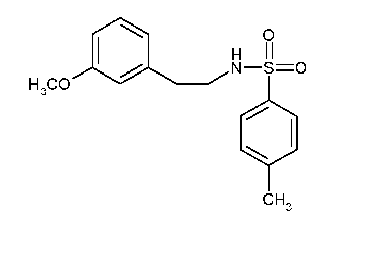 2-(3-Methoxyphenyl)ethane-1-amine (5.0 g, 33.0 mmol) was dissolved in dry pyridine (60 mL). To the stirred solution 4-toluenesulfonylchloride (25.2 g, 132.2 mmol) was added and the mixture stirred for 1.5 h at room temperature. The mixture was treated with water (90 mL) and extracted with CHCl₃ (3 x 50 mL). The combined organic layer was washed with 5 % HCl solution(4 x 50 mL) and H₂O (3 x 50 mL), dried over Na₂SO₄ and evaporated. The residue was purified by flash column chromatography on silica gel (eluting with hexane: ethyl acetate 7 : 3 and 2 % N,N-dimethylethaylmine, ∅ 8 cm, volume of fraction 50 mL, R_{f} = 0.28) to afford the titled compound as a light yellow solid.

### step 1.2 Preparation of 2- {N-[2-(3-Methoxyphenyl)ethyl]-N-(4-tosyl)-amino}acetate

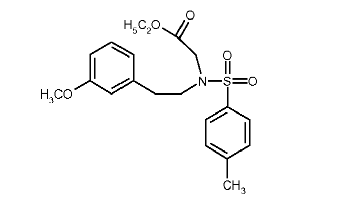

N-[2-(3-Methoxyphenyl)ethyl]-4-toluenesulfonamide of step 1.1 (5.0 g, 16.4 mmol) was dissolved in acetone (85 mL) and to the stirred solution ethylbromacetate (4.2 g, 24.9 mmol) and K₂CO₃ (15.6 g, 113.0 mmol) was added. The mixture was heated for 20 h under reflux. The precipitate was filtert and the solvent was evaporated. The residue was purified by flash column chromatography on silica gel (eluting with hexane: ethyl acetate 7 : 3 and 2 % N,N-dimethylethaylmine, ∅ 8 cm, Volume of fraction 50 mL, R_{f}= 0.51) to afford the titled compound as a light yellow solid.

### step 1.3 Preparation of 2- {N-[2-(3-Methoxyphenyl)ethyl]-N-(4-tosyl)amino} acetic acid

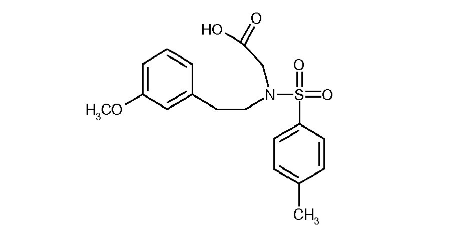

2-{*N*-[2-(3-Methoxyphenyl)ethyl]-*N*-(4-tosyl)-amino}acetate of step 1.2 (6.3 g,16.3 mmol) was dissolved in NaOH (2.9 g, 72.5 mmol) and 50 % ethanol (55 mL) and heated at reflux for 5h. Ethanol was evaporated followed by the addition of H₂O (30 mL) to the solution.

After extraction with diethyl ether (3 x 30 mL) the aqueous phase was acidified with conc. HCl and back extracted three times with diethyl ether (30 mL). The organic layer was extracted with 5% NaHCO₃ solution (30 mL) and the aqueous phase was acified with conc. HCl. After the acified aqueous phase was extracted three times with diethyl ether (30 mL), the organic layer was dried with Na₂S0₄ and concentrated in vacuum.

The residue was purified by flash chromatography (CH₂Cl₂ : CH₃OH 9.5 : 0.5 and 2 % *N,N-*Dimethylethanamine, ∅ 8 cm, Volume of fraction 50 mL, R_{f}= 0.62).

The titled compound was obtained as a colourless solid.

### step 1.4 Preparation of 7-Methoxy-3-(4-tosyl)-2,3,4,5-tetrahydro-3-benzazepine-1-one

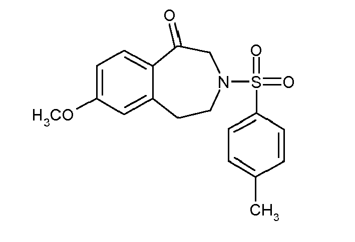

2-{*N*-[2-(3-Methoxyphenyl)ethyl]-*N*-(4-tosyl)amino} acetic acid (1.0 g, 2.76 mmol) from step 1.3 was dissolved in abs. dichlorethane (20 mL) and cooled under nitrogen to 0 °C. Afterwards P₂O₅ (1.96 g, 13.8 mmol) was added to the solution and the mixture was allowed to stirr for 24 h at 0 °C. Afterwards to the suspension was added 3% NaOH to gain a pH-value between pH 13-14 and then extracted three times with dichloromethane (40 mL)

The dichloromethane phase was washed three times with water (50 mL), dried with Na₂SO₄ and concentrated in vacuum. The residue was purified by flash chromatography (*n*-hexane: ethyl acetate 7 : 3 and 2 % N,N-dimethylethanamine, ∅ 4 cm, volume of fraction 50 mL, R_{f}= 0.23. The regioisomer 7-Methoxy-3-(4-tosyl)-2,3,4,5-tetrahydro-3-benzazepine-1-one was separated in ethanol by fractionated crystallization and obtained as a colourless solid.

### step 1.5 Preparation of 7-Methoxy-3-(4-tosyl)-2,3,4,5-tetrahydro-1H-3-benzazepine-1-ol

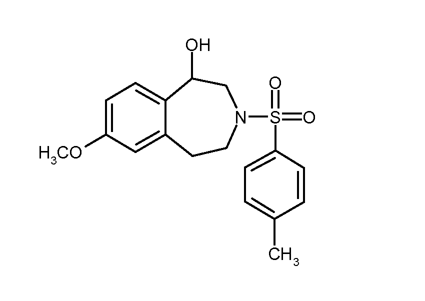

7-Methoxy-3-(4-tosyl)-2,3,4,5-tetrahydro-3-benzazepine-1-one (1.0 g, 2.90 mmol) resulting from step 1.4 was suspended in abs. CH₃OH (15 mL) and to the suspension NaBH₄ (0.230 g, 6.1 mmol) was added in several portions. After 2 h of stirring at room temperature the solvent was evaporated. The residue was combined with H₂O (30 mL) and exctracted with CHCl₃ (4 x 30 mL). The organic layer was washed three times with H₂O (40 mL) and after drying with Na₂SO₄ the solvent was evaporated. The residue was purified by flash chromatography (CH₂Cl₂ : diethyl ether 9.5 : 0,5, ∅ 6 cm, volume of fraction 50 mL, R_{f}= 0.15).
The titled compound was obtained as a colourless solid.

### step 1.6 Preparation of 7-Methoxy-2,3,4,5-tetrahydro-1H-3-benzazepine-1-ol

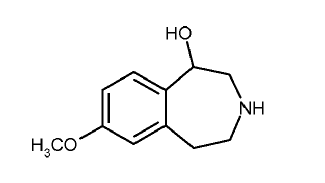

7-Methoxy-3-(4-tosyl)-2,3,4,5-tetrahydro-1*H*-3-benzazepine-1-ol (1.85 g, 5.33 mmol) resulting from step 1.5 was dissolved in abs. CH₃OH (100 mL) together with magnesium turnings (2.83 g, 0.12 mol). The mixture was heated at reflux for 5 h and afterwards mixed with conc. H₂SO₄ (6.55 mL) under ice-cooling. The mixture was filtered to get rid of residues and the filtrate was adjusted with NaOH to an alkaline pH-value between pH=9-10. The water phase was extracted five times with CH₂Cl₂ (30 mL) and dried with Na₂SO₄. The solvent was evaporated. The residue was purified by flash chromatography (CH₂Cl₂ : CH₃OH 19 : 1 and 2 % NH₃, ∅ 4.5 cm, volume of fraction 65 mL, R_{f}= 0.11). The titled compound was obtained as a colourless solid.

### Example 2

### 7-Methoxy-3-(4-phenylbutyl)-2,3,4,5,-tertahydro-1H-3-benzazepine-1-ol

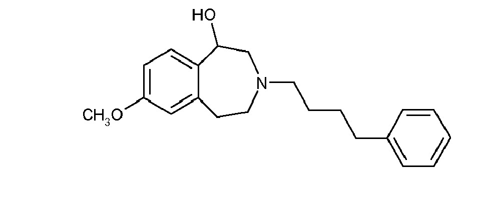

The compound 7-Methoxy-2,3,4,5-tetrahydro-1*H*-3-benzazepine-1-ol (100.0 mg, 0.52 mmol) synthesized according to step 1.6 was dissolved in CH₃CN (10 mL), TBAI (11.1 mg, 0.03 mmol), K₂CO₃ (575.0 mg, 4.16 mmol) and 1-chlor-4-phenylbutane (110.7 µL, 0.62 mmol). The suspension was heated at reflux for 48 h. Afterwards the K₂CO₃ was filtered and the solvent was evaporated. The residue was purified by flash chromatography (*n*-hexane: ethyl acetate 8 : 2 and 1 % *N,N-*dimethylethanamine, O 2 cm, fraction size 10 mL, R_{f} = 0,15).
The titled compound was obtained as a colourless solid.

### Example 3

### 7-Methoxy-3-octyl-2,3,4,5-tetrahydro-1H-3-benzazepine-1-ol

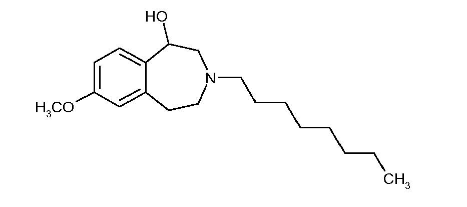

7-Methoxy-2,3,4,5-tetrahydro-1*H*-3-benzazepine-1-ol (100.0 mg, 0.52 mmol), synthesized according to step 1.6, was dissolved in CH₃CN (15 mL), subsequently mixed with K₂CO₃ (512 mg, 3.71 mmol) and 1-Bromoctan (80.5 µL, 0.55 mmol). The suspension was heated at reflux for 20 h. The precipitation was filtered and the solvent was removed in vacuum. The residue was purified by flash chromatography (n-hexane: ethyl acetate 7 : 3 and 1 % N,N-dimethylethanamine, 0 2 cm, fraction size 10 mL, R_{f}= 0.34). The titled compound was obtained as a colourless solid.

### Example 4

*trans*-7-Methoxy-3-(4-phenylcyclohexyl)-2,3,4,5-tetrahydro-1*H*-3-benzazepine-1-ol
and *cis*-7-Methoxy-3-(4-phenylcyclohexyl)-2,3,4,5-tetrahydro-1*H-*3-benzazepine-1-ol

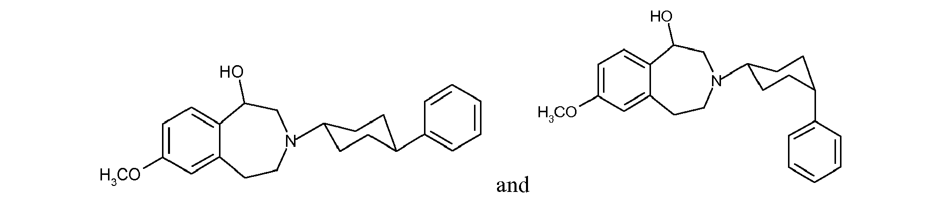

To a suspension of 7-Methoxy-2,3,4,5-tetrahydro-1*H*-3-benzazepine-1-ol (82.7 mg, 0.43 mmol), synthesized according to step 1.6, dichloroethane (2 mL) was added followed by the addition of phenylcyclohexanone (88.8 mg, 0.51 mmol), NaBH(OAc)₃ (136.1 mg, 0.64 mmol) and glacial acetic acid (36.6 µL, 0.64 mmol). The solution was stirred for 3 h at room temperature. Afterwards the solution was combined with saturated NaHCO₃ solution (10 mL) and H₂O (10 mL). The water phase was extracted with CH₂Cl₂. The combined organic layers were dried with Na₂SO₄ and the solvent was evaporated. The residue was purified by flash chromatography (*n*-hexane : ethyl acetate 7 : 3 and 1 % *N*,*N-*dimethylethanamine, ∅ 2 cm, fraction size 10 mL, R_{f}= 0.15).

The diasteromers *trans*-7-Methoxy-3-(4-phenylcyclohexyl)-2,3,4,5-tetrahydro-1*H*-3-benzazepine-1-ol and *cis*-7-Methoxy-3-(4-phenylcyclohexyl)-2,3,4,5-tetrahydro-1*H*-3-benzazepine-1-ol in a ratio trans : cis = 30 : 70, were obtained as a colourless oil.

### Example 5

### 7-Methoxy-3-(5-phenylpentyl)-2,3,4,5-tetrahydro-1H-3-benzazepine-1-ol

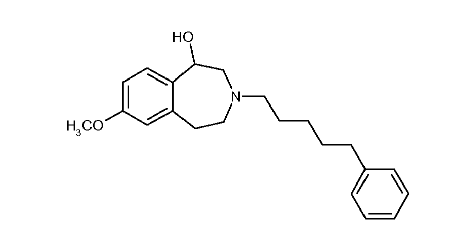

To the compound 7-Methoxy-2,3,4,5-tetrahydro-1*H*-3-benzazepine-1-ol synthesized (103.9 mg, 0.54 mmol), synthesized according to step 1.6, CH₃CN (10 mL), K₂CO₃ (431.2 mg, 3.12 mmol), TBAI (144.1 mg, 0.39 mmol) and 1-chlor-5-phenylpentan (104.5 µL, 0.58 mmol) were added. The suspension was heated at reflux for 39 h. Afterwards the K₂CO₃ was filtered and the solvent was evaporated. The residue was purified by flash chromatography (*n*-hexane: ethyl acetate 7 : 3 and 1 % *N*,*N-*dimethylethanamine, ∅ 3 cm, fraction size 30 mL, R_{f}= 0.28).
The titled compound was obtained as a colourless oil.

### Example 6

1-(4-Fluorphenyl)-4-(1-hydroxy-7-methoxy-2,3,4,5-tetrahydro-1*H* 3-benzazepine-3-yl)-butane-1-one 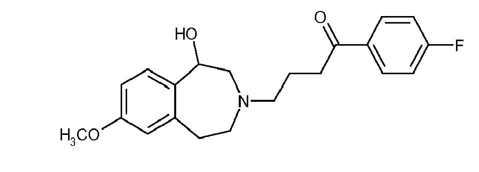 To the compound 7-Methoxy-2,3,4,5-tetrahydro-1*H*-3-benzazepine-1-ol (96.0 mg, 0.50 mmol), synthesized according to step 1.6, K₂CO₃ (552.8 mg, 4.0 mmol), CH₃CN (12 mL), TBAI (183.6 mg, 0.50 mmol) and 4-chloro-1-(4-fluorphenyl)butane-1-one (150.5 mg, 0.75 mmol) were added. The reaction mixture was heated at reflux for 72 h. The solvent was evaporated and the residue was purified by flash chromatography (*n*-hexane: ethyl acetate 6 : 4 and 1 % *N*,*N-*dimethylethanamine, ∅ 2 cm, fraction size 10 mL, R_{f}= 0,09).
The titled compound was obtained as a colourless resin.

### Example 7

Preparation of 7-Methoxy-3-[4-(phenylsulfanyl)butyl]-2,3,4,5-tetrahydro-1*H*-3-benzazepine-1-ol

### Step 7.1 Preparation of 1-Chloro-3-(phenylsulfanyl)propane

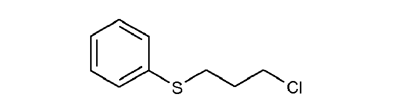

Thiophenol sodium salt (0.75 g, 5.68 mmol) was dissolved in a mixture consisting of dioxane (4 mL) and H₂O (8 mL), followed by the addition of NaOH (0.23 g, 5.68 mmol). To the solution was added dropwise bromo-4-chlorpropane (0.67 mL, 6.80 mmol) and heated for 24 h at 60 °C. The reaction mixture was diluted with H₂O (18 mL) and subsequently extracted with *n*-hexane (15 mL). The combined organic layers were treated with 2M NaOH (6 x 20 mL) und H₂O (2 x 20 mL). The organic phase was dried with Na₂SO₄ and the solvent was evaporated. The residue was purified by flash chromatography (*n*-hexane, ∅ 3 cm, fraction size 10 mL, Rf = 0.19). The titled compound was obtained as a pale yellow oil.

### step 7.2 Preparation of 1-Chloro-4-(phenylsulfanyl)butane

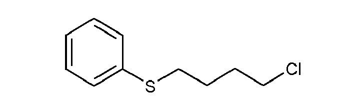

Thiophenol sodium salt (0.75 g, 5.68 mmol) was dissolved in a mixture consisting of dioxane (4 mL) and H₂O (8 mL), followed by the addition of NaOH (0.23 g, 5.68 mmol). To the solution was added dropwise bromo-4-chlorbutane (0.79 mL, 6.82 mmol) and heated for 24 h at 60 °C. The reaction mixture was diluted with H₂O (18 mL) and subsequently extracted with *n*-hexane (15 mL). The combined organic layers were treated with 2M NaOH (6 x 20 mL) und H₂O (2 x 20 mL). The organic phase was dried with Na₂SO₄ and the solvent was evaporated. The residue was purified by flash chromatography (*n*-hexane: ethyl acetate 9.5 : 0.5, ∅ 3 cm, fraction size 10 mL, Rf = 0.75). The titled compound was obtained as a pale yellow oil.

### step 7.3 Preparation of 7-Methoxy-3-[4-(phenylsulfanyl)butyl]-2,3,4,5-tetrahydro-1H-3-benzazepine-1-ol

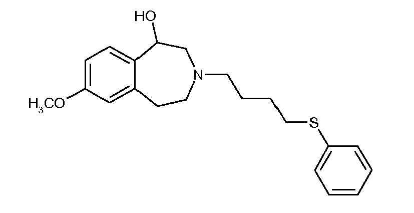

To 7-Methoxy-2,3,4,5-tetrahydro-1*H*-3-benzazepine-1-ol (87.7 mg, 0.45 mmol), synthesized according to step 1.6, CH₃CN (9 mL), TBAI (166.2 mg, 0.59 mmol), K₂CO₃ (497.5 mg, 3.60 mmol) and 1-chloro-4-(phenylsulfanyl)butane (117.8 mg, 0.59 mmol) resulting from step 7.2 were added. The suspension was heated at reflux for 8 h. Afterwards the K₂CO₃ was filtered and the solvent was evaporated. The residue was purified by flash chromatography (*n*-hexane: ethyl acetate 7 : 3 and 1 % *N*,*N-*dimethylethanamine, ∅ 3 cm, fraction size 30 mL, R_{f}= 0.16).
The titled compound was obtained as a pale yellow oil.

### Example 8

### 7-Methoxy-3-[3-(phenylsulfanyl)propyl]-2,3,4,5-tetrahydro-1H-3-benzazepine-1-ol

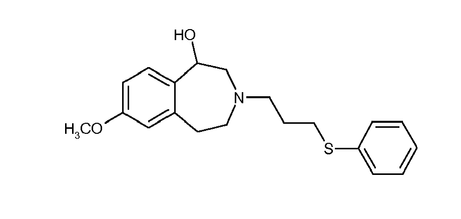

To 7-Methoxy-2,3,4,5-tetrahydro-1*H-*3-benzazepine-1-ol (100.6 mg, 0.52 mmol), synthesized according to step 1.6, (12 mL), TBAI (192.1 mg, 0.52 mmol), K₂CO₃ (574.9 mg, 4.16 mmol) and 1-chloro-3-(phenylsulfanyl)propane (125.1 mg, 0.67 mmol) resulting from step 7.1 were added. The suspension was heated at reflux for 48h. Afterwards the K₂CO₃ was filtered and the solvent was evaporated. The residue was purified by flash chromatography (*n*-hexane: ethyl acetate 7 : 3 and 1 % *N,N-*dimethylethanamin, ∅ 3 cm, fraction size 30 mL, R_{f}= 0.11).
The titled compound was obtained as a colourless oil.

### Example 9

### Preparation of 3-{3-[2-(4-Fluorphenyl)-5,5-dimethyl-1,3-dioxan-2-yl]-propyl)-7-methoxy-2,3,4,5-tetrahydro-1H-3-benzazepine-1-ol

### step 9.1 Preparation of 2-(4-Fluorphenyl)-2-(3-chlorpropyl)-5,5-dimethyl-1,3-dioxane

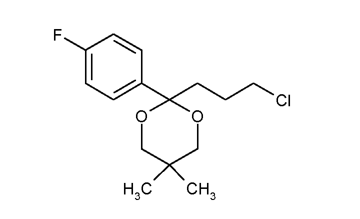

4-Chloro-4'-fluorophenylbutane-1-one (1.0 g, 5.0 mmol), 2,2-dimethylpropane-1,3-diol (610 mg, 5.8 mmol) and p-toluenesulfonic acid (30 mg, 0.19 mmol) were dissolved in toluene (40 mL) and heated at reflux for 8 h at dean-Stark apparatus. The solution was washed with 8% NaHCO₃ solution (3 x 20 mL) and H₂O (2 x 30 mL). The organic phase was dried with Na₂SO₄ and the solvent was evaporated. The residue was purified by flash chromatography (*n*-hexane: ethyl acetate 9.5 : 0.5, ∅ 4 cm, fraction size 20 mL, Rf = 0.51). The titled compound was obtained as colourless solid.

### step9.2 Preparation of 3-{3-[2-(4-Fluorphenyl)-5.5-dimethyl-1.3-dioxan-2-yl]-propyl)-7-methoxy-2,3,4,5-tetrahydro-1H-3-benzazepine-1-ol

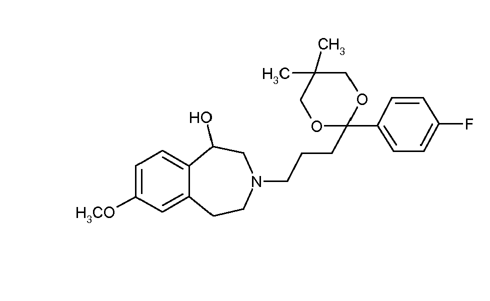

The secondary amine 7-Methoxy-2,3,4,5-tetrahydro-1*H*-3-benzazepine-1-ol (380.0 mg, 1.32 mmol), synthesized according to step 1.6, and TBAI (487.6 mg, 1.32 mmol) were dissolved in CH₃CN (12 mL). Afterwards 2-(4-Fluorphenyl)-2-(3-chlorpropyl)-5,5-dimethyl-1,3-dioxane (200.0 mg, 1.04 mmol) from step 9.1 and K₂CO₃ (575.0 mg, 4.16 mmol) were added. The suspension was heated at reflux for 72 h. In the following step the insoluble residue was filtered and the solvent was evaporated. The residue was purified by flash chromatography (*n*-hexane: ethyl acetate 7 : 3 and 1 % *N,N-*dimethylethanamine, ∅ 2.5 cm, fraction size 10 mL, R_{f}= 0.30). The titled compound was obtained as a colourless oil.

### Example 10

### Preparation of 1-(4-tert-Butylphenyl)-4-(1-hydroxy-7-methoxy-2,3,4,5-tetrahydro-1H-3-benzazepine-3-yl)butane-1-one

### step 10.1 Preparation of 2-(4-tert-Butylphenyl)-2-(3-chlorpropyl)-5,5-dimethyl-1,3-dioxan

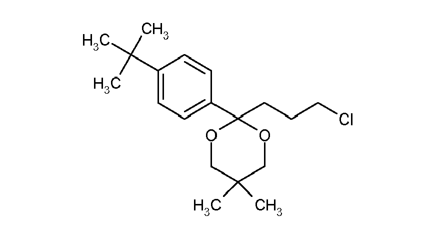

4'-*tert*-Butyl 4-chlorophenylbutane-1-one (1.14 g, 4.78 mmol), 2,2-dimethylpropane-1,3-diol (600 mg, 5.74 mmol) and p-toluenesulfonic acid (30 mg, 0.19 mmol) were dissolved in toluene (40 mL) and heated at reflux for 4 h at dean-Stark apparatus. The solution was washed with 8% NaHCO₃ solution (3 x 20 mL) and H₂O (2 x 30 mL). The organic phase was dried with Na₂SO₄ and the solvent was evaporated. The residue was purified by flash chromatography (*n*-hexane: ethyl acetate 9.5 : 0.5, ∅ 4 cm, fraction size 20 mL, Rf = 0.31). The titled compound was obtained as colourless solid.

### step 10.2 Preparation of 3-{3-[2-(4-tert-Butyl-phenyl)-5.5-dimethyl-1.3-dioxan-2-yl]-propyl}-7-methoxy-2,3,4,5-tetrahydro-1H-3-benzazepine-1-ol

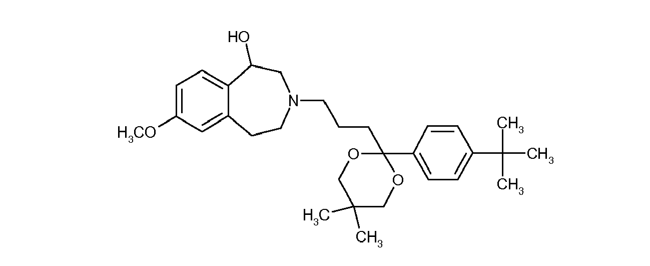

To the compound 7-Methoxy-2,3,4,5-tetrahydro-1*H*-3-benzazepine-1-ol synthesized (161.7 mg, 0.50 mmol) synthesized according to step 1.6 and TBAI (184.7 mg, 0.50 mmol) in CH₃CN (5 mL) was added 2-(4-*tert*-Butylphenyl)-2-(3-chlorpropyl)-5,5-dimethyl-1,3-dioxan resulting from step 10.1 and K₂CO₃ (226.6 mg, 1.64 mmol). The suspension was heated at reflux for 50 h. Finally the insoluble solid was filtered and the solvent was evaporated. The residue was purified by flash chromatography (*n*-hexane: ethyl acetate 7 : 3 and 1 % N,N-dimethylethanamine, ∅ 2 cm, fraction size 10 mL, Rf= 0.21).
The titled compound was obtained as a colourless oil.

### step 10.3 Preparation of 1-(4-tert-Butylphenyl)-4-(1-hydroxy-7-methoxy-2,3,4,5-tetrahydro-1H-3-benzazepine-3-yl)butane-1-one

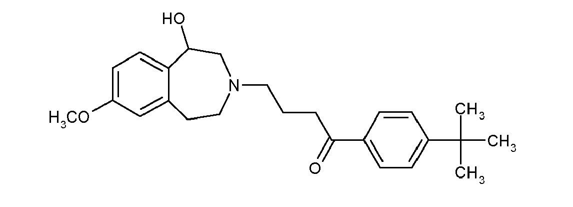

To 3-{3-[2-(4-tert-Butyl-phenyl)-5,5-dimethyl-1,3-dioxan-2-yl]-propyl}-7-methoxy-2,3,4,5-tetrahydro-1*H*-3-benzazepine-1-ol (190.0 mg, 0.39 mmol), synthesized according to step 10.2, diethyl ether (5 mL) was added, followed by the addition of 1M HCl (5 mL). The suspension was stirred for 16 h at room temperature. Afterwards saturated NaHCO₃ solution (3 x 5 mL) was added. The aqueous phase was extracted three times with CH₂CL₂ (5 mL) and the combined organic layers were dried with Na₂SO₄. The solvent was evaporated and the residue was purified by flash chromatography (*n*-hexane:
ethyl acetate 5 : 5 and 1 % *N,N-*dimethylethanamine, ∅ 2 cm, fraction size 10 mL, R_{f} = 0.20). The titled compound was obtained as a colourless oil.

### Example 11

### Preparation of 7-Benzyloxy-2,3,4,5-tetrahydro-1H-3-benzazepine-1-ol

### step 11.1 Preparation of 7-Hydroxy-3-(4-tosyl)-2,3,4,5-tetrahydro-3-benzazepine-1-one

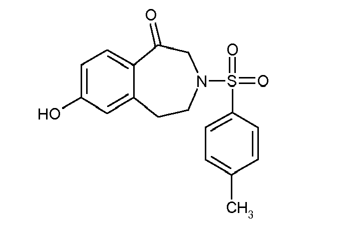

The 7- Methoxy-3-(4-tosyl)-2,3,4,5-tetrahydro-3-benzazepine-1-one (1.06 g, 3.07 mmol), synthesized according to step 1.4, was dissolved in CH₂Cl₂ (50 mL) followed by the addition of ALCL₃ (4.91 g, 36.8 mmol). The suspension was heated at reflux for 23 h. The suspension was mixed with water (60 mL) under ice-cooling und stirred for an additional 1h. The aqueous phase was extracted with a mixture of CH₂CL₂ und CH₃OH ((8 : 2) 3 x 30 mL). The combined organic layers were dried with Na₂SO₄ and the solvent was evaporated. The residue was purified by flash chromatography (*n*-hexane : ethyl acetate 7 : 3, ∅ cm, fraction size 30 mL, R_{f}= 0.14). The titled compound was obtained as a colourless solid.

### step 11.2 Preparation of 7-Benzyloxy-3-(4-tosyl)-2.3.4.5-tetrahydro-3-benzazepine-1-one

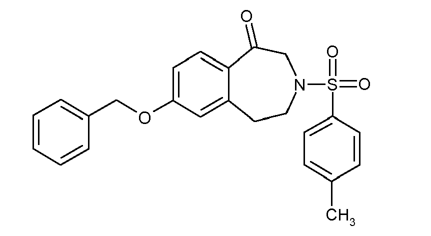

7-Hydroxy-3-(4-tosyl)-2,3,4,5-tetrahydro-3-benzazepine-1-one (0.61 g, 1.83 mmol) synthesized according to step 11.1 was dissolved in acetone (60 mL), followed by the addition of K₂CO₃ (1.01 g, 7.32 mmol) and benzylbromide (0.38 g, 2.20 mmol). The reaction mixture was heated at reflux for 4 h. Afterwards the excess of K₂CO₃ was filtered and the solvent was evaporated. The residue was purified by flash chromatography (*n*-hexane : ethyl acetate 7 : 3, ∅ 5.5 cm, fraction size 65 mL, R_{f}= 0.31). The titled compound was obtained as a colourless solid.

### step 11.3 Preparation of 7-Benzyloxy-3-(4-tosyl)-2,3,4,5-tertahydro-1H-3-benzazepine-1-ol

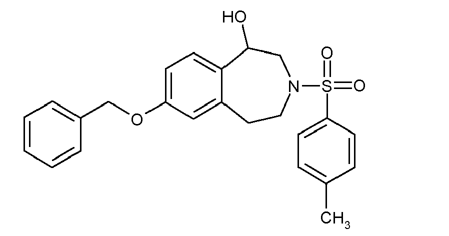

7-Benzyloxy-3-(4-tosyl)-2,3,4,5-tetrahydro-3-benzazepine-1-one (0.40g, 0.94mmol) synthesized according to step 11.2 was suspended in abs. CH₃OH (25 mL) and to the suspension NaBH₄ (0.21 g, 5.64 mmol) was added in several portions. After stirring 6 h at room temperature to the reaction mixture was added H₂O (30 mL) and finally four times extracted with CHCl₃ (30 mL). The organic layer was washed three times with H₂O (40 mL) and after drying with Na₂SO₄ the solvent was evaporated. The residue was purified by flash chromatography (*n*-hexane : ethyl acetate 5 : 5, ∅ 3 cm, fraction size 10 mL, R_{f}= 0.59).
The titled compound was obtained as a colourless solid.

### step 11.4 Preparation of 7-Benzyloxy-2,3,4,5-tetrhydro-1H-3-benzazepine-1-ol

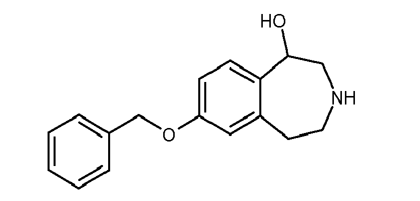

To 7-Benzyloxy-3-(4-tosyl)-2,3,4,5-tertahydro-1*H*-3-benzazepine-1-ol (114.1 mg, 0.27 mmol) synthesized according to step 11.3 in CH₃OH (10 mL) was added Mg (144.2 mg, 5.93 mmol). The mixture was heated at reflux for 5 h, followed by the addition of concentrated H₂SO₄ (0.33 mL) under ice-cooling.
Insoluble material was filtered and the solution was adjusted with NaOH to pH 9. The aqueous phase was extracted five times with CH₂Cl₂ (15 mL) and the organic layer was dried with Na₂SO₄ and removed by evaporation. The residue was purified by flash chromatography (CH₂Cl₂ CH₃OH 9.5 : 0.5 and 2 % NH₃, ∅ 2 cm, fraction size 10 mL, R_{f}= 0.11).
The titled compound was obtained as a colourless solid.

### Example 12

### 7-Benzyloxy-3-(4-phenylbutyl)-2.3.4.5-tetrahydro-1H-3-benzazepine-1-ol

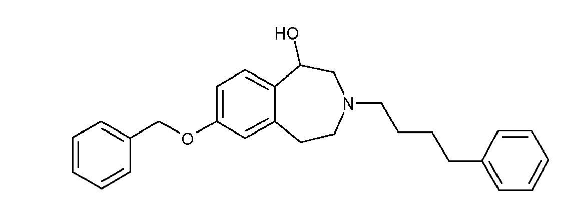

To 7-Benzyloxy-2,3,4,5-tetrahydro-1*H*-3-benzazepine-1-ol (42.9 mg, 0.16 mmol) synthesized according to step 11.4 in CH₃CN (5 mL) was added TBAI (59.1 mg, 0.16 mmol), K₂CO₃ (176.9 mg, 1.28 mmol) and 1-chloro-4-phenylbutane (39.5 µL, 0.24 mmol). The suspension was heated at reflux for 72 h. Afterwards the K₂CO₃ was filtered and the solvent was evaporated. The residue was purified by flash chromatography (*n*-hexane: ethyl acetate 7 : 3 and 1 % N,N-dimethylethanamine, ∅ 2 cm, fraction size 10 mL, R_{f}= 0.30).
The titled compound was obtained as a colourless oil.

### Example 13

### Preparation of 7-(Benzyloxy)-3-[3-(phenylsulfonyl)propyl]-2,3,4,5-tetrahydro-1H-3-benzazepine-1-ol

### step 13.1 Preparation of 1-Chlor-3-(phenylsulfonyl)propane

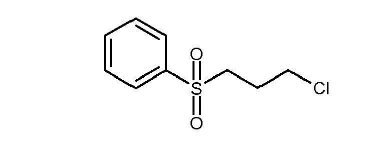

To 1-Chlor-3-(phenylsulfanyl)propane (132.0 mg, 0.71 mmol) synthesized according to step 7.1 was added glacial acetic acid (5 mL) and heated at reflux for 30 min. After removing the heating the dropwise addition of 30% H₂O₂ solution (213.0 µL, 2.41 mmol) followed. Afterwards the solution was heated at reflux for 30 min. To the reaction mixture H₂O (10 mL) was added and extracted three times with toluene (10 mL). The organic layer was washed with 10% Na₂S₂O₃ solution, dried with Na₂SO₄ and the solvent was evaporated. The residue was purified by flash chromatography (*n*-hexane: ethyl acetate 8 : 2, ∅ 2 cm, fraction size 10 mL, Rf = 0.14). The titled compound was obtained as pale yellow oil.

### step 13.2 Preparation of 7-(Benzyloxy)-3-[3-(phenylsulfonyl)propyl]-2,3,4,5-tetrahydro-1H-3-benzazepine-1-ol

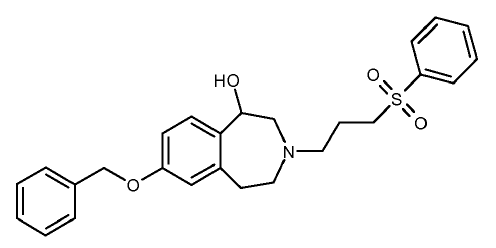

To 7-Benzyloxy-2,3,4,5-tetrahydro-1*H*-3-benzazepine-1-ol (278.3 mg, 1.03 mmol) synthesized
according to step 11.4 in CH₃CN (16 mL) were added TBAI (379.5 mg, 1.13 mmol), K₂CO₃ (569.4 mg, 4.12 mmol) and 1-chloro-3-(phenylsulfonyl)propane (234.4 mg, 1.13 mmol) from step 13.1. The suspension was heated at reflux for 72 h. In the following step the insoluble residue was filtered and the solvent was evaporated.
The residue was purified by flash chromatography (*n*-hexane: ethyl acetate 5 : 5 and 1 % *N,N-*dimethylethanamine, ∅ 4 cm, fraction size 10 mL, R_{f}= 0.15).
The titled compound was obtained as pale yellow oil.

### Example 14

### 7-(Benzyloxy)-3-[3-(phenylsulfanyl)propyl]-2,3,4,5-tetrahydro-1H-3-benzazepine-1-ol

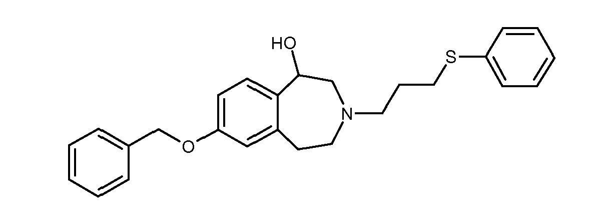

To 7-Benzyloxy-2,3,4,5-tetrahydro-1*H*-3-benzazepine-1-ol (300.0 mg, 1.12 mmol) resulting from step 11.4 in CH₃CN (16 mL) were added TBAI (413.7 mg, 1.12 mmol), K₂CO₃ (619.1 mg, 4.48 mmol) and 1-chloro-3-(phenylsulfanyl)propane from step 7.1 (249.0 mg, 1.34 mmol). The suspension was heated at reflux for 48 h.
In the following step the insoluble residue was filtered and the solvent was evaporated. The residue was purified by flash chromatography (*n*-hexane: ethyl acetate 7 : 3 and 1 % *N*,*N*-dimethylethanamine, ∅ 2 cm, fraction size 10 mL, R_{f}= 0.25). The titled compound was obtained as colourless oil.

### Example 15

### 7-(Benzyloxy)-3-(3-[2-(4-fluorphenyl)-5,5-dimethyl-1,3-dioxan-2-yl]propyl)-2,3,4,5-tetrahydro-1H-3-benzazepine-1-ol

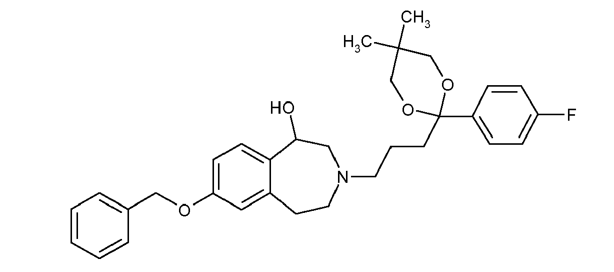

To 7-benzyloxy-2,3,4,5-tetrahydro-1*H*-3-benzazepine-1-ol (292.0 mg, 1.09 mmol) resulting from step 11.4 in CH₃CN (16 mL) was added TBAI (403.0 mg, 1.09 mmol), K₂CO₃ (603.0 mg, 4.36 mmol) and 2-(4-Fluorphenyl)-2-(3-chlorpropyl)-5,5-dimethyl-1,3-dioxane (370.0 mg, 1.30 mmol) from step 9.1. The suspension was heated at reflux for 62 h. In the following step the insoluble residue was filtered and the solvent was evaporated. The residue was purified by flash chromatography (*n*-hexane: ethyl acetate 6 : 4 and 1 % *N*,*N*-dimethylethanamine, ∅ 3 cm, fraction size 10 mL, R_{f}= 0.36).
The titled compound was obtained as colourless oil.

### Example 16

*trans*-7-(Benzyloxy)-3-(4-phenylcyclohexyl)-2,3,4,5-tetrahydro-1*H*-3-benzazepine-1-ol
and
*cis*-7-(Benzyloxy)-3-(4-phenylcyclohexyl)-2,3,4,5-tetrahydro-1*H*-3-benzazepine-1-ol 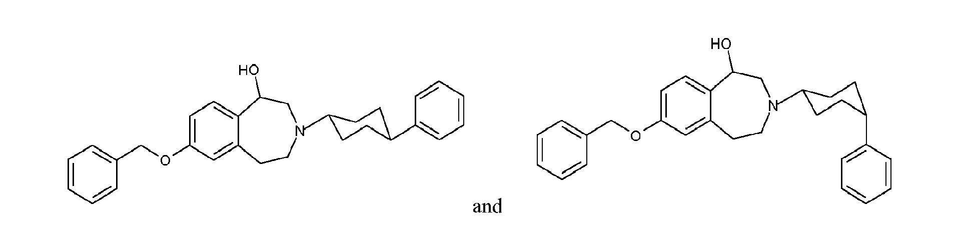 To the secondary amine 7-Benzyloxy-2,3,4,5-tetrahydro-1*H*-3-benzazepine-1-ol (349.7 mg, 1.30 mmol) synthesized according to step 11.4 and 4-phenylcyclohexanone (272.0 mg, 1.56 mmol) dissolved in dichlorethane (25 mL), glacial acetic acid (104.6 µL) was added. Several portions of NaBH(OAc)₃ (495.8 mg, 2.34 mmol) were added to the reaction mixture followed by stirring for 16 h. To the mixture saturated NaHCO₃ solution (20 mL) and H₂O (20 mL) were added. The phases were separated and the aqueous phase was extracted three times with CH₂Cl₂ (40 mL). The combined organic layers were dried with Na₂SO₄ and the solvent was evaporated. The residue was purified by flash chromatography (*n*-hexane: ethyl acetate 9 : 1 and 1 % *N*,*N*-dimethylethanamine, ∅ 3 cm, fraction size 10 mL, R_{f}(*trans*-7-(Benzyloxy)-3-(4-phenylcyclohexyl)-2,3,4,5-tetrahydro-1*H*-3-benzazepine-1-ol) = 0.30, R_{f}(*cis*-7-(Benzyloxy)-3-(4-phenylcyclohexyl)-2,3,4,5-tetrahydro-1*H*-3-benzazepine-1-ol) = 0.36).
*trans*-7-(Benzyloxy)-3-(4-phenylcyclohexyl)-2,3,4,5-tetrahydro-1*H*-3-benzazepine-1-ol was obtained as a colourless solid. *cis*-7-(Benzyloxy)-3-(4-phenylcyclohexyl)-2,3,4,5-tetrahydro-1*H*-3-benzazepine-1-ol was obtained as a colourless oil.

### Example 17

### 7-(Benzyloxy)-3-[(3-phenylbenzyl)-methyl]-2,3,4,5-tetrahydro-1H-3-benzazepme-1-ol

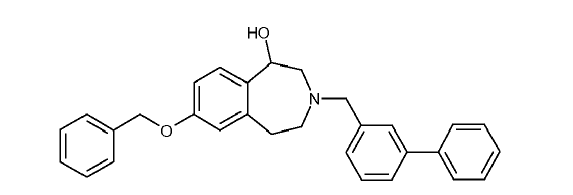

To 7-Benzyloxy-2,3,4,5-tetrahydro-1*H*-3-benzazepine-1-ol (250.1 mg, 0.93 mmol) from step 11.4 in CH₃CN (25 mL), K₂CO₃ (513.7 mg, 3.7 mmol) and 3-phenylbenzyl-bromide (299.0 mg, 1.21 mmol) were added. The suspension was heated at reflux for 72 h. In the following step K₂CO₃ was filtered and the solvent was evaporated. The residue was purified by flash chromatography (*n*-hexane: ethyl acetate 7 : 3 and 1 % *N*,*N*-dimethylethanamine, ∅ 3 cm, fraction size 10 mL, R_{f}= 0.29). The titled compound was obtained as pale yellow oil.

### Example 18 7-Benzyloxy-3-(1,4-dioxaspiro[4.5]decan-8-yl)-2,3,4,5-tetrahydro-1H-3-benzazepine-1-ol

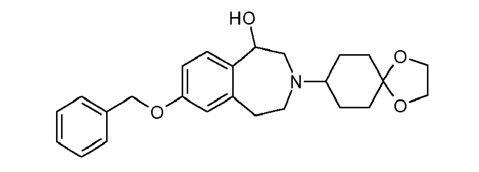

To the secondary amine 7-Benzyloxy-2,3,4,5-tetrahydro-1*H*-3-benzazepine-1-ol (101.5 mg, 0.38 mmol) from step 11.4 and cyclohexane-1,4-dion-monoethylenketal (88.4 mg, 0.57 mmol) in CH₂Cl₂ (3 mL) was added NaBH(OAc)₃ (120.8 mg, 0.57 mmol). The reaction mixture was stirred for 16 h and finally mixed with H₂O (10 mL). The aqueous phase was washed three times with CH₂Cl₂ (10 mL) and the combined organic layers dried with Na₂SO₄. The solvent was evaporated and residue was purified by flash chromatography (*n*-hexane : ethyl acetate 5.5 : 4.5 and 1 % *N*,*N*-dimethylethanamine, ∅ 2 cm, fraction size 10 mL, R_{f}= 0.14). The titled compound was obtained as colourless resin.

### Example 19 3-(4-Phenylbutvl)-2,3,4,5-tetrahydro-1H-3-benzazepine-1,7-diol

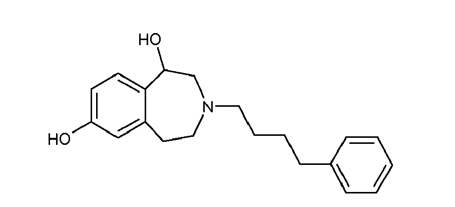

To 7-Benzyloxy-3-(4-phenylbutyl)-2,3,4,5-tetrahydro-1*H*-3-benzazepine-1-ol (39.0 mg, 0.10 mmol) from the example 12 in CH₃OH (5 mL) was added Pd/C (37.0 mg, 10 %). The suspension was stirred under H₂-atmosphere for 30 min at room temperature. The catalysator was filtered and the solvent was removed. The residue was purified by recrystallisation in diisopropyl ether. The titled compound was obtained as pale yellow oil.

### Example 20

### Preparation of N-Benzyl-2-[7-(benzyloxy)-1-hydroxy-2,3,4,5-tetrahydro-1H-3-benzazepine-3-yl]-N-methylacetamide

### step 20.1 Preparation of N-Benzyl-2-chloro-N-methylacetamide

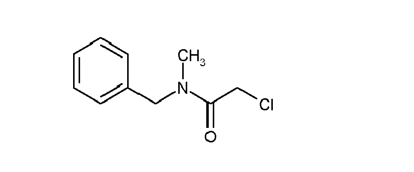

*N*-benzyl-*N*-methylamine (1.0 g, 8.25 mmol) was dissolved in toluene (25 mL) and triethylamine (1.1 mL, 8.25 mmol) was added. Chloracetylchloride (657 µL, 8.25 mmol) in toluene (5 mL) was added dropwise to the solution. The reaction mixture was heated to 35 °C and stirred for 10 h. After addition of H₂O (15 mL) the water phase was extracted three times with diethyl ether (10 mL). The combined organic layers were treated with Na₂SO₄ solution (3 x 20 mL) and dried with K₂CO₃. The solvent was evaporated. And the residue was purified by flash chromatography (*n*-hexane: ethyl acetate 5 : 5, ∅ 4 cm, fraction size 10 mL, Rf = 0.48). The titled compound was obtained as colourless oil.

### step 20.2 Preparation of N-Benzyl-2-[7-(benzyloxy)-1-hydroxy-2,3,4,5-tetrahydro-1H-3-benzazepine-3-yl]-N-methylacetamide

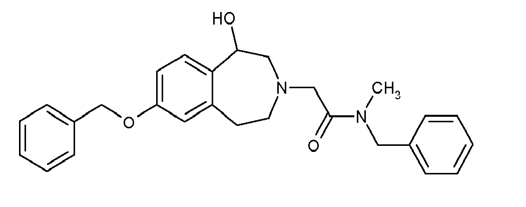

To 7-Benzyloxy-2,3,4,5-tetrahydro-1*H*-3-benzazepine-1-ol (250.0 mg, 0.93 mmol) from step 11.4 , *N-*Benzyl-2-chloro-*N*-methylacetamide (274.6 mg, 1.39 mmol) resulted from step 20.1 TBAI (513.0 mg, 1.39 mmol) and K₂CO₃ (514.1 mg, 3.72 mmol) were added CH₃CN (16 mL). The suspension was heated at reflux for 50 h. The insoluble precipitation was filtered and the solvent was evaporated. The residue was purified by flash chromatography (*n*-hexane: ethyl acetate 2 : 8 and 1 % *N,N-*dimethylethanamine, ∅ 3 cm, fraction size 10 mL, R_{f}= 0.20).
The titled compound was obtained as pale yellow oil.

### Example 21

### N-Benzyl-2-(1,7-dihydroxy-2,3,4,5-tetrahydro-1H-3-benzazepine-3-yl)-N-methylacetamide

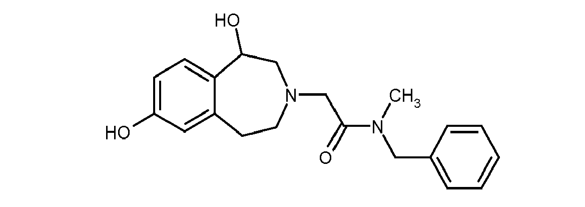

To *N*-Benzyl-2-[7-(benzyloxy)-1-hydroxy-2,3,4,5-tetrahydro-1*H*-3-benzazepine-3-yl]-*N-*methylacetamide (307,1 mg, 0.71 mmol),from example 20, in abs. CH₃OH (30 mL) was added Pd/C (61.4 mg, 10 %). The suspension was stirred under H₂-atmosphere (1 bar) for 1 h at room temperature. The catalysator was filtered and the solvent was removed.
The titled compound was obtained as colourless solid.

### Example 22

*cis*-3-(4-Phenylcyclohexyl)-2,3,4,5-tetrahydro-1*H*-3-benzazepine-1,7-diol 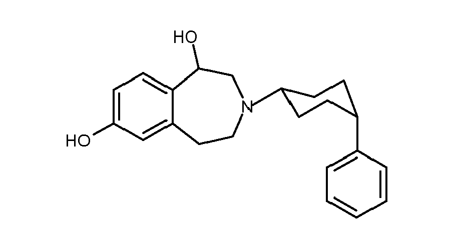 To *cis*-7-(Benzyloxy)-3-(4-phenylcyclohexyl)-2,3,4,5-tetrahydro-1*H*-3-benzazepine-1-ol (340,2 mg, 0.80 mmol), from example 16, in abs. CH₃OH (30 mL) was added Pd/C (200.0 mg, 10 %). The suspension was stirred under H₂-atmosphere (1 bar) for 2 h at room temperature. The catalysator was removed by Celite® 535 filtration then the solvent was removed. The residue was purified by flash chromatography (*n*-hexane : ethyl acetate 6 : 4 and 1 % *N*,*N*-dimethylethanamine, ∅ 2 cm, fraction size 10 mL, R_{f} = 0.19).
The titled compound was obtained as colourless solid.

### Example 23

### 7-Benzyloxy-3-(3-phenoxypropyl)-2,3,4,5-tetrahydro-1H-3-benzazepine-1-ol

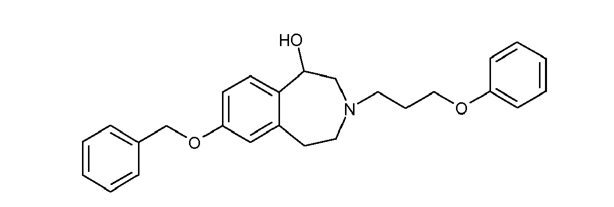

The suspension of the secondary amine 7-benzyloxy-2,3,4,5-tetrahydro-1*H*-3-benzazepine-1-ol (202.8 mg, 0.75 mmol) from step 11.4, 3-phenoxypropylbromide (178.2 µL, 1.13 mmol) und K₂CO₃ (518.2 mg, 3.75 mmol) in CH₃CN (25 mL) were heated at reflux for 50 h. The insoluble precipitation was filtered and the solvent was evaporated. The residue was purified by flash chromatography (*n*-hexane: ethyl acetate 7 : 3 and 1 % *N*,*N*-dimethylethanamine, ∅ 3 cm, fraction size 10 mL, R_{f}= 0.25). The titled compound was obtained as pale yellow oil.

### Example 24

4-(7-Benzyloxy-1-hydroxy-2,3,4,5-tetrahydro-1*H*-3-benzazepine-3-yl)-1-(4-tert-buytlphenyl)butane-1-one 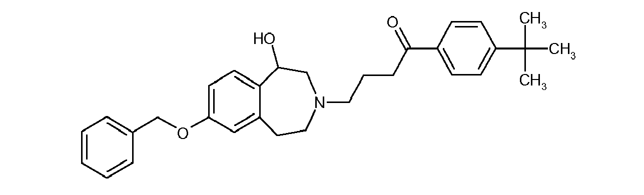 To the compound 7-(Benzyloxy)-3-{3-[2-(4-tert-butylphenyl)-5,5-dimethyl-1,3-dioxan-2-yl]propyl}-2,3,4,5-tetrahydro-1*H*-3-benzazepine-1-ol (115.0 mg, 0.21 mmol) dissolved in diethyl ether (5 mL) was added 1M HCl (5 mL). The suspension was stirred for 16 h at room temperature, followed by the addition of saturated NaHCO₃-solution (5 mL). The water phase was extracted with CH₂Cl₂ (3 x 5 mL) and dried with Na₂SO₄. The solvent was evaporated and the residue was purified by flash chromatography (*n*-hexane : ethyl acetate 5:5+1% *N*,*N*-dimethylethanamine, ∅ 2 cm, fraction size 10 mL, R_{f}= 0.24). The titled compound was obtained as a colourless oil.

### Example 25

### 3-(3-Phenoxypropyl)-2,3,4,5-tetrahydro-1H-3-benzazepine-1,7-diol

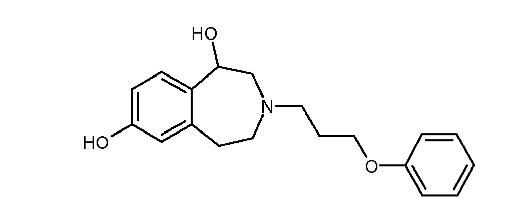

To 7-benzyloxy-3-(3-phenoxypropyl)-2,3,4,5-tetrahydro-1*H*-3-benzazepine-1-ol (250,6 mg, 0.62 mmol),from example 23, in abs. CH₃OH (20 mL) was added Pd/C (202.0 mg, 10 %). The suspension was stirred under H₂-atmosphere (1 bar) for 2h at room temperature. The catalysator was removed by Celite® 535 filtration then the solvent was removed. The residue was purified by flash chromatography (*n*-hexane : ethyl acetate 2 : 8 and 1 % N,N-dimethylethanamine, ∅ 2 cm, fraction size 10 mL, R_{f}= 0.24).
The titled compound was obtained as yellow resin.

### Example 26

*trans*-3-(4-Phenylcyclohexyl)-2,3,4,5-tetrahydro-1*H*-3-benzazepine-1,7-diol 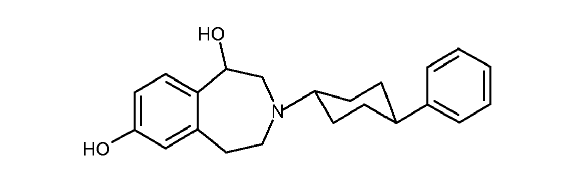 To *trans*-7-(benzyloxy)-3-(4-phenylcyclohexyl)-2,3,4,5-tetrahydro-1*H*-3-benzazepine-1-ol (74.5 mg, 0.17 mmol) ,from example 16, dissolved in abs. THF (5 mL) was added Pd/C (40.0 mg, 10 %). The suspension was stirred under H₂-atmosphere (1 bar) for 2 h at room temperature. The catalysator was removed by Celite^{®} 535 filtration then the solvent was removed. The residue was purified by flash chromatography (*n*-hexane : ethyl acetate 5 : 5 and 1 % *N*,*N*-Dimethylethan-amin, ∅ 2 cm, fraction size 10 mL, R_{f} = 0.18).
The titled compound was obtained as a colourless solid.

### Example 27

### Preparation of 4-(1,7-Dihydroxy-2,3,4,5-tetrahydro-1H-3-benzazepine-3-yl)-1-(4-fluomhenyl)butane-1-one

### step 27.1 Preparation of 4-(7-Benzyloxy-1-hydroxy-2,3,4,5-tetrahydro-1H-3-benzazepine-3-yl)-1-(4-fluorphenyl)butan-1-on

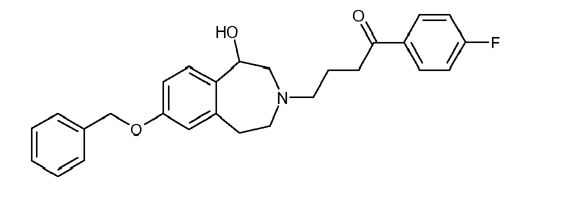

To 7-(Benzyloxy)-3-(3-[2-(4-fluorphenyl)-5,5-dimethyl-1,3-dioxan-2-yl]propyl)-2,3,4,5-tetrahydro-1*H*-3-benzazepine-1-ol (144.2 mg, 0.28 mmol), from example 15, was added diethyl ether (4 mL) and 1M HCl (4 mL). The suspension was stirred for 16 h at room temperature and mixed with saturated NaHCO₃ solution (8 mL). The aqueous phase was extracted with CH₂Cl₂ (3x10 mL) and the combined organic layers were dired with Na₂SO₄. The solvent was evaporated and the residue was purified by flash chromatography (*n*-hexane : ethyl acetate 6 : 4 and 1 % N,N-dimethylethanamine, ∅ 2.5 cm, fraction size 10 mL, Rf = 0.20). The titled compound was obtained as colourless oil.

### step 27.2 Preparation of 4-(1,7-Dihydroxy-2,3,4,5-tetrahydro-1H-3-benzazepine-3-yl)-1-(4-fluorphenyl)butane-1-one

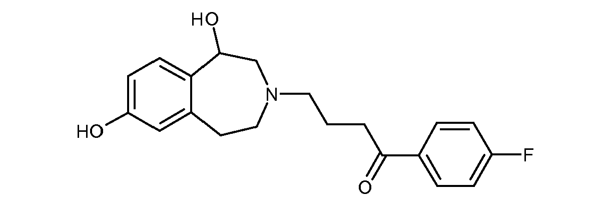

To the 4-(7-Benzyloxy-1-hydroxy-2,3,4,5-tetrahydro-1*H*-3-benzazepine-3-yl)-1-(4-fluorphenyl)butane-1-one (60.9 mg, 0.14 mmol), from the step 27.1, in abs. CH₃OH (6 mL) was added Pd/C (12.2 mg, 10 %). The suspension was stirred under H₂-atmosphere (1 bar) for 1 h at room temperature. The catalysator was removed by Celite^{®} 535 filtration then the solvent was removed. The residue was purified by flash chromatography (*n*-hexane : ethyl acetate 2 : 8 and 1 % *N*,*N*-Dimethylethanamin, ∅ 2 cm, fraction size 10 mL, R_{f}= 0.08).
The titled compound was obtained as pale yellow oil.

### Example 28

### Preparation of 3-(Benzoylpiperidine-4-yl)2,3,4,5-tetrahydro-1H-3-benzazepine-1,7-diol

### step 28.1 Preparation of 3-(1-Benzoylpiperidine-4-yl)-7-benzyloxy-2,3,4,5-tetrahydro-1H-3-benzazepine-1-ol

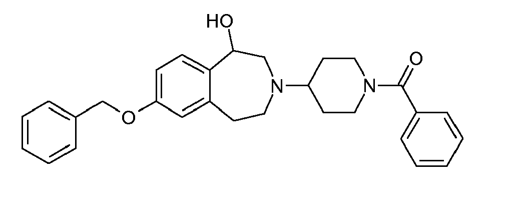

To the secondary amine 7-Benzyloxy-2,3,4,5-tetrahydro-1*H*-3-benzazepine-1-ol (125.4 mg, 0.47 mmol), from step 11.4, and 1-benzoylpiperid-4-one (113.7 mg, 0.56 mmol) in CH₂Cl₂ (4 mL) are added NaBH(OAc)₃ (118.7 mg, 0.56 mmol). The reaction mixture was stirred for 16 h at room temperature and subsequently mixed with H₂O (10 mL). The aquous phase was extracted three times with CH₂Cl₂ (10 mL) and the combined organic layers dried with Na₂SO₄. Then the solvent was removed and the residue was purified by flash chromatography (*n*-hexane : ethyl acetate 3 : 7 and 1 % N,N-dimethylethanamine, ∅ 2 cm, fraction size 10 mL, Rf = 0.23). The titled compound was obtained as pale yellow resin.

### step 28.2 Preparation of 3-(Benzoylpiperidine-4-yl)2,3,4,5-tetrahydro-1H-3-benzazepine-1,7-diol

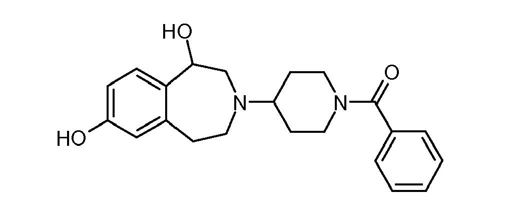

To 3-(1-Benzoylpiperidine-4-yl)-7-benzyloxy-2,3,4,5-tetrahydro-1*H*-3-benzazepine-1-ol (90.1 mg,
0.20 mmol), from step 28.1, in abs. CH₃OH (4 mL) was added Pd/C (60.0 mg, 10 %). The suspension was stirred under H₂-atmosphere (1 bar) for 2 h at room temperature. The catalysator was removed by Celite^{®} 535 filtration then the solvent was removed. The residue was purified by flash chromatography (ethyl acetate : CH₃OH 9 : 1 and 1 % *N*,*N*-dimethylethanamine, ∅ 2 cm, fraction size 10 mL, R_{f}= 0.26).
The titled compound was obtained as colourless solid.

### Example 29

### 3-(1,4-Dioxaspiro[4.5]decane-8-yl)-2,3,4,5-tetrahydro-1H-3-benzazepine-1,7-diol

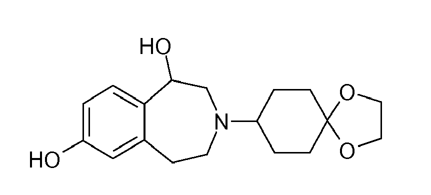

To 7-Benzyloxy-3-(1,4-dioxaspiro[4.5]decan-8-yl)-2,3,4,5-tetrahydro-1*H*-3-benzazepine-1-ol (89.3 3 mg, 0.22 mmol), from example 18, in abs. CH₃OH (6 mL) was added Pd/C (53.2 mg, 10 %). The suspension was stirred under H₂-atmosphere (1 bar) for 1h at room temperature. The catalysator was removed by Celite^{®} 535 filtration then the solvent was removed. The residue was purified by flash chromatography (*n*-hexane : ethyl acetate 4 : 6 and 1 % *N*,*N*-Dimethylethanamin, ∅ 2 cm, fraction size 10 mL, R_{f}= 0.10).
The titled compound was obtained as a colourless solid.

### Example 30

### Preparation of (1S, 2S)-7-Methoxy-2-methyl-3-(4-phenylbutyl)-2,3,4,5-tetrahydro-1H-3-benzazepine-1-ol and (1R, 2R)-7-Methoxy-2-methyl-3-(4-phenylbutyl)-2,3,4,5-tetrahydro-1H-3-benzazepine-1-ol

### step 30.1 Preparation of (S)-Alaninmethylester-Hydrochlorid

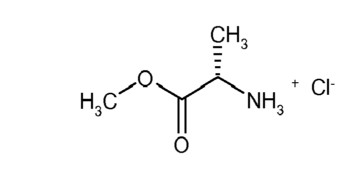

CH₃OH (20 mL) was cooled to -5 °C and SOCl₂ (4.35 mL, 0.06 mol) was added slowly. To the solution (S)-alanine (1.78 g, 0.02 mol) was added in several portions. After the (S)-alanine was dissolved the solution was allowed to come to room temperature and stirred for further 2h. The excess of the SOCl₂ and the CH₃OH was removed by evaporation. The residue was dissolved in CH₃OH (30 mL) and the solvent was evaporated. This procedure was repeated twice. The titled compound was obtained as a colourless solid.

### step 30.2 Preparation of (S)-Methyl-2-(4-tosyl)aminopropionat

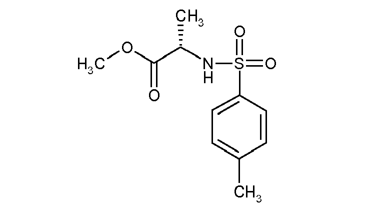

To (S)-alaninmethylester hydrochloride of step 30.1 (3.92 g, 28.1 mmol) in CH₂Cl₂ (95 mL) was added p-toluenesulfonic acid in several portions followed by the addition of triethyamine (7.45 mL, 70.3 mmol). The solution was stirred for 20 h at room temperature. The organic phase was mixed with 0.5M HCl (3 x 30 mL) and F₂O (3 x 30 mL). The organic layers were dried with Na₂SO₄ and concentrated in vacuum. The residue was purified by flash chromatography (*n*-hexane : ethyl acetate 7 : 3 and 1 % N,N-dimethylethanamine, 8 cm, fraction size 65 mL, Rf= 0.62). The titled compound was obtained as colourless solid.

### step 30.3 Preparation of 2-(3-Methoxyphenyl)ethanol

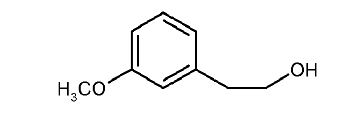

3-Bromanisole (300 µL, 2.39 mmol) was dissolved under nitrogen atmosphere in abs. THF (35 mL)
and cooled to -78 °C. Afterwards *n*-butyllithium in *n*-hexane (1.48 M, 1.62 mL 2.39 mmol) was added dropwise during 30 min and subsequently stirred for 2 h at -78 °C. In the following step ethylensulfat (355.3 mg, 2.87 mmol) was dissolved in abs. THF (5 mL) und transferred dropwise to the solution for 20 min. The reaction mixture was allowed to come to room temperature and strirred for 16 h. After the addition of H₂O (10 mL) and conc. H₂SO₄ (1.8 mL) the suspension was heated at reflux for 47 h. Finally the solution was neutralized by 5M NaOH (20 mL) and extracted three times with CH₂Cl₂ (20 mL). The combined organic layers were dried with Na₂SO₄ and concentrated in vacuum. The residue was purified by flash chromatography (*n*-hexane : ethyl acetate 8 : 2, ∅ 4 cm, fraction size 30 mL, R_{f} =0.16).
The titled compound was obtained as colourless oil.

### step 30.4 Preparation of (S)-Methyl-2-{N-[2-(3-methoxyphenyl)ethyl]-N-(4-tosyl)amino}propionate

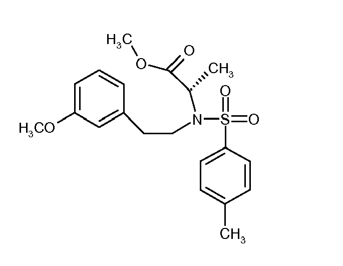

2-(3-Methoxyphenyl)ethanol (7.74 g, 0.05 mol), from step 30.3, dissolved in abs. THF (600 mL) was cooled to 0 °C under nitrogen atmosphere followed by the addition of (S)-Methyl-2-(4-tosyl)aminopropionat (13.1 g, 0.05 mol), from step 29.1, and Ph₃P (40.3 g, 0.15 mol). In the next step DIAD (29.7 mL, 0.15 mol) was added dropwise. After 1h at 0 °C the reaction mixture was allowed to come to room temperature followed by stirring for 16 h. Then the solution was diluted with *n*-hexane (700 mL) and the precipitated Ph₃P=O was removed by filtration. The solvent was evaporated and the residue was was purified by flash chromatography (*n*-hexane : ethyl acetate 8 : 2, ∅ 8 cm, fraction size 65 mL, R_{f}= 0.35).
The titled compound was obtained as colourless oil.

### step 30.5 Preparation of (S)-2-{N-[2-(3-Methoxy-phenyl)ethyl]-N-(4-tosyl)amino}propion acid

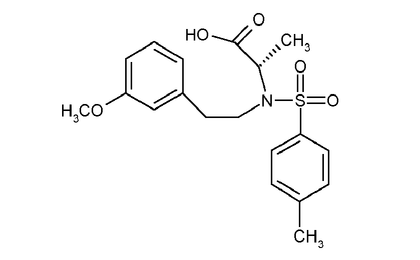

(S)-Methyl-2-{N-[2-(3-methoxyphenyl)ethyl]-N-(4-tosyl)ammo}propionate (1.54 g, 3.95 mmol), from step 30.4, was dissolved in THF : H₂O-Mixture (7 : 3, 60 mL) followed by the addition of LiOH·H₂O (1.26 g, 19.8 mmol). The mixture war stirred for 24 h at room temperature. Afterwards the mixture was acidified with 6M HCl and the aqueous phase was extracted four times with diethyl ether (30 mL). The combined organic layers were dried with Na₂SO₄ and the solvent was evaporated. The residue was purified by flash chromatography (*n*-hexane : ethyl acetate 2 : 8, ∅ 2 cm, fraction size 30 mL, R_{f}= 0.35). The titled compound was obtained as colourless oil.

### step 30.6 Preparation of (S)-7-Methoxy-2-methyl-3-(4-tosyl)-2,3,4,5-tetrahydro-3-benzazepine-1-one

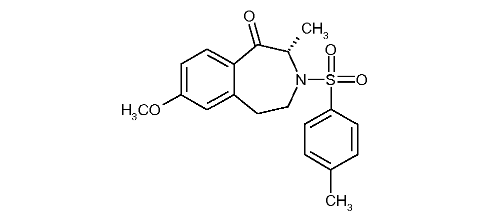

in mixture with
(S)-9-Methoxy-2-methyl-3-(4-tosyl)-2,3,4,5-tetrahydro-3-benzazepine-1-one 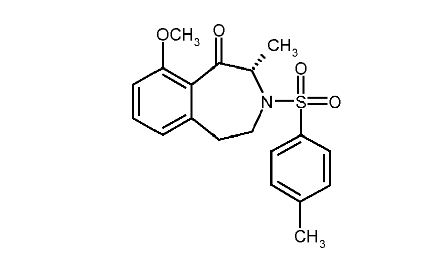 (S)-2-{N-[2-(3-Methoxy-phenyl)ethyl]-N-(4-tosyl)amino}propion acid (5.10 g, 14.0 mmol), from step 30.5, was dissolved in dichloromethane (300 mL) under nitrogen atmosphere and cooled to -30 °C. Then trifluoracetic acid anhydride (9.56 mL, 68.0 mmol) was added and stirred for 1 h at -30 °C. Afterwards SnCl₄ (6.55 mL, 56.0 mmol) was added slowly. After 22 h stirring at -30 °C the solution was neutralized with 5M NaOH at 0 °C and the aqueous phase was extracted three times with CH₂Cl₂ (50 mL). The combined organic layers were dried with Na₂SO₄ and the solvent was evaporated. The residue was purified by flash chromatography (*n*-hexane : ethyl acetate 7 : 3, ∅ 8 cm, fraction size 65 mL, R_{f}= 0.36).
The regioisomers (S)-7-Methoxy-2-methyl-3-(4-tosyl)-2,3,4,5-tetrahydro-3-benzazepine-1-one and (S)-9-Methoxy-2-methyl-3-(4-tosyl)-2,3,4,5-tetrahydro-3-benzazepine-1-one were obtained as colourless solid and were used in the following step as mixture.

### step 30. 7 Preparation of (1S,2S)-7-Methoxy-2-methyl-3-(4-tolylsulfony)-2,3,4,5-tetrahydro-1H-3-benzazepine-1-ol

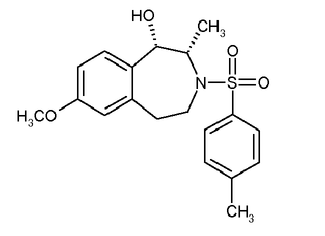

and
(1R,2S)-7-Methoxy-2-methyl-3-(4-tolylsulfony)-2,3,4,5-tetrahydro-1H-3-benzazepine-1-ol 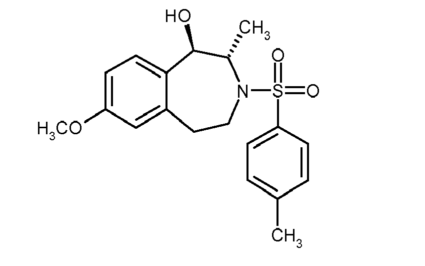 A mixture of (S)-7-methoxy-2-methyl-3-(4-tosyl)-2,3,4,5-tetrahydro-3-benzazepine-1-one and compound (S)-9-Methoxy-2-methyl-3-(4-tosyl)-2,3,4,5-tetrahydro-3-benzazepine-1-one (6.34 g, 17.63 mmol) of step 30.6 were dissolved in THF (400 mL) under nitrogen atmosphere and cooled to -90 °C. Afterwards a 2M solution of LiBH₄ (22.04 mL, 44.02 mmol) in THF was added slowly. After 24h stirring at -90 °C H₂O (100 mL) was added and the reaction mixture was allowed to come to room temperature. The aqueous phase was extracted three times with CHCl₃ (40 mL) and the combined organic layers were dried with Na₂SO₄. The solvent was evaporated. The residue was purified by flash chromatography (*n*-hexane : ethyl acetate7 : 3, ∅ 8 cm, fraction size 65 mL, R_{f}((1S,2S)-7-Methoxy-2-methyl-3-(4-tolylsulfony)-2,3,4,5-tetrahydro-1H-3-benzazepine-1-ol) = 0.45, R_{f}((1R,2S)-7-Methoxy-2-methyl-3-(4-tolylsulfony)-2,3,4,5-tetrahydro-1H-3-benzazepine-1-ol) = 0.24).
(1S,2S)-7-Methoxy-2-methyl-3-(4-tolylsulfony)-2,3,4,5-tetrahydro-1H-3-benzazepine-1-ol) was obtained as a colourless oil. (1R,2S)-7-Methoxy-2-methyl-3-(4-tolylsulfony)-2,3,4,5-tetrahydro-1H-3-benzazepine-1-ol was obtained as a colourless oil.

### step 30.8 Preparation of (1S,2S)-7-Methoxy-2-methyl-2,3,4,5-tetrahydro-1H-3-benzazepin-1-ol

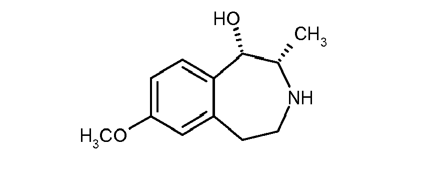

To compound (1S,2S)-7-Methoxy-2-methyl-3-(4-tolylsulfony)-2,3,4,5-tetrahydro-1H-3-benzazepine-1-ol of step 30.7 (1.13 g, 3.26 mmol) dissolved in CH₃OH (170 mL) was added Mg (1.72 g, 71.7 mmol). The reaction mixture was heated at reflux for 18 h and afterwards acidified with 2M HCl and the unspent Mg was filtered. The solution was adjusted with 2M NaOH to an alkaline pH-value (pH 9-10), the water phase was subsequently extracted with CH₂Cl₂ (5 x 60 mL) and dried with Na₂SO₄. The solvent was evaporated and the residue was purified by flash chromatography (ethyl acetate : CH₃OH 9 : 1 + 2 % *N*,*N*-dimethylethanamine, ∅ 4 cm, fraction size 30 mL, R_{f} = 0.08). The titled compound was obtained as a colourless solid.

### step 30.9 Preparation of (1S, 2S)-7-Methoxy-2-methyl-3-(4-phenylbutyl)-2,3,4,5-tetrahydro-1H-3-benzazepine-1-ol and (1R, 2R)-7-Methoxy-2-methyl-3-(4-phenylbutyl)-2,3,4,5-tetrahydro-1H-3-benzazepine-1-ol

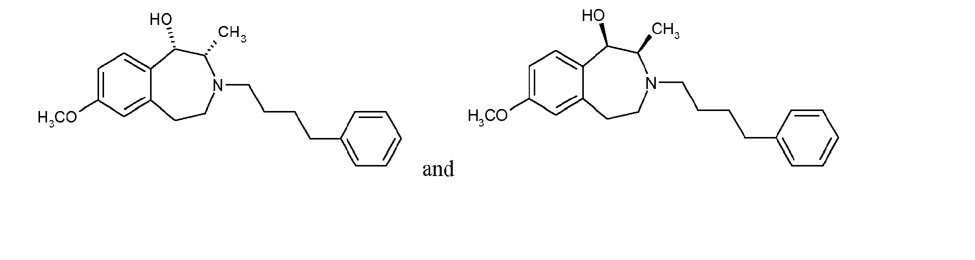

The compound Preparation of (1S,2S)-7-Methoxy-2-methyl-2,3,4,5-tetrahydro-1H-3-benzazepin-1-ol of step 30.8 (70.7 mg, 0.34 mmol), 1-chlor-4-phenylbutane (108.5 µL, 0.68 mmol), TBAI (251.2 mg, 0.68 mmol) and K₂CO₃ (375.9 mg, 2.72 mmol) were dissolved in CH₃CN (9 mL). After 72 h heating at reflux the precipitation was filtered and the solvent was evaporated. The residue was purified by flash chromatography (*n*-hexane : ethyl acetate 7 : 3 and 1 % N,N-Dimethylethanamin, ∅ 2 cm, fraction size 10 mL, R_{f}= 0.29). The titled enatiomeres as a mixture were obtained as colourless solid.
The separation of the enantiomeres was carried out on a chiral HPLC (column: Chiralpak^{®} AD (Chiral Technologies Europe), solvent: n-Hexan : Isopropanol (95.5 : 4.5), t_{R} ((1S, 2S)-7-Methoxy-2-methyl-3-(4-phenylbutyl)-2,3,4,5-tetrahydro-1H-3-benzazepine-1-ol) = 16.6 min, t_{R} (, 2R)-7-Methoxy-2-methyl-3-(4-phenylbutyl)-2,3,4,5-tetrahydro-1H-3-benzazepine-1-ol) = 19.9 min).
(1S, 2S)-7-Methoxy-2-methyl-3-(4-phenylbutyl)-2,3,4,5-tetrahydro-1H-3-benzazepine-1-ol was obtained as a colourless resin. (1R, 2R)-7-Methoxy-2-methyl-3-(4-phenylbutyl)-2,3,4,5-tetrahydro-1H-3-benzazepine-1-ol was obtained as a colourless solid.

### Example 31

### Preparation of (1R,2S)-2-Methyl-3-(4-phenylbutyl)-2,3,4,5-tetrahydro-1H-3-benzazepine-1,7-diol and (1S,2R)-2-Methyl-3-(4-phenylbutyl)-2,3,4,5-tetrahydro-1H-3-benzazepine-1,7-diol

### Step 31.1 Preparation of (S)-Alaninmethylester-hydrochloride

The procedure was carried out as described in step 30.1.

### Step 31.2 Preparation of (S)-Methyl-2-(4-tosyl)aminopropionate

The procedure was carried out as described in step 30.2.

### Step 31.3 Preparation of 2-(3-Benzyloxyphenyl)-ethanol

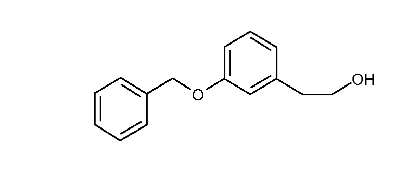

3-(2-Hydroxyethyl)phenole (216.4 mg, 1.6 mmol) and benzylbromide (203.8 µL, 1.7 mmol) were dissolved in a 10 mL microwave pressure vessel. To the solution K₂CO₃ (862.4 mg, 6.2 mmol) was added. Parameters of reaction in the mircowave (Discover, CEM GmbH) were: standard mode, stir on, 220 watt power, maximum temperature 100 °C, maximum pressure 4 bar, 5 min ramp time, 40 min hold time and 5 min cool off time. Afterwards the K₂CO₃ was filtered and the solvent was evaporated. The residue was purified by flash chromatography (*n*-hexane : ethyl acetate 8 : 2, ∅ 2 cm, fraction size 10 mL, R_{f}= 0,15). The titled compound was obtained as a colourless solid.

### Step 31.4 Preparation of (S)-Methyl-2-{N-[2-(3-benzyloxyphenyl)ethyl]-N-(4-tosyl)amino}-propionate

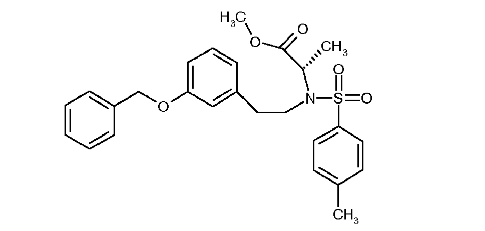

2-(3-Benzyloxyphenyl)-ethanol of step 31.3 (7.74 g, 0.05 mol) was dissolved in abs. THF (600 mL) and cooled under nitrogen atmosphere to 0 °C. To the solution (S)-methyl-2-(4-tosyl)aminopropionate of step 31.2 and Ph₃P (40.3 g, 0.15 mol) were added and afterwards DIAD (29.7 mL, 0.15 mol) was added drop wise. After 1h at 0 °C the reaction mixture was allowed to come to room temperature and stirred for 16h. To the solution *n*-hexane (700 mL) was added and the precipitated Ph₃P=O was removed by filtration. The solvent was evaporated and residue was purified by flash chromatography (*n*-hexane : ethyl acetate 8 : 2, ∅ 8 cm, fraction size 65 mL, R_{f}= 0.35). The titled compound was obtained as colourless oil.

Step 31.5 Preparation of (S)-2-{*N*-[2-(3-Benzyloxyphenyl)ethyl]-*N*-(4-tosyl)amino}propionic acid 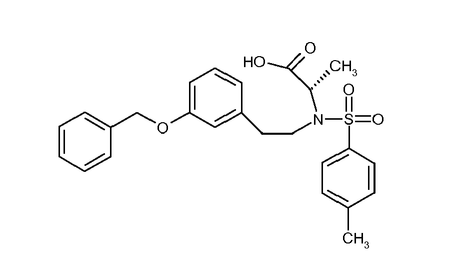 To (S)-methyl-2-{*N*-[2-(3-benzyloxyphenyl)ethyl]-*N*-(4-tosyl)amino}propionate of step 31.4 (785.4 mg, 1.7 mmol) dissolved in THF (21 mL) was added H₂O (9 mL) and LiOH·H₂O (660 mg, 15,1 mmol). The mixture was stirred at room temperature for 23 h, afterwards the pH was adjusted to pH=6 and extracted with CH₂Cl₂ (3x15 mL). The combined organic layers were dried with K₂CO₃ and the solvent was evaporated. The residue was purified by flash chromatography (*n*-hexane : ethyl acetate 2:8, ∅ 3 cm, fraction size 30 mL, R_{f}= 0.43).
The titled compound was obtained as colourless oil.

### Step 31.6 Preparation of (S)-7-Benzyloxy-2-methyl-3-(4-tosyl)-2,3,4,5-tetrahydro-3-benzazepine-1-one in mixture with

### (S)-9-Benzyloxy-2-methyl-3-(4-tosyl)-2,3,4,5-tetrahydro-3-benzazepine-1-one

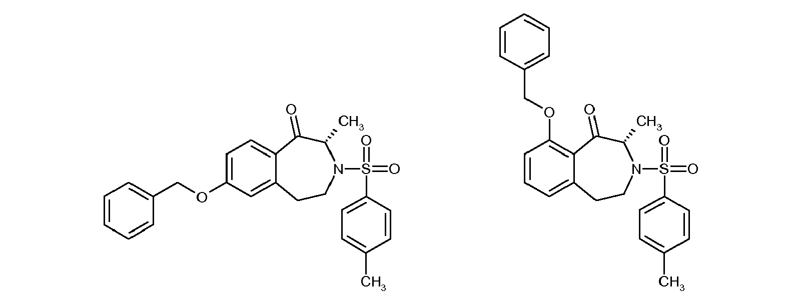

To (S)-2-{*N*-[2-(3-benzyloxyphenyl)ethyl]-*N*-(4-tosyl)amino}propionic acid of step 31.5 (4.77 g, 10.5 mmol) wad added CH₂Cl₂ (295 mL) under nitrogen atmosphere at -15 °C. After addition of trifluoracetic acid anhydride (5.56 mL, 39.4 mmol) the mixture was stirred for 30 min. Then SnCl₄ (4.61 mL, 39.4 mmol) was added drop wise to the mixture and subsequently stirred for 24h at -15 °C. H₂O (150 mL) was added drop wise and the mixture was allowed to come to room temperature. The water phase was neutralized with 2M NaOH, saturated with brine and extracted with CH₂Cl₂ (3 x 75 mL). The combined organic layers were dried with Na₂SO₄ and the solvent was evaporated. The residue was dissolved in acetone (180 mL) followed by the addition of K₂CO₃ (5.44 g, 39.4 mmol) and benzylbromide (1.56 mL, 13.1 mmol). The suspension was heated at reflux for 6h. The insoluble residue was filtered and the solvent was evaporated. The residue was purified by flash chromatography (*n*-hexane : ethyl acetate 7 : 3, ∅ 8 cm, fraction size 65 mL, R_{f} ((S)-7-Benzyloxy-2-methyl-3-(4-tosyl)-2,3,4,5-tetrahydro-3-benzazepine-1-one (30a) and (S)-9-Benzyloxy-2-methyl-3-(4-tosyl)-2,3,4,5-tetrahydro-3-benzazepine-1-one) = 0.34).
The mixture of the titled compounds was obtained as a colourless solid.
The regioisomers (S)-7-Benzyloxy-2-methyl-3-(4-tosyl)-2,3,4,5-tetrahydro-3-benzazepine-1-one (30a) and (S)-9-Benzyloxy-2-methyl-3-(4-tosyl)-2,3,4,5-tetrahydro-3-benzazepine-1-one could not be separated by flash chromatography and were further used as mixture.

### Step 31.7 Preparation of (1R,2S)-7-Benzyloxy-2-methyl-3-(4-tosyl)-2,3,4,5-tetrahydro-1H-3-benzazepine-1-ol and (1S,2S)-7-Benzyloxy-2-methyl-3-(4-tosyl)-2,3,4,5-tetrahydro-1H-3-benzazepine-1-ol

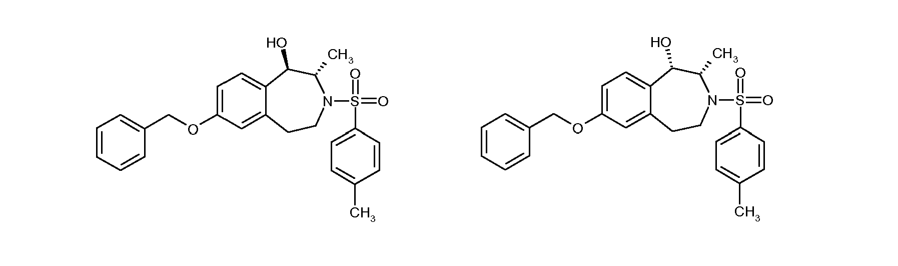

To a mixture of the compounds (S)-7-Benzyloxy-2-methyl-3-(4-tosyl)-2,3,4,5-tetrahydro-3-benzazepine-1-one (30a) and (S)-9-Benzyloxy-2-methyl-3-(4-tosyl)-2,3,4,5-tetrahydro-3-benzazepine-1-one of step 31.6 (3.11 g, 7.16 mmol) were added abs. CH₃OH (75 mL) in nitrogen atmosphere. The suspension was added NaBH₄ (0.55 g, 14.3 mmol) in several portions and stirred for 20 h at room temperature. After the addition of water H₂O (30 mL) the water phase was extracted with CH₂Cl₂ (3 x 20 mL) and the combined organic layers were dried with Na₂SO₄.
The solvent was evaporated and the residue was purified by flash chromatography (*n*-hexane : ethyl acetate 7 : 3, ∅ 8 cm, fraction size 65 mL, R_{f} ((1R,2S)-7-Benzyloxy-2-methyl-3-(4-tosyl)-2,3,4,5-tetrahydro-1*H*-3-benzazepine-1-ol) = 0.44, R_{f} ((1S,2S)-7-Benzyloxy-2-methyl-3-(4-tosyl)-2,3,4,5-tetrahydro-1*H*-3-benzazepine-1-ol) = 0.29).
The compound (1S,2S)-7-Benzyloxy-2-methyl-3-(4-tosyl)-2,3,4,5-tetrahydro-1*H*-3-benzazepine-1-ol was obtained as a colourless solid. The compound (1R,2S)-7-Benzyloxy-2-methyl-3-(4-tosyl)-2,3,4,5-tetrahydro-1*H*-3-benzazepine-1-ol was obtained as a colourless solid.

### Step 31.8 Preparation of (1R,2S)-7-Benzyloxy-2-methyl-2,3,4,5-tetrahydro-1H-3-benzazepine-1-ol

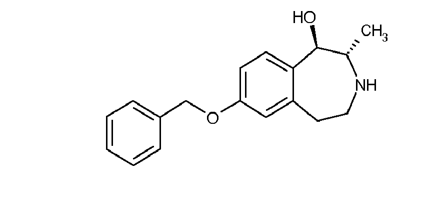

To compound (1R,2S)-7-Benzyloxy-2-methyl-3-(4-tosyl)-2,3,4,5-tetrahydro-1*H*-3-benzazepine-1-ol of step 31.7 (1.07 g, 2.45 mmol) in CH₃OH (125 mL) was added Mg (1.8 g, 75.0 mmol). The reaction mixture was heated at reflux for 36h. Afterwards the pH was adjusted with 2M HCl to pH=6 and unspent Mg was filtered, followed by the addition of NaOH to adjust to an alkaline pH-value (pH 10) of the filtrate. The water phase was subsequently extracted with CH₂Cl₂ (5 x 50 mL). The combined organic layers were dried with Na₂SO₄ and the solvent was evaporated. The residue was purified by flash chromatography (ethyl acetate : CH₃OH 9 : 1 + 2 % *N*,*N*-dimethylethanamine, ∅ 6 cm, fraction size 65 mL, R_{f}= 0.13) and finally purified in CH₃CN by fractionated crystallization. The titled compound was obtained as a colourless solid.

### Step 31.9 Preparation of (1R,2S)-7-Benzyloxy-2-methyl-3-(4-phenylbutyl)-2,3,4,5-tetrahydro-1H-3-benzazepine-1-ol and (1S,2R)-7-Benzyloxy-2-methyl-3-(4-phenylbutyl)-2,3,4,5-tetrahydro-1H-3-benzazepine-1-ol

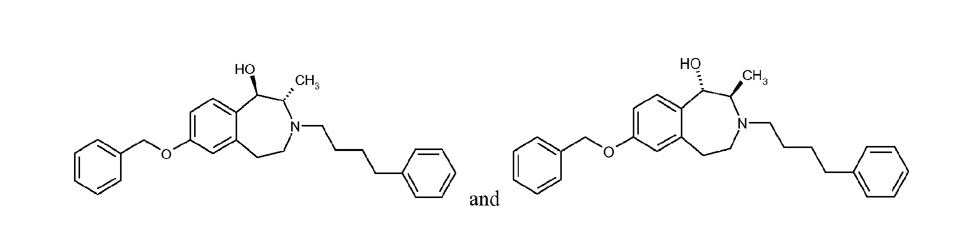

To (1R,2S)-7-Benzyloxy-2-methyl-2,3,4,5-tetrahydro-1*H*-3-benzazepine-1-ol of step 31.8 (101.2 mg, 0.36 mmol), 1-chlor-4-phenylbutane (88.8 µL, 0.54 mmol), Tetrabutylammonium iodide (TBAI, 199.5 mg, 0.54 mmol) and K₂CO₃ (248.8 mg, 1.80 mmol) was added CH₃CN (14 mL) and subsequently heated at reflux for 72h. The precipitation was filtered and the solvent was evaporated. The residue was purified by flash chromatography (*n*-hexane : ethyl acetate 9:1+1% *N*-*N*-dimethylethanamine, ∅ 3 cm, fraction size 10 mL, R_{f}= 0.30). The mixture of the titled compounds was obtained as a colourless solid.
The separation of the enatiomeres (1R,2S)-7-Benzyloxy-2-methyl-3-(4-phenylbutyl)-2,3,4,5-tetrahydro-1*H*-3-benzazepine-1-ol and (1 S,2R)-7-Benzyloxy-2-methyl-3-(4-phenylbutyl)-2,3,4,5-tetrahydro-1*H*-3-benzazepine-1-ol was carried out on a chiral HPLC according to (column: Chiralpak^{®} AD, *n*-hexane : isopropanol 75 : 25, t_{R} = 15.8 min, t_{R} = 10.2 min)
The compound (1R,2S)-7-Benzyloxy-2-methyl-3-(4-phenylbutyl)-2,3,4,5-tetrahydro-1*H*-3-benzazepine-1-ol was obtained as a colourless oil.
The compound (1S,2R)-7-Benzyloxy-2-methyl-3-(4-phenylbutyl)-2,3,4,5-tetrahydro-1*H*-3-benzazepine-1-ol was obtained as a colourless oil.

### Step 31.10 Preparation of (1R,2S)-2-Methyl-3-(4-phenylbutyl)-2,3,4,5-tetrahydro-1H-3-benzazepine-1,7-diol in mixture with

### (1S,2R)-2-Methyl-3-(4-phenylbutyl)-2,3,4,5-tetrahtdro-1H-3-benzazepine-1,7-diol

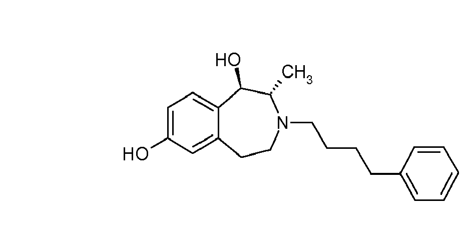

in mixture with 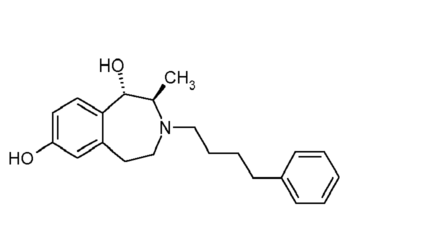 To the mixture of the compounds (1R,2S)-7-Benzyloxy-2-methyl-3-(4-phenylbutyl)-2,3,4,5-tetrahydro-1*H*-3-benzazepine-1-ol and (1 S,2R)-7-Benzyloxy-2-methyl-3-(4-phenylbutyl)-2,3,4,5-tetrahydro-1*H*-3-benzazepine-1-ol of step 31.9 (163.2 mg, 0.39 mmol) dissolved in abs. CH₃OH (16 mL) was added Pd/C (110.2 mg, 10 %). The suspension was stirred 1h under H₂-atmosphere (1 bar) at room temperature. The catalysator was removed by Celite^{®} 535 filtration then the solvent was removed. The residue was purified by flash chromatography (*n*-hexane : ethyl acetate 5 : 5 + 1 % *N,N-*dimethylethanamine, ∅ 2 cm, fraction size 10 mL, R_{f}= 0.36). The mixture of the titled compounds was obtained as a pale yellow solid.
The separation of the enatiomeres (1R,2S)-2-Methyl-3-(4-phenylbutyl)-2,3,4,5-tetrahydro-1*H*-3-benzazepine-1,7-diol and (1S,2R)-2-Methyl-3-(4-phenylbutyl)-2,3,4,5-tetrahydro-1*H*-3-benzazepine-1,7-diol war carried out on a chiral preparative HPLC (*n*-hexane : ethanol (90 : 10), t_{R} ((1R,2S)-2-Methyl-3-(4-phenylbutyl)-2,3,4,5-tetrahydro-1*H*-3-benzazepine-1,7-diol) = 69.7 min, t_{R} ((1S,2R)-2-Methyl-3-(4-phenylbutyl)-2,3,4,5-tetrahydro-1*H*-3-benzazepine-1,7-diol) = 77.9 min).
The compound 1R,2S)-2-Methyl-3-(4-phenylbutyl)-2,3,4,5-tetrahydro-1*H*-3-benzazepine-1,7-diol was obtained as a pale yellow resin.
The compound (1S,2R)-2-Methyl-3-(4-phenylbutyl)-2,3,4,5-tetrahydro-1*H*-3-benzazepine-1,7-diol was obtained as a pale yellow oil.

### Example 32

### Preparation of (1S,2S)-2-Methyl-3-(4-phenylbutyl)-2,3,4,5-tetrahydro-1H-3-benzazepine-1,7-diol

### Step 32.1 Preparation of (R)-Alaninmethylester-hydrochloride

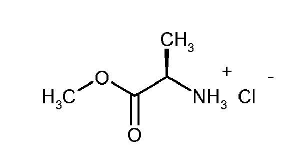

The procedure was carried out as described for (S)-alaninmethylester-hydrochloride in step 31.1 in CH₃OH (112 mL), SOCl₂ (23.9 mL, 0.33 mol) und (R)-alanine (10.0 g, 0.11 mol). The titled compound was obtained as a colourless solid.

### Step 32.2 Preparation of (R)-Methyl-2-(4-tosyl)aminopropionate

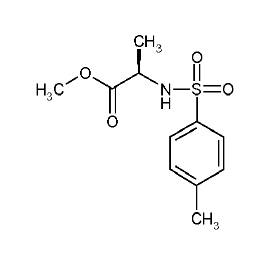

The procedure was carried out as described for (S)-methyl-2-(4-tosyl)aminopropionate in step 31.2 with (R)-alaninmethylester-hydrochloride (9.64 g, 69.4 mmol), CH₂Cl₂ (160 mL), p-toluolsulfonylchloride (19.8 g, 97.0 mmol) und triethylamine (15.45 mL, 0.15 mol).

### Step 32.3 Preparation of 2-(3-Benzyloxyphenyl)-ethanol

The procedure was carried out as described in step 31.3.

### Step 32.4 Preparation of (R)-Methyl-2-{N-[2-(3-benzyloxyphenyl)ethyl]-N-(4-tosyl)amino}-propionate

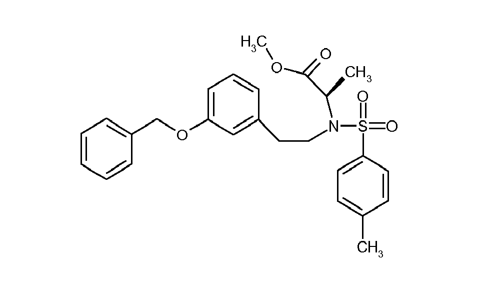

The procedure was carried out as described above in step 31.4 with (R)-Methyl-2-(4-tosyl)aminopropionate (3.83 g, 14.9 mmol), Ph₃P (10.4 g, 39.6 mmol), DIAD (7.68 mL, 39.6 mmol) and abs. THF (240 mL). The titled compound was obtained as colourless oil.

### Step 32.5 Preparation of (R)-2-{N-[2-(3-Benzyloxyphenyl)ethyl]-N-(4-tosyl)amino}propion acid

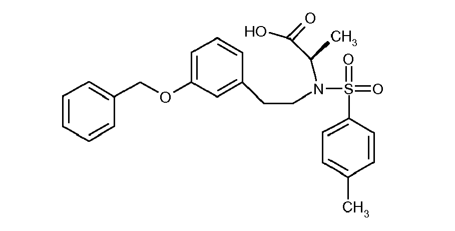

The procedure was carried out as described above in step 31.5 with (R)-methyl-2- {*N*-[2-(3-benzyloxyphenyl)ethyl]-*N*-(4-tosyl)amino}propionate (4.40 g, 9.42 mmol), LiOH·H₂O (3.38 g, 80.4 mmol) and THF (130 mL). The titled compound was obtained as colourless oil.

### Step 32.6 Preparation of (R)-7-Benzyloxy-2-methyl-3-(4-tosyl)-2,3,4,5-tetrahydro-3-benzazepine-1-one in mixture with (R)-9-Benzyloxy-2-methyl-3-(4-tosyl)-2,3,4,5-tetrahydro-3-benzazepine-1-one

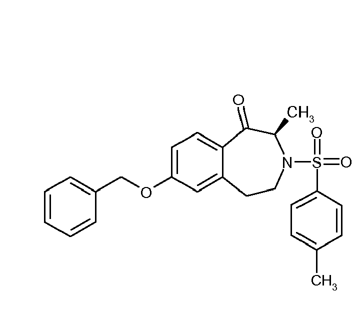

in mixture with 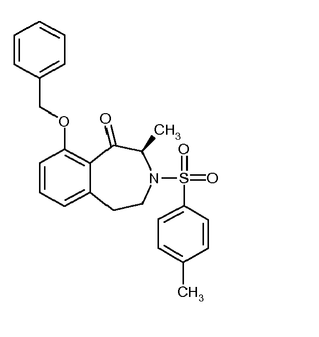 The procedure was carried out as described above in step 31.6 with (R)-2-{*N*-[2-(3-Benzyloxyphenyl)ethyl]-*N*-(4-tosyl)amino}propinsäure (3.95 g, 8.72 mmol), CH₂Cl₂ (250 mL), trifluoracetic acid anhydride (3.69 mL, 26.2 mmol) and SnCl₄ (3.06 mL, 26.16 mmol).
The mixture of the titled compounds was obtained as a pale yellow solid.
The R_{f} of the regioisomers was 0.34. The compounds were further used as mixture.

### Step 32.7 Preparation of (1S,2R)-7-Benzyloxy-2-methyl-3-(4-tosyl)-2,3,4,5-tetrahydro-1H-3-benzazepine-1-ol and

### (1R,2R)-7-Benzyloxy-2-methyl-3-(4-tosyl)-2,3,4,5-tetrahydro-1H-3-benzazepine-1-ol

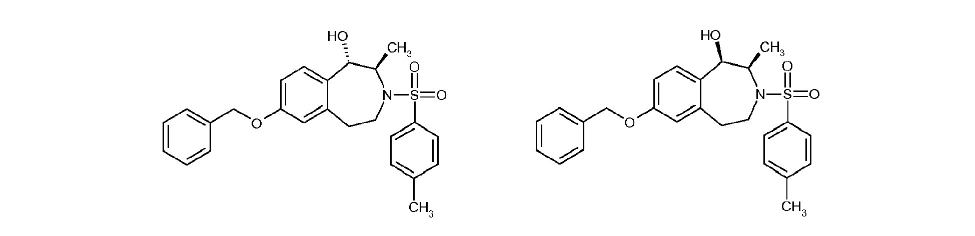

The procedure was carried out as described above in step 31.6 with the mixture of (R)-7-Benzyloxy-2-methyl-3-(4-tosyl)-2,3,4,5-tertrahydro-3-benzazepine-1-one and (R)-9-Benzyloxy-2-methyl-3-(4-tosyl)-2,3,4,5-tetrahydro-3-benzazepine-1-one (5.20 g, 12.0 mmol), NaBH₄ (909.2 mg, 23.9 mmol) and CH₃OH (125 mL).
The compound (1S,2R)-7-Benzyloxy-2-methyl-3-(4-tosyl)-2,3,4,5-tetrahydro-1*H*-3-benzazepine-1-ol was obtained as a colourless solid.
The compound (1R,2R)-7-Benzyloxy-2-methyl-3-(4-tosyl)-2,3,4,5-tetrahydro-1*H*-3-benzazepine-1-ol was obtained as a colourless solid.

### Step 32.8 Preparation of (1S,2S)-7-Benzyloxy-2-methyl-2,3,4,5-tetrahydro-1H-3-benzazepine-1-ol in mixture with

### (1R,2R)-7-Benzyloxy-2-methyl-2,3,4,5-tetrahydro-1H-3-benzazepine-1-ol

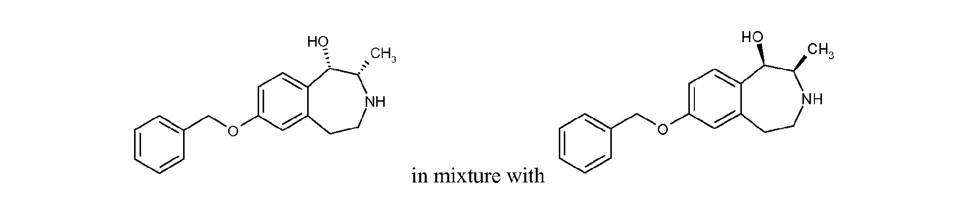

To the compounds (1R,2R)-7-Benzyloxy-2-methyl-3-(4-tosyl)-2,3,4,5-tetrahydro-1*H*-3-benzazepine-1-ol of step 32.7 and (1S,2S)-7-Benzyloxy-2-methyl-3-(4-tosyl)-2,3,4,5-tetrahydro-1*H*-3-benzazepine-1-ol of step 32.7 and step 31.7 (1.13 g, 2.58 mmol) dissolved in CH₃OH (130 mL) was added Mg (1.37 g, 56.9 mmol). The reaction mixture was heated at reflux for 36h. Afterwards the pH was adjusted with 2M HCl to pH=3 and unspent Mg was filtered, followed by the addition of NaOH to adjust to an alkaline pH-value (pH 10) of the filtrate. The water phase was subsequently extracted with CH₂Cl₂ (5 x 50 mL). The combined organic layers were dried with Na₂SO₄ and the solvent was evaporated.
The residue was purified by flash chromatography (ethyl acetate CH₃OH 9.5 : 0.5 + 2 % *N*,*N-*dimethylethanamine, ∅ 6 cm, fraction size 30 mL, R_{f}= 0.23). The mixture of the titled compounds was obtained as a pale yellow solid.

### Step 32.9 Preparation of (1S,2S)-7-Benzyloxy-2-methyl-3-(4-phenylbutyl)-2,3,4,5-tetrahydro-1H-3-benzazepine-1-ol and

### (1R,2R)-7-Benzyloxy-2-methyl-3-(4-phenylbutyl)-2,3,4,5-tetrahydro-1H-3-benzazepine-1-ol

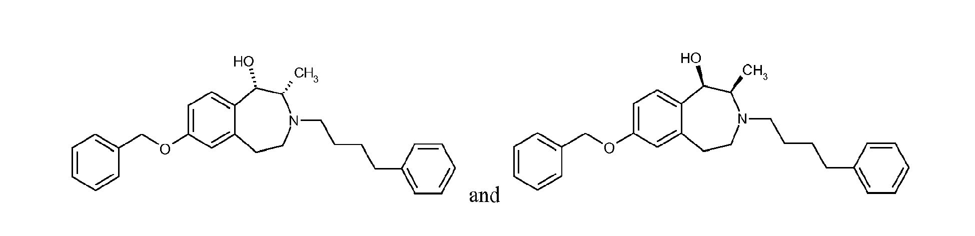

The mixture of compound (1S,2S)-7-Benzyloxy-2-methyl-2,3,4,5-tetrahydro-1*H*-3-benzazepine-1-ol and (1R,2R)-7-Benzyloxy-2-methyl-2,3,4,5-tetrahydro-1*H*-3-benzazepine-1-ol of step 32.8 (122.2 mg, 0.43 mmol) was added 1-chlor-4-phenylbutane (108.0 µL, 0.66 mmol), TBAI (241.0 mg, 0.65 mmol) and K₂CO₃ (301.0 mg, 2.18 mmol) in CH₃CN (20 mL) and subsequently heated at reflux for 72h. The precipitation was filtered and the solvent was evaporated. The residue was purified by flash chromatography (*n*-hexane : ethyl acetate 7 : 3 + 1 % *N*-*N*-dimethylethanamine, ∅ 3 cm, fraction size 10 mL, R_{f}= 0.22). The mixture of the titled compounds was obtained as a colourless solid.
The separation of the enatiomeres compound (1S,2S)-7-Benzyloxy-2-methyl-3-(4-phenylbutyl)-2,3,4,5-tetrahydro-1*H*-3-benzazepine-1-ol and (1R,2R)-7-Benzyloxy-2-methyl-3-(4-phenylbutyl)-2,3,4,5-tetrahydro-1*H*-3-benzazepine-1-ol war carried out on a chiral preparative HPLC (*n*-hexane : ethanol: isopropanol 96 : 3 : 1, flow rate 6.9 mL/min, t_{R} ((1S,2S)-7-Benzyloxy-2-methyl-3-(4-phenylbutyl)-2,3,4,5-tetrahydro-1*H*-3-benzazepine-1-ol) = 48.6 min, t_{R} ((1R,2R)-7-Benzyloxy-2-methyl-3-(4-phenylbutyl)-2,3,4,5-tetrahydro-1*H*-3-benzazepine-1-ol) = 58.6 min).
The titled compound (1S,2S)-7-Benzyloxy-2-methyl-3-(4-phenylbutyl)-2,3,4,5-tetrahydro-1*H*-3-benzazepine-1-ol was obtained as colourless resin.
The titled compound (1R,2R)-7-Benzyloxy-2-methyl-3-(4-phenylbutyl)-2,3,4,5-tetrahydro-1*H*-3-benzazepine-1-ol was obtained as colourless resin.

### Step 32.10 Preparation of (1S,2S)-2-Methyl-3-(4-phenylbutyl)-2,3,4,5-tetrahydro-1H-3-benzazepine-1,7-diol

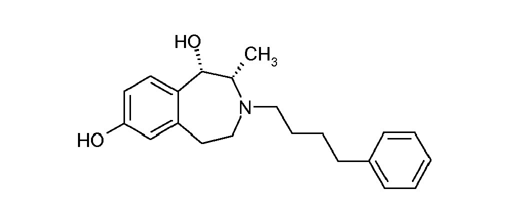

To compound (1S,2S)-7-Benzyloxy-2-methyl-3-(4-phenylbutyl)-2,3,4,5-tetrahydro-1*H*-3-benzazepine-1-ol (60.1 mg, 0.14 mmol) dissolved in abs. CH₃OH (6 mL) was added Pd/C (50.0 mg, 10 %) The suspension was stirred 1 h under H₂-atmosphere (1 bar) at room temperature. The catalysator was removed by Celite^{®} 535 filtration then the solvent was removed. The residue was purified by flash chromatography (*n*-hexane : ethyl acetate: 5 + 1 % *N*,*N*-dimethylethanamine, ∅ 2 cm, fraction size 10 mL, R_{f}= 0.13). The titled compound was obtained as a colourless solid.

### Example 33

(1R,2R)-2-Methyl-3-(4-phenylbutyl)-2,3,4,5-tetrahydro-1*H*-3-benzazepine-1,7-diol 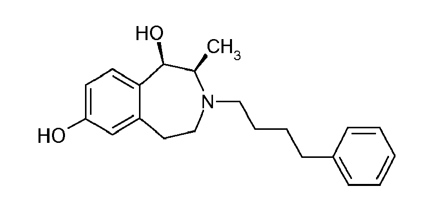 The procedure was carried out as described above in Step 32.10 with compound (1R,2R)-7-Benzyloxy-2-methyl-3-(4-phenylbutyl)-2,3,4,5-tetrahydro-1*H*-3-benzazepine-1-ol (73.6 mg, 0.18 mmol), CH₃OH (6 mL) und Pd/C (50.2 mg, 10 %). The titled compound was obtained as a colourless solid.

### Example 34

Determination of the affinity to the polyamine binding site of the NR2B subunit of the NMDA receptor
The determination was performed using a filtration-based receptor binding assay on 96-well-multiplates for the polyamine binding site of the NR2B subunit of the NMDA receptor using [³H]-Ifenprodil as radioligand.

### Cell culture and preparation of membrane homogenates

Mouse L(tk-) cells stably transfected with the dexamethasone-inducible eukaryotic expression vectors pMSG NR1-1a, pMSG NR2B in a 1:5 ratio were a generous gift from D. Steinhilber (Department of Pharmacy, University of Frankfurt, Germany). The transformed L(tk-) cells were grown in Modified Earl's Medium (MEM) containing 10% of standardized FCS (Biochrom AG, Berlin, Germany). The expression of the NMDA receptor at the cell surface was induced after the cell density of the adherent growing cells had reached approximately 90% of confluency. For the induction, the original growth medium was replaced by growth medium containing 4 µM dexamethasone and 4 µM ketamine (final concentration). After 24 h the cells were harvested by trypsination and pelleted (10 min, 5,000 x g, Hettich Rotina 35R centrifuge, Tuttlingen, Germany).

For the binding assay, the cell pellet was resuspended in PBS buffer and the number of cells was determined using an improved Neubauer's counting chamber (VWR, Darmstadt, Germany). Subsequently, the cells were lysed by sonication (4 °C, 6 x 10 s cycles with breaks of 10 sec). The resulting cell fragments were centrifuged with a high performance cool centrifuge (20,000 x g, 4 °C, Sorvall RC-5 plus, Thermo Scientific). The supernatant was discarded and the pellet resuspended in a defined volume of phosphate buffer saline (PBS) yielding cell fragments of approximately 500,000 cells/mL. The suspension of membrane homogenates was sonicated again (4 °C, 2 x 10 s cycles with a break of 10 min) and stored at -80 °C.

### Receptor binding assay

The competitive binding assay was performed with the radioligand [³H]-Ifenprodil (60 Ci/mmol; Perkin Elmer) using standard 96-well-multiplates (Diagonal, Muenster, Germany). The thawed cell membrane preparation (about 20 µg protein) was incubated with 6 different concentrations of test compounds usually 10 µM, 1 µM, 100 nM, 10 nM, 1 nM and 0.1 µM, 5 nM [³H]-Ifenprodil, and TRIS/EDTA-buffer (5 mM/1 mM, pH 7.5) in a total volume of 200 µL for 180 min at 37 °C. The cell membrane suspension was added last. All experiments were carried out in triplicates. The incubation was terminated by rapid filtration through filtermats using a cell harvester (MicroBeta FilterMate-96 Harvester, Perkin Elmer). Prior to harvesting, the filtermats were presoaked in 0.5% aqueous polyethylenimine for 2 h at room temperature. After washing each well five times with 300 µL of water, the filtermats were dried at 95 °C. Subsequently, the solid scintillator was placed on the filtermat and melted at 95 °C. After 5 min, the solid scintillator was allowed to solidify at room temperature. The bound radioactivity trapped on the filters was counted in the scintillation analyzer (Microbeta Counter, Perkin Elmer). The overall counting efficiency was 20%. The non-specific binding was determined with 10 µM unlabeled Ifenprodil.

### Protein determination

The protein concentration was determined by the method of Bradford, modified by Stoscheck. The Bradford solution was prepared by dissolving 5 mg of Coomassie Brilliant Blue G 250 in 2.5 mL EtOH (95%, v/v). 10 mL deionized H₂O and 5 mL phosphoric acid (85%, m/v) were added to this solution, the mixture was stirred and filled up to a total volume of 50.0 mL with deionized water. The calibration was carried out using bovine serum protein as a standard in concentrations between 0.1 and 10 mg /L. In a 96-well standard multiplate, 10 µL of the calibration solution or 10 µL of the membrane receptor preparation were mixed with 190 µL of the Bradford solution, respectively. After 5 min, the UV absorption of the protein-dye complex at λ = 595 nm was measured with a platereader (Tecan Genios, Tecan, Crailsheim, Germany).

### Saturation experiment

The saturation analysis was performed by incubating increasing concentrations of [³H]-Ifenprodil (0.5 nM, 1 nM, 2.5 nM, 5 nM, 10 nM, 15 nM, 20 nM, 25 nM and 50 nM) together with 20 µg of the receptor protein in TRIS/EDTA-buffer (5 mM/1 mM, pH 7.5) for 2 h at 37 °C. For each concentration, the nonspecific binding was determined with an excess of non-labelled Ifenprodil (10 µM). K_{d} and Bₘₐₓ were calculated as described in the "Data analysis" section.

### Data Analysis

Data analysis was performed with Graph Pad Prism® Software, Version 3.0 (Graph Pad Software Inc., San Diego, CA, USA).

Saturation analyses were made by nonlinear regression using the "one-site-saturation" calculation method. The IC₅₀ values of the test compounds used in the competitive binding experiments were determined by nonlinear regression using the "one-site-competition" calculation method. Subsequently, the Kᵢ values of the test compounds were calculated according to the equation of Cheng and Prusoff (Y. Cheng, H. W. Prusoff, Biochem. Pharmacol., 1973, 22, 3099-3108). The Kᵢ values are given as mean values from three independent experiments ± Standard Error of the Mean (S.E.M).

The determination of the affinity to the polyamine binding site of the NR2B subunit of the NMDA receptor was performed for the compounds according to formulas (1) to (30).

It was observed that most of the compounds according to formulas (1) to (30) showed an affinity to the polyamine binding site of the NR2B subunit of the NMDA receptor in the nanomolecular range. The compounds according to formulas (1) to (30) showed an affinity to the NR2B binding site of the NMDA receptor of 5.12 nM, 591 ± 150 nM, 192 ± 26 nM, 15.3 ± 1.2 nM, 325 nM, 258 nM, 1.04 µM, 1.88 µM, 249 ± 63 nM, 45.2 nM, 107 nM, 46 nM, 99 nM, 60.4 nM, 1.24 µM, 7.68 µM, 209 nM, 586 nM, 156 nM, 2.51 µM, 4.86 nM, 23.4 nM, 34.2 nM, 47.3 nM, 1.12 nM, 393 nM, 12.3 nM, 22.1 nM, 171 nM, and 16.4 nM, respectively.

This shows that the compounds according to formulas (1) to (30) exhibit a high affinity to the polyamine binding site of the NR2B subunit.

### Example 35

Determination of the affinity to the phencyclidine binding site of the NMDA receptor

### Materials and general procedures

The pig brains were a donation of the local slaughterhouse (Coesfeld, Germany). Homogenizer: Elvehjem Potter (B. Braun Biotech International, Melsungen, Germany). Centrifuge: High-speed cooling centrifuge model Sorvall RC-5C plus (Thermo Fisher Scientific, Langenselbold, Germany). Filter: Printed Filtermat Typ A and B (Perkin Elmer LAS, Rodgau-Jügesheim, Germany), presoaked in 0.5% aqueous polyethylenimine for 2 h at room temperature before use. TRIS/EDTA-buffer was prepared by dissolving 606 mg Tris-Base and 372 mg Na-EDTA (both from Sigma-Aldrich, Deisenhofen, Germany) in 900 mL H₂O. Before filling to the final volume of 1000 mL, the pH of the solution was adjusted to pH=7.5 by dropwise addition of 1M HCl. The filtration was carried out with a MicroBeta FilterMate-96 Harvester (Perkin Elmer). The scintillation analysis was performed using Meltilex (Typ A or B) solid scintillator (Perkin Elmer). The solid scintillator was melted on the filtermat at a temperature of 95 °C for 5 minutes. After solidifying of the scintillator at room temperature, the scintillation was measured using a MicroBeta Trilux scintillation analyzer (Perkin Elmer). The overall counting efficiency was 20 %. All experiments were carried out in triplicates using standard 96-well-multiplates (Diagonal, Muenster, Germany). The IC₅₀-values were determined in competition experiments with at least six concentrations of the test compounds, usually 10 µM, 1 µM, 100 nM, 10 nM, 1 nM and 0.1 nM, and were calculated with the program GraphPad Prism^{®} 3.0 (GraphPad Software, San Diego, CA, USA) by non-linear regression analysis. The Kᵢ-values were calculated according to the formula of Cheng and Prusoff (Y. Cheng, H. W. Prusoff, Biochem. Pharmacol., 1973, 22, 3099-3108). The Kᵢ-values are given as mean value ± SEM from three independent experiments.

### Preparation of the tissue

Fresh pig brain cortex was homogenized with the potter (500-800 rpm, 10 up-and-down strokes) in 6 volumes of cold 0.32 M sucrose. The suspension was centrifuged at 1200 x g for 10 min at 4°C. The supernatant was separated and centrifuged at 23500 x g for 20 min at 4 °C. The pellet was resuspended in 5-6 volumes of TRIS/EDTA-buffer (5 mM/1 mM, pH 7.5) and centrifuged again at 31000 x g (20 min, 4 °C). This procedure was repeated twice. The final pellet was resuspended in 5-6 volumes of buffer, the protein concentration was determined according to the method of Bradford (M. M. Bradford, Anal. Biochem., 1976, 72, 248-254) using bovine serum albumin as standard, and subsequently the preparation was frozen (-80°C) in 1.5 mL portions containing about 0.8 mg protein/mL.

### Performance of the assay

The test was performed with the radioligand [³H]-(+)-MK-801 (22.0 Ci/mmol; Perkin Elmer). The thawed membrane preparation (about 100 µg of the protein) was incubated with various concentrations of test compounds, 2 nM [³H]-(+)-MK-801, and TRIS/EDTA-buffer (5 mM/1 mM, pH 7.5) in a total volume of 200 µL for 150 min at room temperature. The incubation was terminated by rapid filtration through the presoaked filtermats using a cell harvester. After washing each well five times with 300 µL of water, the filtermats were dried at 95 °C. The bound radioactivity trapped on the filters was counted in the scintillation analyzer as described in the "General procedures" section. The non-specific binding was determined with 10 µM unlabeled (+)-MK-801. The K_{d}-value of (+)-MK-80 is 2.26 nM (T. Utech, Dissertation, 2003, Universität Freiburg).

The determination of the affinity to the phencyclidine binding site of the NMDA receptor was performed for the compounds according to formulas (1) to (30).

It was observed that the compounds according to formulas (1) to (30) showed only minor affinity to the phencyclidine binding site of the NMDA receptor, wherein inhibition of the phencyclidine binding site only was detected at concentrations above 1 µM or 10 µM.

This shows that the compounds according to formulas (1) to (30) exhibit a good selectivity to the NR2B subunit of the NMDA receptor in comparison to the phencyclidine binding site of the NMDA receptor.

### Example 36

### Determination of the affinity to the σ₁ receptor

Unless defined otherwise, materials and general procedures were as described according to example 35.

### Preparation of the tissue

Five guinea pig brains (Harlan Winkelmann, Borchen, Germany) were homogenized with the potter (500-800 rpm, 10 up-and-down strokes) in 6 volumes of cold 0.32 M sucrose. The suspension was centrifuged at 1200 x g for 10 min at 4 °C. The supernatant was separated and centrifuged at 23500 x g for 20 min at 4 °C. The pellet was resuspended in 5-6 volumes of buffer (50 mM TRIS, pH 7.4) and centrifuged again at 23500 x g (20 min, 4 °C). This procedure was repeated twice. The final pellet was resuspended in 5 to 6 volumes of buffer, the protein concentration was determined according to the method of Bradford using bovine serum albumin as standard, and subsequently the preparation was frozen (-80 °C) in 1.5 mL portions containing about 1.5 mg protein/mL.

### Performance of the assay

The test was performed with the radioligand [³H]-(+)-pentazocine (42,5 Ci/mmol; Perkin Elmer). The thawed membrane preparation (about 75 µg of the protein) was incubated with various concentrations of test compounds, 2 nM [³H]-(+)-pentazocine, and buffer (50 mM TRIS, pH 7.4) in a total volume of 200 µL for 180 min at 37 °C. The incubation was terminated by rapid filtration through the presoaked filtermats using a cell harvester. After washing each well five times with 300 µl of water, the filtermats were dried at 95 °C. The bound radioactivity trapped on the filters was counted in the scintillation analyzer as described in the "General procedures" section. The non-specific binding was determined with 10 µM unlabeled (+)-pentazocine. The K_{d}-value of (+)-pentazocine is 2.9 nM.

The determination of the affinity to the σ₁ receptor was performed for the compounds according to formulas (1) to (30).

It was observed that the compounds according to formulas (1) to (5), (7) to (14), (16), (17), (18), (20) to (25), and (27) to (30) showed an affinity to the σ₁ receptor of 182 ± 67 nM, 44.6 ± 21.4 nM, 33.1 ± 29.8 nM, 194 nM, 65.3± 12.3 nM, 94.7± 51 nM, 155 nM, 293 ± 101 nM, 349 nM, 45.6 nM, 376 nM, 604 nM, 44.3 ± 14.1 nM, 172 nM, 42.1 ± 9.9 nM, 3.21 ± 1.42 nM, 10.4 ± 1.4 nM, 123 ± 19 nM, 82.2 nM, 200 ± 62 nM, 1.05 µM, 183 nM, 85.5 nM, 393 nM, 40.8 nM, and 846 nM, respectively, wherein the compounds according to formulas (6), (15), (19), and (26) showed inhibition of the σ₁ receptor only at concentrations above 1 µM or 10 µM.

This shows that the compounds according to formulas (1) to (5), (7) to (14), (16), (17), (18), (20) to (25), and (27) to (30) showed a good affinity to the σ₁ receptor, too.

### Example 37

### Determination of affinity to the σ₂ receptor

Unless defined otherwise, materials and general procedures were as described according to example 35.

### Preparation of the tissue

Two rat livers (Harlan Winkelmann, Borchen, Germany) were cut into smaller pieces and homogenized with the potter (500-800 rpm, 10 up-and-down strokes) in 6 volumes of cold 0.32 M sucrose. The suspension was centrifuged at 1200 x g for 10 min at 4 °C. The supernatant was separated and centrifuged at 31000 x g for 20 min at 4 °C. The pellet was resuspended in 5-6 volumes of buffer (50 mM TRIS, pH 8.0) and incubated at room temperature for 30 minutes. After the incubation, the suspension was centrifuged again at 31000 x g for 20 min at 4 °C. The final pellet was resuspended in 5 to 6 volumes of buffer, the protein concentration was determined according to the method of Bradford using bovine serum albumin as standard, and subsequently the preparation was frozen (-80 °C) in 1.5 mL portions containing about 2 mg protein/mL.

### Performance of the assay

The test was performed with the radioligand [³H]-ditolylguanidine (50 Ci/mmol; ARC). The thawed membrane preparation (about 100 µg of the protein) was incubated with various concentrations of test compounds, 3 nM [³H]-ditolylguanidine, and buffer containing (+)-pentazocine (2 µM (+)-pentazocine in 50 mM TRIS, pH 8.0) in a total volume of 200 µL for 180 min at room temperature. The incubation was terminated by rapid filtration through the presoaked filtermats using a cell harvester. After washing each well five times with 300 µL of water, the filtermats were dried at 95 °C. Subsequently, the solid scintillator was placed on the filtermat and melted at 95 °C. The bound radioactivity trapped on the filters was counted in the scintillation analyzer as described in the "General procedures" section. The non-specific binding was determined with 10 µM unlabeled ditolylguanidine. The K_{d}-value of ditolylguanidine is 17.9 nM.

The determination of the affinity to the σ₂ receptor was performed for the compounds according to formulas (1) to (28).

It was observed that the compounds according to formulas (1) to (3), (5), (7) to (14), (16), (17), (18), and (20) to (28), showed an affinity to the σ₂ receptor of 554 ± 221 nM, 108 ± 90 nM, 81.6 ± 44.4 nM, 305 nM, 308 ± 116 nM, 1.52 µM, 1.05 µM, 3.04 µM, 103 nM, 653 nM, 6.43 µM, 267 ± 55 nM, 1.58 µM, 6.08 ± 1.48 nM, 18.5 ± 4.2 nM, 311 nM, 8.56 ± 4.33 nM, 5.89 ± 2.86 nM, 8.93 ± 2.57 nM, 33.5± 21.5 nM, 753 nM, 97.7 nM, 142 nM, and 103 nM, respectively, wherein the compounds according to formulas (4), (6), (15), and (19) showed inhibition of the σ₂ receptor only at concentrations above 1 µM.

This shows that the compounds according to formulas (1) to (3), (5), (7) to (14), (16), (17), (18), and (20) to (28), showed a good affinity to the σ₂ receptor, too.

## Claims

1. Compounds according to general formula (I) as given as follows and/or racemates, enantiomers, diastereomers, solvates, hydrates, and pharmaceutically acceptable salts and/or esters thereof: 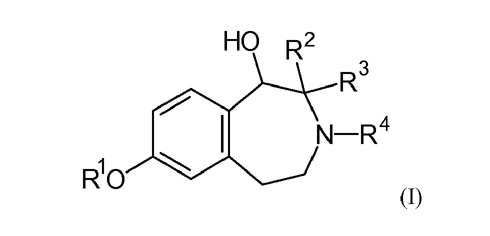 wherein:
R¹ is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; and/or C₇- C₁₄-arylalkyl wherein the aryl group has 6 to 10 carbon atoms and the alkyl group has 1 to 4 carbon atoms;
R² is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms;
R³ is selected from the group comprising hydrogen; linear or branched C₁-C₈-alkyl; C₂-C₈- alkenyl; C₃-C₈-cycloalkyl; C₆-C₁₀-aryl; C₄-C₁₀-cycloalkylalkyl wherein the cycloalkyl group has 3 to 6 carbon atoms and the alkyl group has 1 to 4 carbon atoms; C₇-C₁₄- arylalkyl wherein the aryl group has 6 to 10 carbon atoms and the alkyl group has 1 to 4 carbon atoms; linear or branched alkyl groups of the type -CₙH₂ₙ-U-D wherein n is 1, 2, 3 or 4, U is selected from the group comprising O, CO, COO, CONH, S, guanidine and/or NH and D is selected from the group comprising H
or and/or C₁-C₃-alkyl; -CH₂-C₆H₄-X wherein X is selected from the group comprising OH, SH, C₁-C₃-alkyl and/or NH₂; -CH₂-imidazole; -CH₂-indole; -CH₂-(furanyl-3-yl); -CH₂- (pyridyl-3-yl) and/or -CH₂-(imidazolyl-3-yl);
R² and R³ together with the carbon atom to which they are attached form a 5- to 7-membered non aromatic carbocycle or heterocycle comprising from 1 to 3 hetero atoms selected from the group comprising O, N and/or S;
R⁴ is selected from the group comprising hydrogen; C₁-C₁₀-alkyl; -W and/or -Y-Z;
Y is selected from the group comprising C₁-C₆-alkyl; C₂-C₆-alkenyl; C₂-C₆-alkynyl, C₃-C₆-cycloalkyl; C₄-C₁₀-cycloalkylalkyl wherein the cycloalkyl group has 3 to 6 carbon atoms and the alkyl group has 1 to 4 carbon atoms; C₆-C₁₀-aryl; C₇-C₁₄-arylalkyl wherein the aryl group has 6 to 10 carbon atoms and the alkyl group has 1 to 4 carbon atoms; C₁-C₆-alkyl comprising at least one moiety independently selected from the group comprising oxygen, sulphur, SO, SO₂, CO, NH, N(C₁-C₃-alkyl) and/or T; a 3- to 6-membered aromatic or non aromatic carbocycle or heterocycle containing at least one of O, N or S as heteroatoms; and/or a structural element comprising a 3- to 6-membered aromatic or non aromatic carbocycle or heterocycle comprising from 1 to 3 heteroatoms selected from the group comprising O, N and/or S and a group selected from the group comprising oxygen, sulphur, SO, SO₂, CO, NH, N(C₁-C₃-alkyl) and/or C₁-C₆-alkyl comprising at least one moiety independently selected from the group comprising oxygen, sulphur, SO, SO₂, CO, NH and/or N(C₁-C₃-alkyl);
T is selected from the group comprising 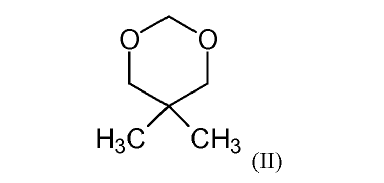
W is selected from the group comprising 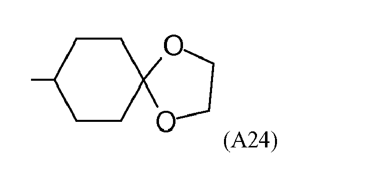
Z is selected from the group comprising mono-, bi- or tricyclic aromatic or non aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl;
or R³ and R⁴ together with the ring atoms to which they are attached form a 5- to 7-membered non aromatic heterocycle comprising from 1 to 3 heteroatoms selected from the group comprising O, N and/or S.

2. Compounds according to claim 1, **characterized in that**
R² is hydrogen and R³ is a side chain of an amino acid selected from the group comprising hydrogen; linear or branched C₁-C₄-alkyl; linear or branched alkyl groups of the type - CₙH₂ₙ-U-D wherein n is 1, 2, 3 or 4, U is selected from the group comprising O, CO, COO, CONH, S, guanidine and/or NH, and D is selected from the group comprising H and/or methyl; -CH₂-C₆H₄-OH; -CH₂-imidazole and/or -CH₂-indole; or
R³ and R⁴ together with the ring atoms to which they are attached form a 5-membered non aromatic heterocycle having one N atom.

3. Compounds according to claims 1 or 2, **characterized in that**
R⁴ is selected from the group comprising the structural elements as given as follows:

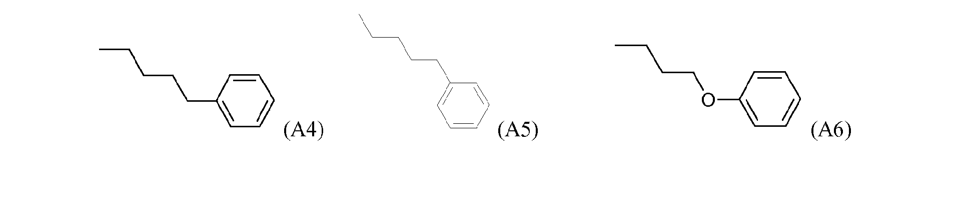

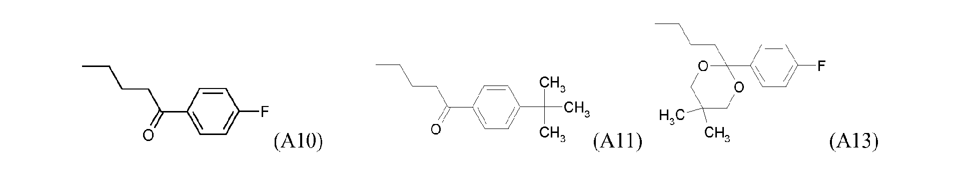

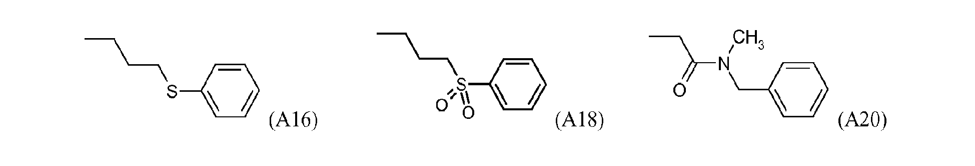

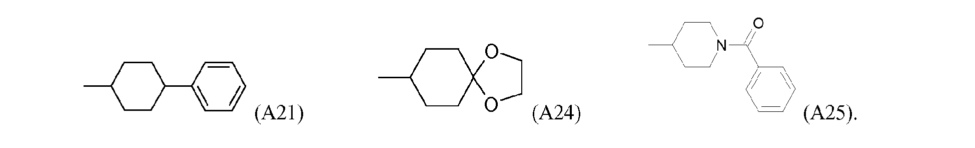

4. Compounds according to any one of the foregoing claims, **characterized in that** the compound is a compounds according to general formula (III) as given as follows 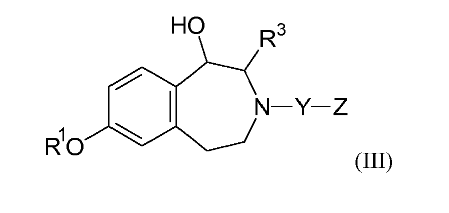 wherein:
R¹ is selected from the group comprising hydrogen; linear or branched C₁-C₆-alkyl and/or benzyl;
R³ is a side chain of an amino acid selected from the group comprising hydrogen; linear or branched C₁-C₄-alkyl; linear or branched alkyl groups of the type -CₙH₂ₙ-U-D wherein n is 1, 2, 3 or 4, U is selected from the group comprising O, CO, COO, CONH, S, guanidine and/or NH, and D is selected from the group comprising H and/or methyl; -CH₂-C₆H₄-OH; -CH₂-imidazole and/or -CH₂-indole;
Y is selected from the group comprising -(CH₂)ₘ- wherein m represents 3, 4 or 5; C₃-C₅-alkenyl; C₅-C₆-cycloalkyl; C₃-C₅-alkyl comprising at least one moiety independently selected from the group comprising oxygen, sulphur, SO, SO₂, CO, NH and/or N(C₁-C₃-alkyl); a 5- to 6-membered non aromatic carbocycle or heterocycle comprising from 1 to 3 heteroatoms selected from the group comprising O, N and/or S; and/or a structural element comprising a 5- to 6-membered non aromatic carbocycle and a group selected from the group comprising oxygen, sulphur, SO, SO₂, CO, NH, N(C₁-C₃- alkyl) and/or C₁-C₃-alkyl comprising at least one moiety independently selected from the group comprising oxygen, sulphur, SO, SO₂, CO, NH and/or N(C₁-C₃-alkyl);
Z is selected from the group comprising mono-, bi- or tricyclic aromatic carbocycles or heterocycles comprising from 1 to 3 heteroatoms selected from the group comprising O, N and/or S, wherein the carbocycle or heterocycle optionally is substituted by at least one group selected from the group comprising halogen, cyano, OH, CF₃, C₁-C₄-alkyloxy and/or C₁-C₆-alkyl.

5. Compounds according to any one of the foregoing claims, **characterized in that** the compound is selected from the group comprising compounds according to the formulas as given as follows:

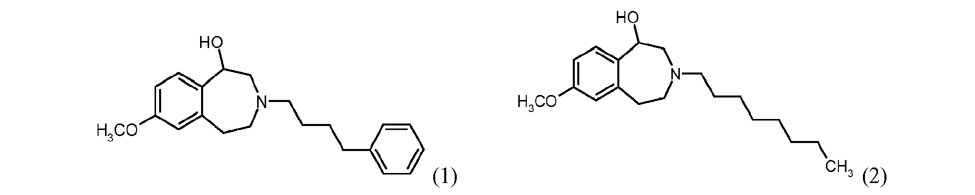

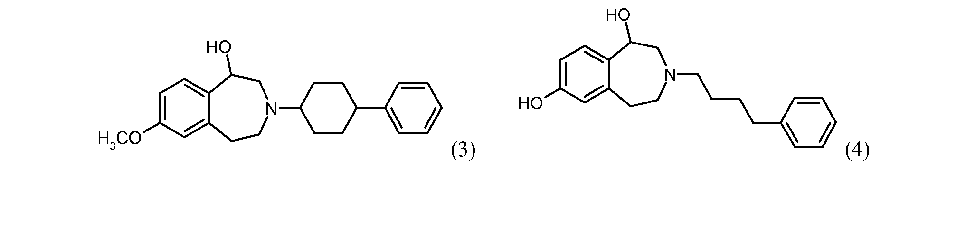

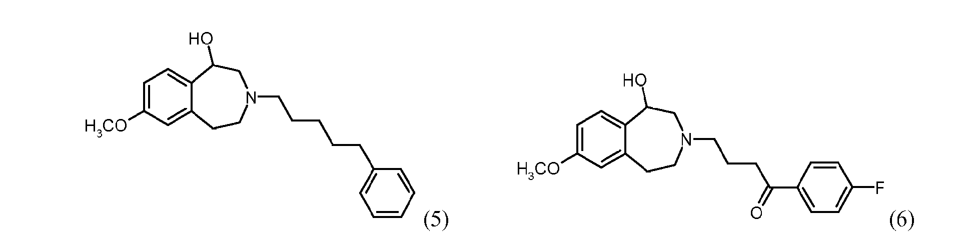

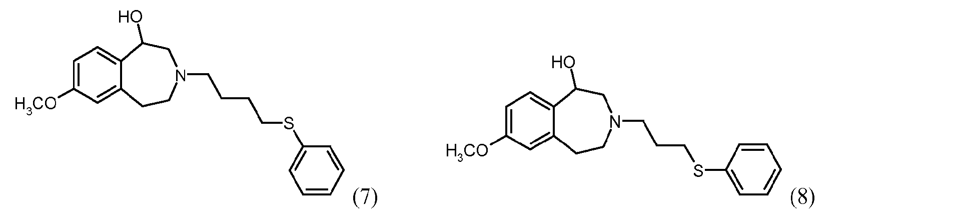

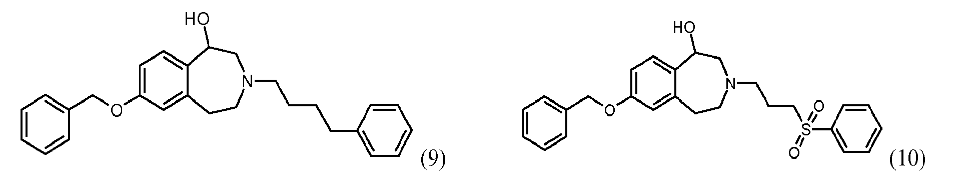

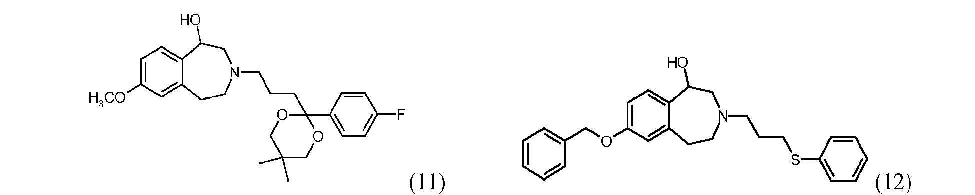

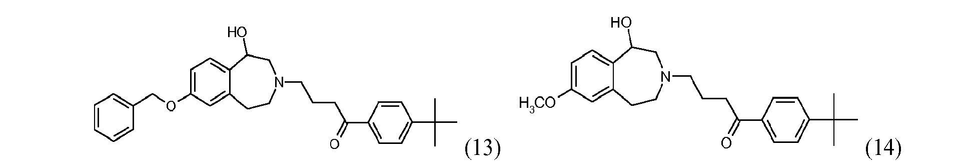

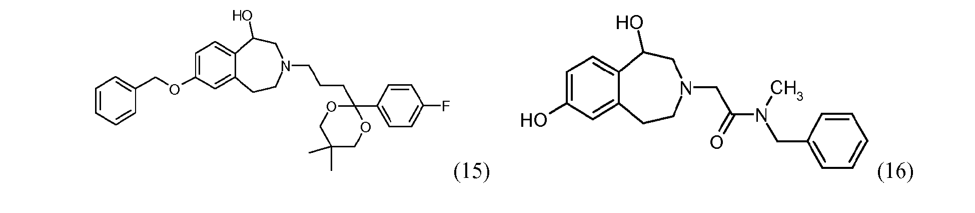

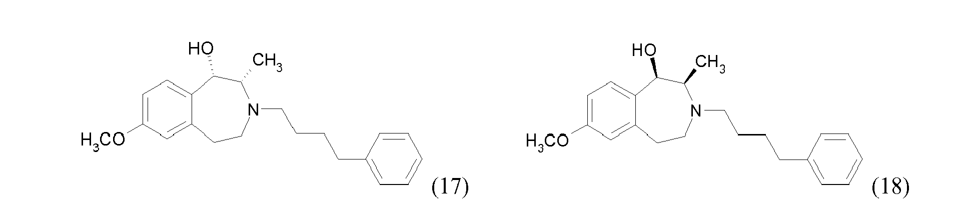

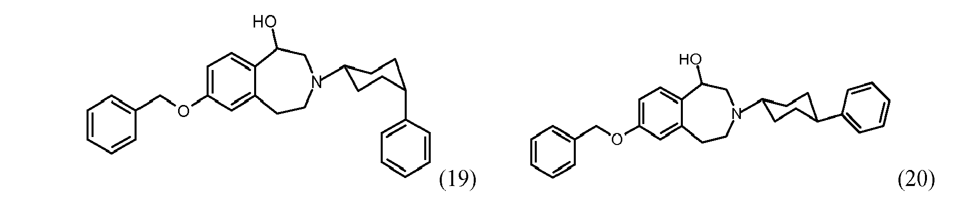

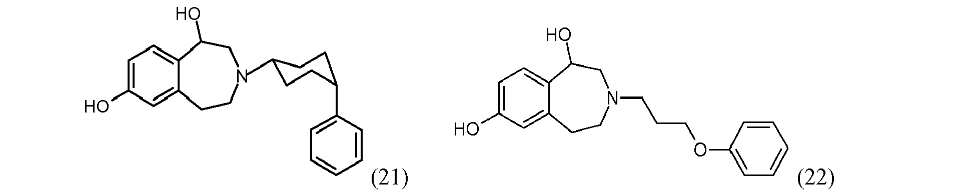

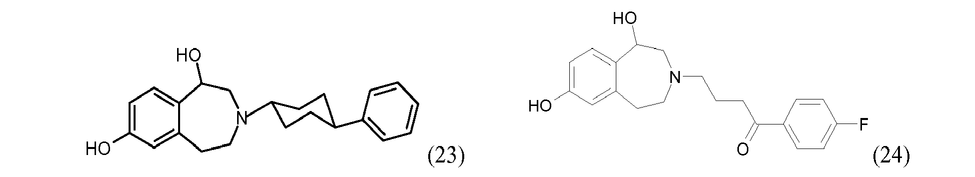

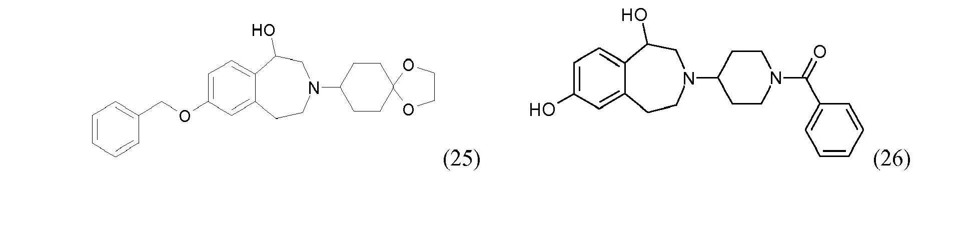

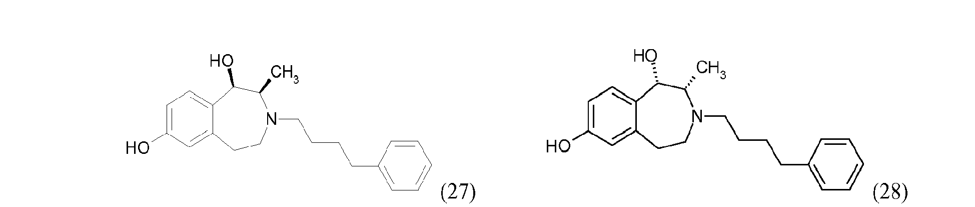

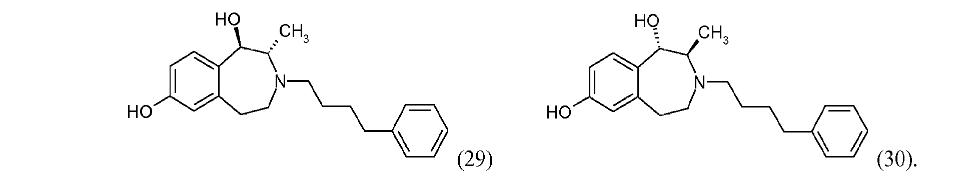

6. Compounds according any one to of the foregoing claims and/or racemates, enantiomers, diastereomers, solvates, hydrates, and pharmaceutically acceptable salts and/or esters thereof for use as a medicament.

7. Compounds according to any one of the foregoing claims and/or racemates, enantiomers, diastereomers, solvates, hydrates, and pharmaceutically acceptable salts and/or esters thereof for use in the treatment of therapeutic and/or prophylactic treatment of a disease selected from the group comprising neuropathological, neuropsychiatric and/or neurodegenerative diseases selected from the group comprising chronic or acute pain, stroke preferably ischemic or hemorrhagic stroke, ischemic brain injury, traumatic brain injury, Parkinson's disease, Alzheimer's disease, Huntington's disease, depression, amyotrophic lateral sclerosis, schizophrenia, anxiety, migraine, epilepsy, addictive disorders, alcohol abuse, intoxication disorders, nicotine abuse, psychoactive substances abuse, withdrawal syndromes, nicotine addiction, cocaine addiction, alcohol and amphetamine addiction, attention deficit hyperactivity disorder, cognition impairment, anxiety disorders, generalized anxiety disorder, panic attacks, panic disorder, bipolar disorder, manic depression, manic-depressive disorder, behavioral disturbances, obsessive compulsive disorders, posttraumatic stress disorder, acute stress disorder, social phobia, simple phobias, pre-menstrual dysphoric disorder, cognitive memory disorders, learning disorders, social anxiety disorder, major depressive disorder, postnatal depression, dysthymia, depression associated with Alzheimer's disease, Parkinson's disease, or psychosis, eating disorders, obesity, anorexia nervosa, bulimia nervosa, binge eating disorder, analgesia, Lesch-Nyhan syndrome, neurodegenerative diseases, late luteal phase syndrome or narcolepsy, psychiatric symptoms, anger, rejection sensitivity, movement disorders, extrapyramidal syndrome, Tic disorders, restless leg syndrome, tardive dyskinesia, supranuclear palsy, sleep disorders, sleep related eating disorder, night eating syndrome, stress urinary incontinence, migraine, neuropathic pain, diabetic neuropathy, fibromyalgia syndrome, chronic fatigue syndrome, sexual dysfunction, premature ejaculation, male impotence, and/or thermoregulatory disorders, and/or for use in a neuroprotective treatment.

8. Compounds according to claim 7 **characterized in that** the disease is selected from the group comprising chronic or acute pain, stroke preferably ischemic or hemorrhagic stroke, ischemic brain injury, traumatic brain injury, Parkinson's disease, Alzheimer's disease, Huntington's disease, depression, schizophrenia, anxiety, migraine, addictive disorders, alcohol abuse, intoxication disorders, nicotine abuse, psychoactive substances abuse, withdrawal syndromes, nicotine addiction, cocaine addiction, alcohol and/or amphetamine addiction.

9. Use of compounds according any one to of the foregoing claims and/or racemates, enantiomers, diastereomers, solvates, hydrates, and pharmaceutically acceptable salts and/or esters thereof for the manufacture of a medicament.

10. Use of compounds according to any one of the foregoing claims and/or racemates, enantiomers, diastereomers, solvates, hydrates, and pharmaceutically acceptable salts and/or esters thereof for the manufacture of a medicament for therapeutic and/or prophylactic treatment of a disease selected from the group comprising neuropathological, neuropsychiatric and/or neurodegenerative diseases selected from the group comprising chronic or acute pain, stroke preferably ischemic or hemorrhagic stroke, ischemic brain injury, traumatic brain injury, Parkinson's disease, Alzheimer's disease, Huntington's disease, depression, amyotrophic lateral sclerosis, schizophrenia, anxiety, migraine, epilepsy, addictive disorders, alcohol abuse, intoxication disorders, nicotine abuse, psychoactive substances abuse, withdrawal syndromes, nicotine addiction, cocaine addiction, alcohol and amphetamine addiction, attention deficit hyperactivity disorder, cognition impairment, anxiety disorders, generalized anxiety disorder, panic attacks, panic disorder, bipolar disorder, manic depression, manic-depressive disorder, behavioral disturbances, obsessive compulsive disorders, posttraumatic stress disorder, acute stress disorder, social phobia, simple phobias, pre-menstrual dysphoric disorder, cognitive memory disorders, learning disorders, social anxiety disorder, major depressive disorder, postnatal depression, dysthymia, depression associated with Alzheimer's disease, Parkinson's disease, or psychosis, eating disorders, obesity, anorexia nervosa, bulimia nervosa, binge eating disorder, analgesia, Lesch-Nyhan syndrome, neurodegenerative diseases, late luteal phase syndrome or narcolepsy, psychiatric symptoms, anger, rejection sensitivity, movement disorders, extrapyramidal syndrome, Tic disorders, restless leg syndrome, tardive dyskinesia, supranuclear palsy, sleep disorders, sleep related eating disorder, night eating syndrome, stress urinary incontinence, migraine, neuropathic pain, diabetic neuropathy, fibromyalgia syndrome, chronic fatigue syndrome, sexual dysfunction, premature ejaculation, male impotence, and/or thermoregulatory disorders, and/or for neuroprotective treatment.

11. Use of compounds according to claim 10 **characterized in that** the disease is selected from the group comprising chronic or acute pain, stroke preferably ischemic or hemorrhagic stroke, ischemic brain injury, traumatic brain injury, Parkinson's disease, Alzheimer's disease, Huntington's disease, depression, schizophrenia, anxiety, migraine, addictive disorders, alcohol abuse, intoxication disorders, nicotine abuse, psychoactive substances abuse, withdrawal syndromes, nicotine addiction, cocaine addiction, alcohol and/or amphetamine addiction.

12. Pharmaceutical composition comprising as an active ingredient a compound according to any one of the foregoing claims and/or racemates, enantiomers, diastereomers, solvates, hydrates, pharmaceutically acceptable salts and/or esters thereof.

13. Method for preparing a compound according to any one of the foregoing claims wherein R³ is not hydrogen comprising the steps of:
a) Preparation of a 2-(3-alkoxyphenyl)ethanol;
b) Esterification of the COOH group of an amino acid other than glycine;
c) Introduction of a protecting group to the nitrogen of the esterified amino acid of step b);
d) Nucleophilic substitution of the protected amino acid of step c) with 2-(3-alkoxyphenyl)ethanol of step a);
e) Alkaline hydrolysis of the alkyl ester of the amino acetate of step d) to the respective carboxylic acid;
f) Cyclisation of the carboxylic acid of step e) by a Friedel-Crafts acylation;
g) Reduction of the ketone product of step f) to the respective alcohol;
h) Elimination of the protecting group from the nitrogen and thereby obtaining the secondary amine of the compound of step g);
i) Introduction of a group R⁴ to the nitrogen of the compound of step h) by alkylation;
j) optionally hydrogenolytic elimination of the alkoxy group of step a) to receive a group R¹ that is hydrogen.

14. Method for preparing a compound according to any one of the foregoing claims wherein R² and R³ is hydrogen comprising the steps of:
1) Introduction of a protecting group to the nitrogen of 2-(3-alkoxyphenyl)ethane amine;
2) Nucleophilic substitution of the protected amine of step 1) with ethyl bromoacetate;
3) Alkaline hydrolysis of the ethyl ester of the amino acetate of step 2) to the respective carboxylic acid;
4) Cyclisation of the carboxylic acid of step 3) by a Friedel-Crafts acylation;
5) optionally Splitting the alkyl ether of the compound of step 4) and receiving the phenol;
6) optionally Benzylation of the phenol of step α);
7) Reduction of the ketone product of step 4) or step 6) to the respective alcohol;
8) Elimination of the protecting group from the nitrogen and thereby obtaining the secondary amine of the compound of step 7);
9) Introduction of a group R⁴ to the nitrogen of the compound of step 8) by alkylation or acylation;
10) optionally hydrogenolytic elimination of the benzyl group to receive a group R¹ that is hydrogen.
